# EUROPEAN PATENT APPLICATION

(11) **EP 1 785 426 A1**
(43) Date of publication of application: **16.05.2007**
(21) Application number: 05775867.4
(22) Date of filing: 01.09.2005
(51) Int. Cl.: C07D 477/00, A61K 31/407, A61K 31/4184, A61K 31/423, A61K 31/4709, A61K 31/4725, A61K 31/535, A61P 31/04

(54) **NOVEL CARBAPENEM COMPOUND**

(30) Priority: 03.09.2004 JP 2004257634
(71) Applicant: Dainippon Sumitomo Pharma Co., Ltd., Osaka 541-8524 (JP)
(72) Inventor: SUNAGAWA, Makoto, Hyogo 664-0875 (JP); SASAKI, Akira, Hyogo 665-0805 (JP)
(74) Representative: Vossius & Partner
(86) International application number: PCT/JP2005/015972
(87) International publication number: WO 2006/025475

(57) **Abstract**

A carbapenem compound represented by a following formula [1], 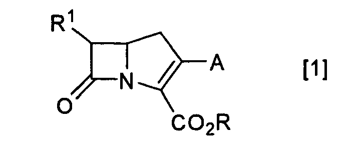 wherein R¹ is C₁ to C₃ alkyl or C₁ to C₃ alkyl substituted by hydroxy. R is hydrogen atom or a group which regenerates a carboxyl group by hydrolysis in a living body,
A is a following formula [1a], 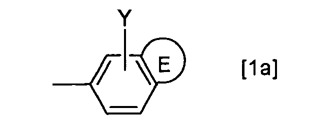 wherein E is a 5 to 7 membered cyclic ring optionally containing 1 to 3 hetero atoms (excluding benzene ring) which forms a bicyclic ring in corporation with the benzene ring,
Y is hydrogen atom, C₁ to C₄ alkyl, C₁ to C₄ alkoxy, trifluoromethoxy, halogen atom or cyano group,
or its pharmaceutically acceptable salt.

## Description

### TECHNICAL FIELD

The present invention relates to a novel carbapenem compound. In more detail, the present invention relates to a carbapenem compound, wherein a phenyl fused with a secondary cyclic ring is directly substituted at position 3 of 7-oxo-1-azabicyclo[3.2.0]hept-2-ene which is a basic nucleus of the carbapenem compound. Furthermore, the present invention relates to an antibacterial agent containing such a compound as an active ingredient.

### BACKGROUND ART

The carbapenem compounds which have been developed and commercialized are poor in absorbability from the digestive tract and therefore, they are clinically used only in the form of injection, mainly intravenous injection. However, in the clinical field, it is desirable to select several administration routes from the viewpoint of circumstances or wishes of a patient, a therapeutic object, etc. Especially, oral administration of an antibacterial agent is easy and convenient for administration to a patient in comparison with injection. In view of the care of a patient at home, oral administration of the antibacterial agent is more convenient and the clinical usability is extremely high. It has been strongly desired in the clinical field to develop a carbapenem compound which has a potent antibacterial activity especially against Haemophilus influenzae (which widely gain resistance to the inhibitory effect of existing β-lactam agents together with mutation of a penicillin binding proteins (PBP), such as β-lactamase non-producing ampicillin resistant Haemophilus influenzae (BLNAR), and penicillin resistant Streptococcus pneumoniae (PRSP), which have been recently increasingly isolated and provide a clinical trouble), and is rich in safety and is orally administrable. However none of such agents has been put on the market. Tricyclic carbapenem compounds which have been studied and developed until now are disclosed for example, in WO 92/03437. These compounds have a characteristic structure in a side chain having a ring which is fused via C-C bond and they are modified to a prodrug thereof for increase of oral absorbability, but their safety in the clinical test is not reported. Besides, there are several known 1β methylcarbapenem compounds (Japanese patent 2-49783A, Japanese patent 8-53453A, Japanese patent 4-279588A, Japanese patent 2-223587A, WO 92/03437, WO 98/34936, WO 99/57121, Antimicrobial Agents and Chemotherapy, Mar. 1999, p460-464). All of them have a structural property having 1β-methyl group and a side chain via sulfide bond which are the said to contribute to an increase of chemical stability and in a living body (biological) stability, and are modified to a prodrug of them for increase of oral absorbability. Especially, the clinical trials are carried out on compounds disclosed in Japanese patent 2-49783A and Japanese patent 8-53453A, but the safety of them and so on have been not clear.

On the other hand, carbapenem compounds having an aryl ring via C-C bond as a side chain structure were known since 1980s (US Patent 4543257, US Patent 4775669, US Patent 5258509, Tetrahedron, 1983, Vol. 39, p2531-2549, and Journal of Medicinal Chemistry, 1987, Vol. 30, p871-880). These reports are concerned only to studies and developments on injections thereof, but not to studies for oral application thereof.

Although WO 02/053566, WO 03/040146 and WO 03/089431 relate to agents for oral administration, the substituent at position 3 of 7-oxo-1-azabicyclo[3.2.0]hept-2-ene which is a basic nucleus of the carbapenem compound is limited to a monocyclic ring such as benzene, thiophene or pyridine ring. As the substituent at position 3, carbapenem compounds having only naphthalene ring (as bicyclic ring wherein benzene ring fused with another cyclic ring) are known (US Patent 5006519, US Patent 5032587, EP 466253B and EP 466254B), but other substituents on said position 3 are not referred to therein and such compounds are not applied for oral administration.

Therefore, carbapenem derivatives having above substituents of the present invention are novel, and the oral application of them is not reported.
Patent document 1: WO 92/03437
Patent document 2: Japanese patent 2-49783A
Patent document 3: Japanese patent 8-53453A
Patent document 4: WO 98/34936
Patent document 5: WO 99/57121
Patent document 6: Japanese patent 4-279588A
Patent document 7: Japanese patent 2-223587A
Patent document 8: US Patent 4543257
Patent document 9: US Patent 4775669
Patent document 10: US Patent 5258509
Patent document 11: WO 02/053566
Patent document 12: WO 03/040146
Patent document 13: WO 03/089431
Patent document 14: US Patent 5006519
Patent document 15: US Patent 5032587
Patent document 16: EP 466253B
Patent document 17: EP 466254B
Non patent document 1: Antimicrobial Agents and Chemotherapy, Mar. 1999, p460-464
Non patent document 2: Tetrahedron, 1983, Vol. 39, p2531-2549
Non patent document 3: Journal of Medicinal Chemistry, 1987, Vol.30, p871-880

### DISCLOSURE OF INVENTION

The object of the present invention is to provide a carbapenem compound which has a potent antibacterial activity against Gram positive bacteria and Gram negative bacteria, especially Haemophilus influenzae (which obtain resistance to the inhibitory effect of existing β-lactam agents together with mutation of a penicillin binding proteins (PBP) such as β-lactamase non-producing ampicillin resistant Haemophilus influenzae (BLNAR), and penicillin resistant Streptococcus pneumoniae (PRSP), which are recently increasingly isolated and provide a clinical problem) and has excellent oral absorbability.

The present inventors have intensively studied to find that the carbapenem compound, wherein a phenyl fused with a secondary cyclic ring is directly substituted at position 3 of 7-oxo-1-azabicyclo[3.2.0]hept-2-ene which is a basic nucleus of the carbapenem compound, has a potent antibacterial activity against Gram positive bacteria and Gram negative bacteria, especially Haemophilus influenzae which obtain resistance to inhibitory effect of existing β-lactam agents together with mutation of a penicillin binding proteins (PBP) such as β-lactamase non-producing ampicillin resistant Haemophilus influenzae (BLNAR), and penicillin resistant Streptococcus pneumoniae (PRSP), which have been recently increasingly isolated and provide a clinical problem. Further, they have also found that a compound having a group substituted onto the 2-carboxyl group, the said group being capable of regenerating a carboxyl group by hydrolyzing in the living body, shows a good absorbability from the digestive tract by oral administration, and shows a potent antibacterial activity after converted into a 2-de-esterified compound in the living body, and further shows an excellent resistance to renal dehydropeptidase, and finally have accomplished the present invention.

Namely, the present invention relates to any one of the following 1 to 14:
1. The carbapenem compound represented by a following formula [1], 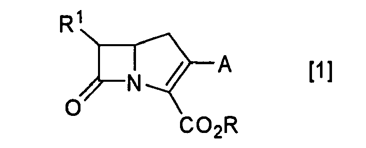 wherein R¹ is C₁ to C₃ alkyl or C₁ to C₃alkyl substituted by hydroxy, R is hydrogen atom or a group which regenerates a carboxyl group by hydrolysis in a living body,
   A is a following formula [1a], 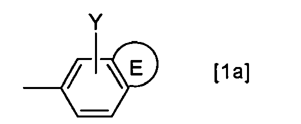 wherein E is a 5 to 7 membered cyclic ring optionally containing 1 to 3 hetero atoms (excluding benzene ring) which forms a bicyclic ring in corporation with the benzene ring,
   Y is hydrogen atom, C₁ to C₄ alkyl, C₁ to C₄ alkoxy, trifluoromethoxy, halogen atom or cyano group,
   or its pharmaceutically acceptable salt.
2. The carbapenem compound or its pharmaceutically acceptable salt according to the above 1, wherein the group which regenerates a carboxyl group by hydrolysis in a living body is C₁ to C₄ alkyl, C₂ to C₁₂ alkyloxyalkyl, a following formula [2], 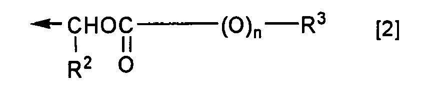 wherein R² is hydrogen atom or C₁ to C₆ alkyl, R³ is optionally substituted C₁ to C₁₀ alkyl or optionally substituted C₃ to C₁₀ cycloalkyl and n is 0 or 1,
   or a following formula [3], 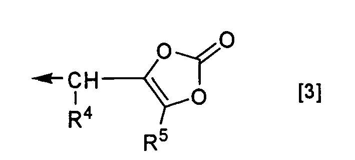 wherein R⁴ is hydrogen atom or C₁ to C₆ alkyl, and R⁵ is hydrogen atom, C₁ to C₆ alkyl, C₃ to C₁₀ cycloalkyl, or phenyl.
3. The carbapenem compound or its pharmaceutically acceptable salt according to above 1, wherein R is hydrogen atom, pivaloyloxymethyl group or (5-methyl-2-oxo-1,3-dioxol-4-yl)methyl.
4. The carbapenem compound or its pharmaceutically acceptable salt according to any one of above 1 to 3, wherein R¹ is 1-hydroxyethyl group.
5. The carbapenem compound or its pharmaceutically acceptable salt according to any of the above 1 to 4, wherein A is a following formula [1b], 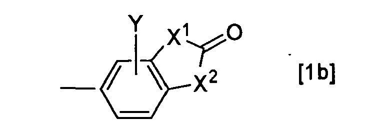 wherein Y is the same as defined in above 1, X¹ is -O-, -NR⁶-(wherein R⁶ is hydrogen atom, a group which is metabolized in a living body to produce imino group, or optionally substituted C₁ to C₄ alkyl group) or methylene, X² is -O- or -NR⁷ (wherein R⁷ is hydrogen atom, a group which is metabolized in a living body to produce imino group, or optionally substituted C₁ to C₄ alkyl group) or methylene.
6. The carbapenem compound or its pharmaceutically acceptable salt according to the above 5, wherein X¹ is -O- or methylene, X² is -NR⁷-(wherein R⁷ is the same as in the above 5).
7. The carbapenem compound or its pharmaceutically acceptable salt according to the above 5, wherein X¹ is -NR⁶- (wherein R⁶ is the same as in the above 5) and X² is -O- or methylene.
8. The carbapenem compound or its pharmaceutically acceptable salt according to any one of the above 1 to 4, wherein A is represented by a following formula [1c], 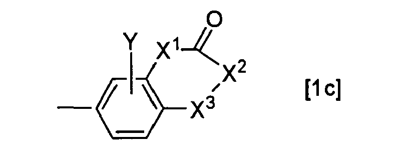 wherein X¹, X² and Y are the same as defined in above 5, X³ is -O-, -NR⁸- (wherein R⁸ is hydrogen atom, a group which is metabolized in a living body to produce imino group or optionally substituted C₁ to C₄ alkyl group) or methylene provided that when X³ is -O-, X² is -NR⁵- (wherein R⁵ is the same as defined in above 5) or methylene.
9. The carbapenem compound or its pharmaceutically acceptable salt according to the above 8, wherein X¹ is -NR⁶- (wherein R⁶ is the same as defined in above 5), X² is methylene and X³ is -O-.
10. The carbapenem compound or its pharmaceutically acceptable salt according to any one of the above 1 to 4, wherein A is a following formula [1d], 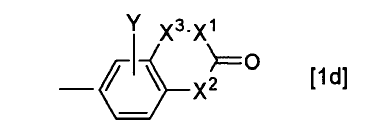 wherein X¹, X², X³ and Y are the same as defined in above 8, provided that when X³ is -O-, X¹ is -NR⁶- (wherein R⁶ is the same as defined in above 5) or methylene.
11. The carbapenem compound or its pharmaceutically acceptable salt according to any one of the above 1 to 4, wherein A is a following formula [1e], 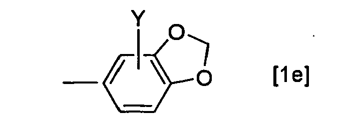 wherein Y is the same as defined in above 8.
12. A medicament for oral administration, which comprises the carbapenem compound or its pharmaceutically acceptable salt according to any one of the above 1 to 11 as an active ingredient.
13. An antibacterial agent containing the carbapenem compound or its pharmaceutically acceptable salt according to any one of the above 1 to 11 as an active ingredient.
14. An antibacterial agent for oral administration containing the carbapenem compound or its pharmaceutically acceptable salt according to any one of the above 1 to 11 as an active ingredient.

### EFFECT OF INVENTION

According to the present invention it becomes possible to provide a carbapenem compound which has a potent antibacterial activity against Gram positive bacteria and Gram negative bacteria, especially Haemophilus influenzae (which obtain resistance to the inhibitory effect of existing β-lactam agents together with mutation of a penicillin binding proteins (PBP) such as β-lactamase non-producing ampicillin resistant Haemophilus influenzae (BLNAR), and penicillin resistant Streptococcus pneumoniae (PRSP), which are recently increasingly isolated and provide a clinical problem) and has excellent oral absorbability.

### BEST MODE FOR CARRYING OUT THE INVENTION

The first aspect of the present invention relates to the above carbapenem compounds. Various terms and preferable examples referred to the present specification are explained as follows.

"C₁ to C₃ alkyl" in R¹ includes a straight or branched chain C₁ to C₃ alkyl, such as methyl, ethyl, n-propyl, isopropyl, etc., preferably ethyl or isopropyl.

"C₁ to C₃ alkyl substituted by hydroxy" in R¹ includes a group having C₁ to C₃, such as hydroxymethyl, 1-hydroxyethyl, 2-hydroxyethyl, 1-hydroxy-1-methylethyl, 1-hydroxypropyl, etc., preferably 1-hydroxyethyl, 2-hydroxyethyl, 1-hydroxy-1-methylethyl, and more preferably or 1-hydroxyethyl.

"C₁ to C₄ alkyl" in Y includes such as methyl, ethyl, n-propyl, isopropyl, n-butyl, isobutyl, tert-butyl, preferably methyl, ethyl, n-propyl, isopropyl, and especially preferably methyl or ethyl.

"C₁ to C₄ alkyloxy" in Y includes methoxy, ethoxy, n-propoxy, isopropoxy, n-butoxy, isobutoxy, sec-butoxy, tert-butoxy, preferably methoxy, ethoxy n-propoxy, isopropoxy, and more preferably methoxy or ethoxy.

"A halogen atom" in Y includes fluorine atom, chlorine atom, bromine atom or iodine atom, preferably fluorine atom, chlorine atom.

Cyclic ring in E includes a 5 to 7 membered monocyclic aromatic or non-aromatic cyclic ring optionally containing 1 to 3 hetero atoms selected from nitrogen, oxygen and sulfur atom. These rings are preferably illustrated below: 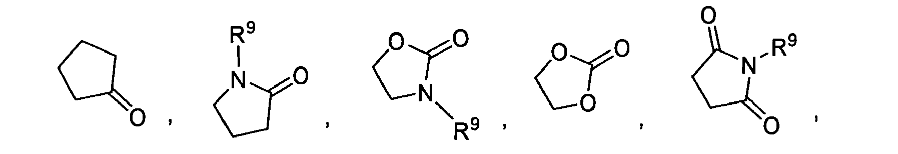 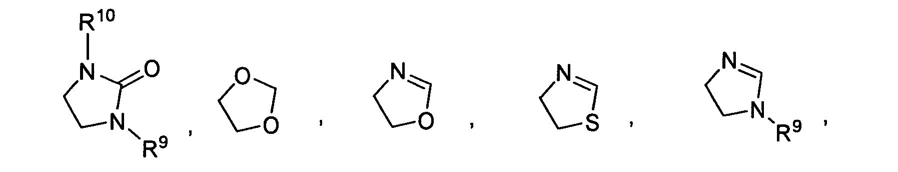 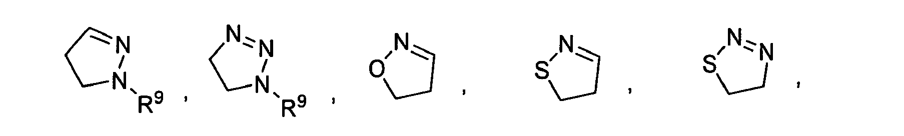 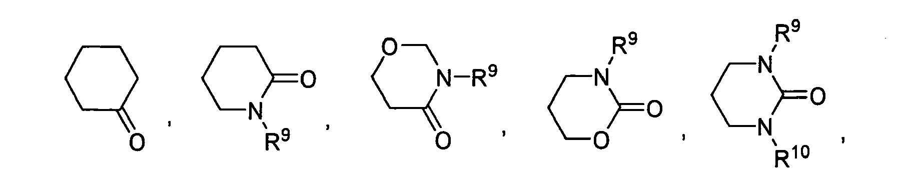 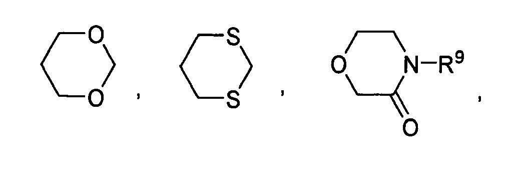 wherein R⁹ and R¹⁰ are independently a substituent on a nitrogen atom and the above ring fuses via optional ethylene with a benzene ring to form a bicyclic ring.

When a nitrogen atom is contained in said ring, a substituent on the nitrogen atom presents, and said substituent includes hydrogen atom, amino protective group, amido protective group, a group which is metabolized in a living body to produce imino group or optionally substituted C₁ to C₄ alkyl group.

The substituent of "optionally substituted C₁ to C₄ alkyl", includes hydroxy group, C₁ to C₃ alkyloxy, C₂ to C₇ alkylcarbonyloxy, C₂ to C₇ alkyloxycarbonyl, C₃ to C₇ cycloalkyl, carboxyl, a halogen atom, cyano, amino, C₁ to C₃ alkylamino, di (C₁ to C₃ alkyl)amino, morpholino, 1-piperidino, 1-piperazinyl, 1-pyrrolodinyl, aminocarbonyl, aminocarbonyloxy, C₂ to C₄ alkylaminocarbonyl, di (C₁ to C₃ alkyl)aminocarbonyl, morpholinocarbonyloxy, 1-piperidinocarbonyloxy, 1-piperazinylcarbonyloxy or 1-pyrrolidinylcarbonyloxy, etc.

C₁ to C₄ alkyl in "optionally substituted C₁ to C₄ alkyl" mentioned above includes straight or branched C₁ to C₄ alkyl, such as methyl, ethyl, n-propyl, isopropyl, n-butyl, sec-butyl, isobutyl or tert-butyl.

"C₁ to C₃ alkyloxy" mentioned above includes methoxy, ethoxy, n-propoxy or isopropoxy.

"C₂ to C₇ alkylcarbonyloxy" mentioned above includes methylcarbonyloxy, ethylcarbonyloxy, n-propylcarbonyloxy or isopropylcarbonyloxy.

"C₂ to C₇ alkyloxycarbonyl" mentioned above includes methyloxycarbonyl, ethyloxycarbonyl, n-propyloxycarbonyl, or isopropyloxycarbonyl.

"C₃ to C₇ cycloalkyl" mentioned above includes cyclopropyl, cyclobutyl, cyclopentyl, cyclohexyl or cycloheptyl.

"A halogen atom" mentioned above includes fluorine atom, chlorine atom, bromine atom or iodine atom, preferably fluorine atom, chlorine atom or bromine atom.

"C₁ to C₃ alkylamino" mentioned above includes methylamino, ethylamino, n-propylamino or isopropylamino.

"Di (C₁ to C₃ alkyl)amino" mentioned above includes dimethylamino, diethylamino, di n-propylamino or diisopropylamino.

"C₂ to C₄ alkylaminocarbonyl" mentioned above includes methylaminocarbonyl, ethylaminocarbonyl, n-propylaminocarbonyl or isopropylaminocarbonyl.

"Di (C₁ to C₃ alkyl)aminocarbonyl" mentioned above includes dimethylaminocarbonyl, diethylaminocarbonyl, di-n-propylaminocarbonyl or diisopropylaminocarbonyl.

These substituents are optionally protected by an appropriate protecting group. The substituted position and its number are not limited as long as these are chemically possible.

"A group which is metabolized in a living body to produce imino group" includes any group as long as the group regenerates imino group by hydrolysis in a living body, and includes any group which is used for conversion into a compound called a prodrug, preferably 2-oxo-1,3-dioxol-4-yl)methyl, (5-methyl-2-oxo-1,3-dioxol-4-yl)methyl, (5-t-butyl-2-oxo-1,3-dioxol-4-yl)methyl, (5-phenyl-2-oxo-1,3-dioxol-4-yl)methyl, 2-oxo-1,3-dioxol-4-yl)methyloxycarbonyl, (5-methyl-2-oxo-1,3-dioxol-4-yl)methyloxycarbonyl, (5-t-butyl-2-oxo-1,3-dioxol-4-yl)methyloxycarbonyl, (5-phenyl-2-oxo-1,3-dioxol-4-yl)methyloxycarbonyl, etc., more preferably (5-methyl-2-oxo-1,3-dioxol-4-yl)methyl, or (5-methyl-2-oxo-1,3-dioxol-4-yl)methyloxycarbonyl.

"A group which regenerates a carboxyl group by hydrolysis in a living body" includes any group as long as the group regenerates a carboxyl group by hydrolysis in a living body, and includes any group which is used for conversion into a compound called a prodrug, preferably C₁ to C₄ alkyl such as methyl, ethyl, propyl, isopropyl or butyl, C₂ to C₁₂ alkyloxyalkyl such as methoxymethyl, ethoxymethyl, 2-methoxyethyl or 2-methoxyethoxymethyl, a group represented by a following formula [2], 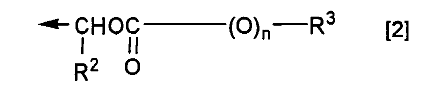 wherein R² is hydrogen atom or C₁ to C₆ alkyl group, R³ is an optionally substituted C₁ to C₁₀ alkyl or an optionally substituted C₃ to C₁₀ cycloalkyl, and n is 0 or 1,
or a group represented by a following formula [3], 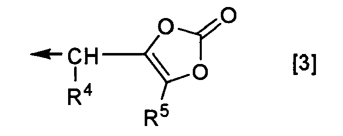 wherein R⁴ is hydrogen atom or C₁ to C₄ alkyl, and R⁵ is hydrogen atom, C₁ to C₆ alkyl, C₃ to C₁₀ cycloalkyl or phenyl.
"C₁ to C₆ alkyl" in R² of the formula [2] includes a straight or branched C₁ to C₆ alkyl, such as methyl, ethyl, n-propyl, isobutyl, tert-butyl, n-pentyl or n-hexyl, and preferably methyl.
"C₁ to C₁₀ alkyl" in R³ includes a straight or branched C₁ to C₁₀ alkyl such as methyl, ethyl, n-propyl, isobutyl, tert-butyl, n-pentyl, n-hexyl, n-heptyl, n-octyl, n-nonyl or n-decyl, and preferably methyl, ethyl, n-propyl, isobutyl, tert-butyl, n-pentyl or n-hexyl.
"C₃ to C₁₀ cycloalkyl" in R³ includes cyclopropyl, cyclobutyl, cyclopentyl, cyclohexyl, cycloheptyl, cyclooctyl, cyclononyl, cyclodecyl, and preferably cyclopropyl, cyclobutyl, cyclopentyl or cyclohexyl.
The substituent of "optionally substituted C₁ to C₁₀ alkyl" inR³ includes C₃ to C₁₀ cycloalkyl, such as cyclopropyl, cyclobutyl, cyclopentyl or cyclohexyl, and preferably cyclohexyl. The substituent of "optionally substituted C₃ to C₁₀ cycloalkyl" includes a straight or branched C₁ to C₆ alkyl, such as methyl, ethyl, n-propyl, isobutyl, tert-butyl, n-pentyl or n-hexyl, and preferably methyl or ethyl.
A group represented by the formula [2] includes, for example pivaloyloxymethyl, acetyloxymethyl, cyclohexylacetyloxymethyl, 1-methylcyclohexylcarbonyloxymethyl, ethoxycarbonyloxy-1-ethyl or cyclohexyloxycarbonyloxy-1-ethyl, etc., and preferably pivaloyloxymethyl.
C₁ to C₄ alkyl in R⁴ of the formula [3] includes methyl, ethyl, propyl, isopropyl, butyl, etc. C₁ to C₆ alkyl in R⁵ includes methyl, ethyl, propyl, isopropyl, butyl, sec-butyl, isobutyl, t-butyl, pentyl, hexyl, etc. C₃ to C₁₀ cycloalkyl in R⁵ includes cyclopropyl, cyclobutyl, cyclopentyl, cyclohexyl, etc. A group represented by the formula [3] includes preferably (2-oxo-1,3-dioxol-4-yl)methyl, (5-methyl-2-oxo-1,3-dioxol-4-yl)methyl, (5-t-butyl-2-oxo-1,3-dioxol-4-yl)methyl, (5-phenyl-2-oxo-1,3-dioxol-4-yl)methyl, etc., and especially preferably (5-methyl-2-oxo-1,3-dioxol-4-yl)methyl.

Furthermore, a preferable other group of groups which are hydrolyzed in a living body to produce carboxyl group is phthalidyl group.

Various protecting groups used usually can be used as the protecting group of carboxyl group and include preferably a straight or branched C₁ to C₆ alkyl, such as methyl, ethyl, isopropyl, tert-butyl, etc., C₁ to C₆ halogenoalkyl, such as 2-iodoethyl, 2,2,2-trichloroethyl, etc., C₂ to C₇ alkyloxymethyl, such as methoxymethyl, ethoxymethyl, isobutoxymethyl, etc, C₂ to C₇ alkylcarbonyloxymethyl, such as acetyloxymethyl, propionyloxymethyl, butyryloxymethyl, pivaloyloxymethyl, etc., C₄ to C₁₁ 1-alkyloxycarbonyloxyethyl, such as 1-ethoxycarbonyloxyethyl, etc., aralkyl, such as benzyl, p-methoxybenzyl, o-nitrobenzyl, p-nitrobenzyl, etc., C₃ to C₇ alkenyl, such as allyl, 3-methylallyl, etc., benzhydryl, phthalidyl, (2-oxo-1,3-dioxol-4-yl)methyl, (5-methyl-2-oxo-1,3-dioxol-4-yl)methyl, (5-t-butyl-2-oxo-1,3-dioxol-4-yl)methyl, (5-phenyl-2-oxo-1,3-dioxol-4-yl)methyl, etc.

Various protecting groups used usually can be used as the protecting group of hydroxy, amino and amido group and include preferably C₂ to C₇ alkyloxycarbonyl, such as tert-butoxycarbonyl, etc, C₁ to C₅ halogenoalkyloxycarbonyl, such as 2-iodoethoxycarbonyl, 2,2,2-trichloroethoxycarbonyl, etc., optionally substituted C₂ to C₇ alkenyloxycarbonyl, such as allyloxycarbonyl, etc., aralkyloxycarbonyl, such as benzyloxycarbonyl, p-methoxybenzyloxycarbonyl, o-nitrobenzyloxycarbonyl, p-nitrobenzyloxycarbonyl, etc., trialkylsilyl, such as trimethylsilyl, triethylsilyl, tert-butyldimethylsilyl, etc. Furthermore, various protecting groups which regenerates a hydroxy, amino and/or amido group by hydrolysis in a living body, and a protected group such as (5-methyl-1,3-dioxolen-2-on-4-yl)methyl, or (5-methyl-1,3-dioxolen-2-on-4-yl)methoxycarbonyl can be preferably used.

The pharmaceutically acceptable salt of the carbapenem of the present invention includes a conventional non-toxic salt. Such salts include, as a salt with an intramolecular carboxylic acid, a salt with an inorganic base, such as sodium, potassium, calcium, magnesium, ammonium salt, etc., a salt with an organic base, such as triethylammonium, pyridinium, diisopropylammonium salt, etc., or as a salt with an intramolecular basic group, a salt with an inorganic acid such as hydrochloric acid, sulfuric acid, phosphoric acid, or a salt with an organic acid, such as formic acid, acetic acid, oxalic acid, methanesulfonic acid, benzenesulfonic acid, etc.

The carbapenem compound of the present invention or the pharmaceutically acceptable salt thereof may be in the form of an anhydride thereof, a hydrate thereof, or a solvate thereof.

The second aspect of the present invention relates to a pharmaceutical composition containing a carbapenem compound as an active ingredient.

Since the carbapenem compound of the present invention has a potent antibacterial activity, excellent oral absorbability and furthermore, has excellent stability to DHP-1, the compound is expected as a potent antibacterial agent which is clinically applicable.

The carbapenem compound of the present invention exhibits broad antibacterial spectrum including gram positive bacteria, such as Staphylococcus aureus, Staphylococcus epidermidis, Streptococcus pyogenes, Streptococcus pneumoniae, Enterococcus faecalis, etc., and gram negative bacteria, such as Escherichia coli, the genus Proteus, Klebsiella pneumoniae, Haemophilus influenzae, Neisseria gonorrhoe, the genus Branhamella, etc. The carbapenem compound of the present invention has been found to have a potent antibacterial activity especially against Haemophilus influenzae (which widely gain resistance to the inhibitory effect of existing β-lactam agents together with mutation of a penicillin binding proteins (PBP), such as β-lactamase non-producing ampicillin resistant Haemophilus influenzae (BLNAR), and penicillin resistant Streptococcus pneumoniae (PRSP), which have been recently increasingly isolated and provide a clinical trouble).

It is well known that dehydropeptidase-I (DHP-I), a renal enzyme can easily hydrolyze a carbapenem derived from natural sources. However, the carbapenem compounds of the present invention is stable to DHP-I and it is possible to use it solely. However, it is possible to use the compound of the present invention together with a DHP-I inhibitor, if necessary.

When used as an antibacterial agent in the treatment of infectious diseases caused by bacteria, the carbapenem compounds of the present invention are administered, for example, orally in the form of a tablet, a capsule, powders, syrup, etc., or parenterally such as intravenous injection, intramuscular injection, or intrarectal administration. The compound wherein R in the formula [1] means a group which regenerates a carboxyl group by hydrolysis in a living body is especially preferable as an antibacterial agent administered orally (orally administered antibacterial agent).

The suitable administration forms as mentioned above may be prepared in a conventional manner by mixing an active ingredient with a pharmaceutically acceptable carrier, excipient, binder, stabilizer, etc. When administered in the form of injection, a pharmaceutically acceptable buffering agent, a solubilizer, an isotonic agent, etc. may be added thereto.

The dosage of the compound varies according to the symptoms, ages, body weights, the administration form, the frequency of the administration, etc., but it is usually in the range of 100 to 3000mg per day for an adult, which is administered once or divided into several dosage units. Besides, the dosage of the compound may be increased or decreased, if necessary.

The carbapenem compound of the present invention is prepared by various known methods (Tetrahedron, 39, 2531-2549 (1983), Tetrahedron Letters, 31, 2853-2856 (1990), ibid. 34, 3211-3214 (1993), ibid. 36, 4563-4566 (1995), Japanese patent 4-40357B, WO 02/053566, etc.). One of these methods, for example is illustrated as follows: 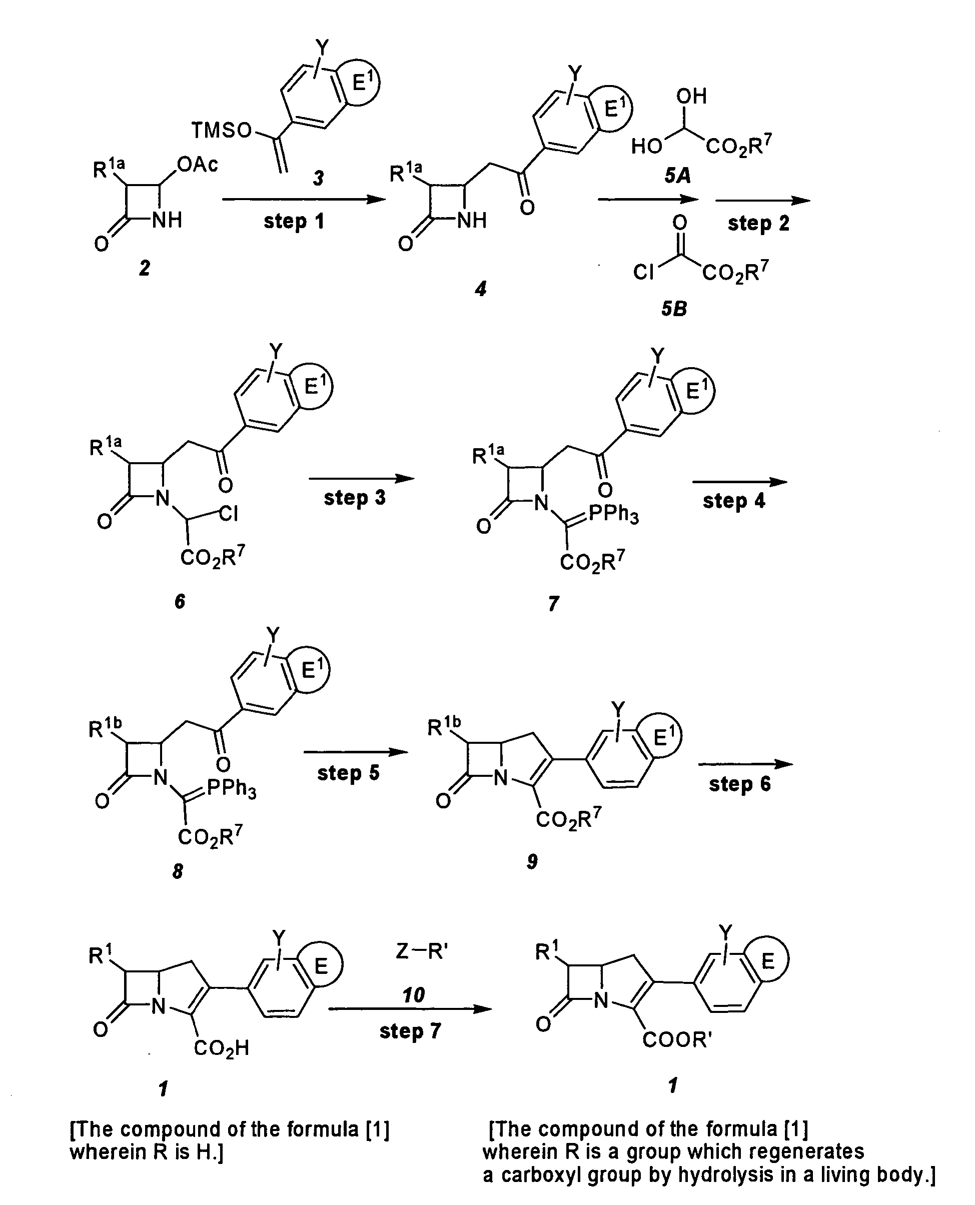 wherein R¹, E and Y are the same as define above, R⁷ is a protective group of carboxyl group, R^{1a} and R^{1b} are independently C₁ to C₃ alkyl group or C₁ to C₃ alkyl group which is substituted by a protected hydroxy group, E¹ means a cyclic ring wherein a substituent of the nitrogen atom which constitutes the ring is not hydrogen atom among cylric rings E, R' means a group which easily regenarates a carboxyl group by hydrolysis in a living body and Z is chlorine atom, bromine atom or iodine atom.

### Step 1: Process for preparing compound 4

Compound 4 is prepared by reacting compound 2 and compound 3 in the presence of acid catalyst in an inert solvent. The acid catalyst includes zinc chloride, zinc bromide, zinc iodide, stannous tetrachloride, trifluoromethanesulfonic acid trimethylsilyl ester, BF₃·diethyl ether, etc. The inert solvent includes methylene chloride, 1,2-dichloroethane, acetonitrile, monochlorobenzene, dioxane, tetrahydrofuran, benzene, toluene, etc. The reaction temperature is -78°C to +60°C, preferably -30°C to +40°C.

Starting compound 3 can be prepared by enol-etherfication of various ketones which are prepared by the known method (e.g. Synthesis and reaction of organic compound [II] page 751-875 (1977), Sin Jikken Kagaku Kouza edited by The Chemical Society of Japan, Vol. 14 (Maruzen); Organic Synthesis [III], Aldehyde-Ketone-Quinone, page 149-353 (1991), Sin Jikken Kagaku Kouza edited by The Chemical Society of Japan, Vol. 21, 4th Edition (Maruzen); or
Comprehensive Organic Transformation by R. C. Larock (VCH Publisher Inc., 1989).

### Step 2: Process for preparing compound 6

Corresponding hemiacetal of compound 6 can be prepared by heating compound 4 and compound 5A under dehydrated condition in an inert solvent. The inert solvent includes methylene chloride, 1,2-dichloroethane, monochlorobenzene, benzene, toluene, xylene, etc. The reaction temperature is +50°C to +200°C, preferably +80°C to +150°C. In accordance with the known method (for example, Journal of Organic Chemistry, 61, 7889-7894(1996)), compound 4 and compound 5B are reacted in the presence of a base in an inert solvent to give an imido compound. The imido compound is then reduced to give a corresponding hemiacetal, too. The base includes triethylamine, diisopropylethylamine, N-methylmorpholine, etc. The inert solvent used in the imidation reaction includes methylene chloride, 1,2-dichloroethane, monochlorobenzene, etc. The imidation was carried out at -50°C to +50°C, preferably -30°C to +30°C. The reducing agent is preferably zinc and the solvent used in the reduction preferably includes a mixed solvent such as a mixture of acetic acid and methylene chloride, a mixture of acetic acid and 1,2-dichloroethane, or a mixture of acetic acid and monochlorobenzene. The reduction is carried out at -50°C to +50°C, preferably -30°C to +30°C.

Thus obtained hemiacetal is chlorinated using a chlorinating agent such as thionyl chloride, oxalyl chloride or phosphorous oxychloride to give compound 6. The chlorination is carried out in an inert solvent such as ether, tetrahydrofuran, methylene chloride, etc. in the presence of a base such as lutidine, pyridine, quinoline, diisopropylethylamine, triethylamine, etc. The reaction temperature is -78°C to +60°C, preferably -30°C to +40°C.

### Step 3: Process for preparing compound 7

Compound 6 is reacted with triphenylphosphine in an inert solvent such as tetrahydrofuran, dioxane, dimethoxyethane, etc., in the presence of a base such as lutidine, pyridine, quinoline, diisopropylethylamine, triethylamine, etc., to give compound 7. The reaction temperature is 0°C to +100°C, preferably +10°C to +70°C.

### Step 4: Process for preparing compound 8

If necessary, the removal of the protective group of hydroxy group in R^{1a} is carried out, followed by reprotection of it to give compound 8.

The removal of the protective group and the introduction of the protective group are carried out by conventional methods (See T. W. Greene, P. G. M. Wuts: Protective Groups in Organic Synthesis; 3rd edition, Wiley, New York (1999) or P. Kocienski, Protecting Groups, Thieme, Stuttgart (1994)).

### Step 5: Process for preparing compound 9

Compound 8 is subjected to cyclization reaction in an inert solvent such as benzene, toluene, xylene, etc, at temperature of +80°C to +200°C to give compound 9.

### Step 6: Process for preparing a carbapenem compound 1 (R = hydrogen atom)

Carbapenem compound 1 can be prepared by removing a protective group of carboxyl group in R⁷ of compound 9, or by removing the protective group of hydroxy group when R^{1b} has a protected hydroxy group. The removal of the protective group is carried out by the known method by treating with an acid, a base or a reducing agent, for example is referred to T. W. Greene, P. G. M. Wuts: Protective Groups in Organic Synthesis; 3rd edition, Wiley, New York (1999) or P. Kocienski, Protecting Groups, Thieme, Stuttgart (1994).

### Step 7: Process for preparing carbapenem compound 1 (R = a group which regenerates a carboxyl group by hydrolysis in a living body)

By introducing a protecting group which regenerates a carboxyl group by hydrolysis in a living body into carbapenem compound 1 (R = hydrogen atom) in accordance with a conventional method, carbapenem compound 1 (R = a group which regenerates a carboxyl group by hydrolysis in a living body) is obtainable. For example, a carbapenem compound 1 (R = hydrogen atom) or its carboxylic acid salt is esterified with a halide 10, if necessary in the presence of a base, such as diisopropylethylamine, triethylamine, 4-dimethylaminopyridine, potassium carbonate or sodium hydrogencarbonate, or in the presence of a phase-transfer catalyst such as triethylbenzyl ammonium chloride, tetrabutylammonium bromide as to give a carbapenem compound 1 (R = a group which regenerates a hydroxy group by hydrolysis in a living body). The reaction solvent is not limited as long as it is an inactive solvent, and preferably dimethylformamide, dimethyl sulfoxide, hexamethylphosphoramide, acetonitrile, dioxane, tetrahydrofuran, acetone, etc. The carboxylic acid salt includes preferably sodium salt and potassium salt. The reaction temperature is at -78°C to +100°C, preferably -20°C to +60°C. Compound 10 includes a halogenated compound of the group represented by the above formula [2] or [3].

In the above steps, when compound 5A or compound 5B, wherein R⁷ is a group which regenerates a carboxyl group by hydrolysis in a living body, is used, and the remaining steps are carried out, carbapenem compound 1 (R = a group which regenerates carboxyl group by hydrolysis in a living body) can be directly prepared.

When the above reaction is completed, a reaction product is isolated by a conventional organic procedure, but when a water soluble product is obtained, a solution of the reaction mixture is neutralized, and the solution is subjected to a column chromatography using absorption resin, etc., and parts which an object compound is eluted are separated and lyophilized to give a reaction product.

The processes for preparing carbapenem compounds of the present invention are not limited by the above methods.

The optical isomers based on asymmetric carbon atoms on the carbapenem compound of the present invention at the 5- and 6- positions of 7-oxo-1-azabicyclo[3.2.0]hept-2-ene, a basic nuclear, present as shown in a following formula [1],

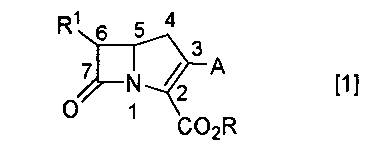

These isomers are all conveniently expressed by only one formula, but the scope of the present invention should not be construed to be limited thereto, and includes all isomers and a mixture of isomers based on each asymmetric carbon atom. The preferable isomers are ones wherein the 5-carbon atom has an R-configuration such as (5R, 6R)-compounds or (5R, 6S)-compounds. More preferable compounds are ones represented by a following formula [1A],

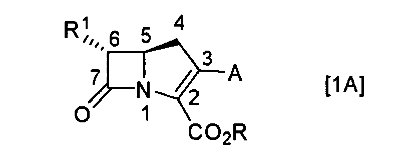

Furthermore, when R¹ is 1-hydroxyethyl group, there are isomers having an R-configuration and an S-configuration at the position 8 as shown in a following formula [1B], and an isomer having the R-configuration is preferable.

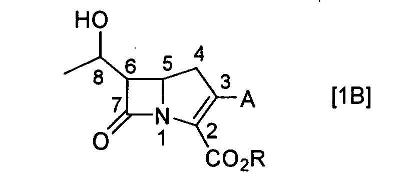

In regard to the cyclic ring E included in a side chain A at the position 3, optical isomers can be present, and the present invention includes such all isomers and a mixture of the isomers.

Carbapenem compounds of the present invention are illustrated by compounds 1 to 48 in the following tables 1 to 7.

**Table 1**

| | | |
|---|---|---|
| 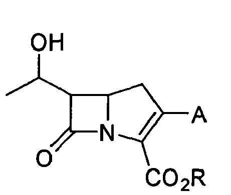 | | |

| No. | R | A |
|---|---|---|
| 1 | -CH₂OCOt-Bu | 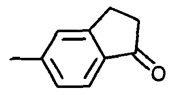 |
| 2 | -CH₂OAc | 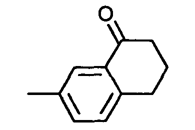 |
| 3 | 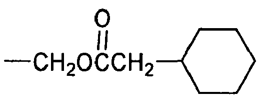 | 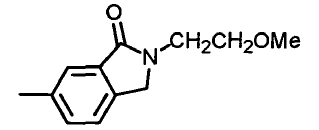 |
| 4 | 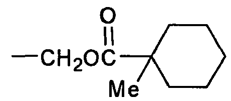 | 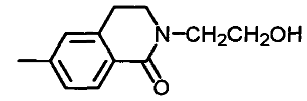 |
| 5 | 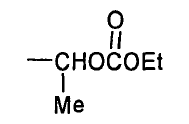 | 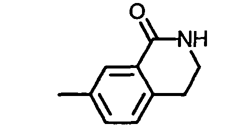 |
| 6 | 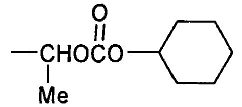 | 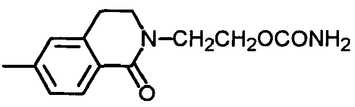 |
| 7 | 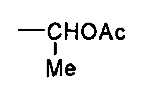 | 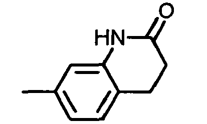 |

**Table 2**

| | | |
|---|---|---|
| 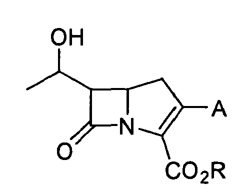 | | |

| No. | R | A |
|---|---|---|
| 8 | -CH₂OCOt-Bu | 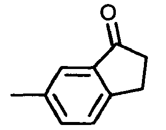 |
| 9 | -CH₂OCOt-Bu | 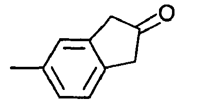 |
| 10 | -CH₂OCOt-Bu | 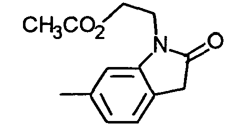 |
| 11 | -CH₂OCOt-Bu | 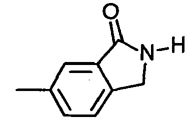 |
| 12 | -CH₂OCOt-Bu | 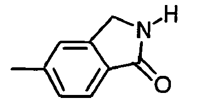 |
| 13 | -CH₂OCOt-Bu | 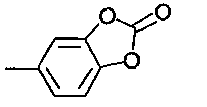 |
| 14 | -CH₂OCOt-Bu | 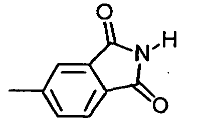 |

**Table 3**

| | | |
|---|---|---|
| 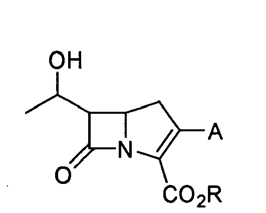 | | |

| No. | R | A |
|---|---|---|
| 15 | 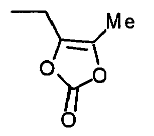 | 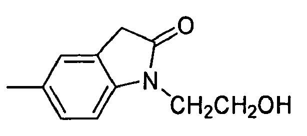 |
| 16 | 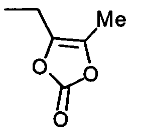 | 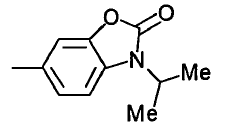 |
| 17 | -CH₂OCOt-Bu | 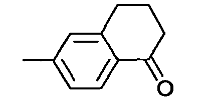 |
| 18 | -CH₂OCOt-Bu | 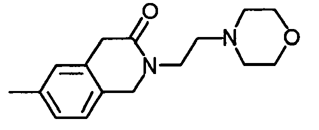 |
| 19 | -CH₂OCOt-Bu | 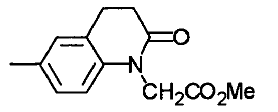 |
| 20 | -CH₂OCOt-Bu | 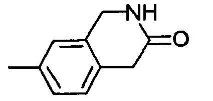 |
| 21 | -CH₂OCOt-Bu | 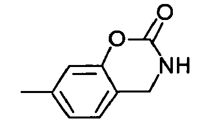 |

**Table 4**

| | | |
|---|---|---|
| 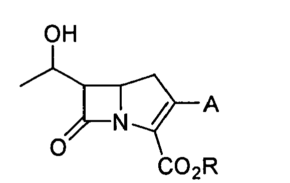 | | |

| No. | R | A |
|---|---|---|
| 22 | -CH₂OCOt-Bu | 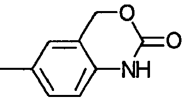 |
| 23 | 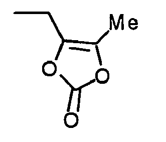 | 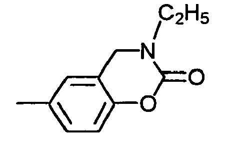 |
| 24 | 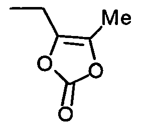 | 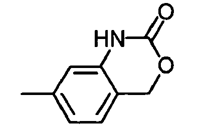 |
| 25 | H | 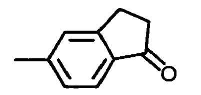 |
| 26 | H | 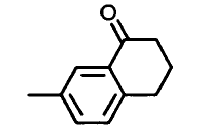 |
| 27 | H | 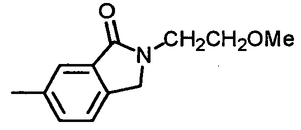 |
| 28 | H | 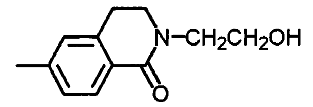 |

**Table 5**

| | | |
|---|---|---|
| 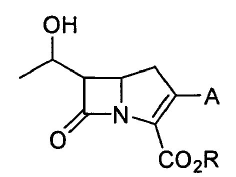 | | |

| No. | R | A |
|---|---|---|
| 29 | H | 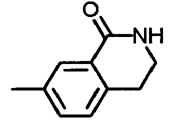 |
| 30 | H | 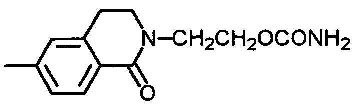 |
| 31 | H | 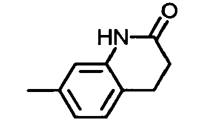 |
| 32 | H | 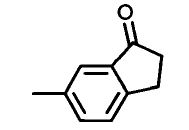 |
| 33 | H | 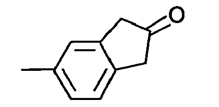 |
| 34 | H | 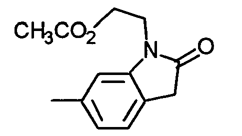 |
| 35 | H | 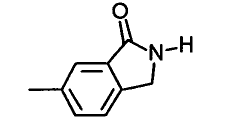 |

**Table 6**

| | | |
|---|---|---|
| 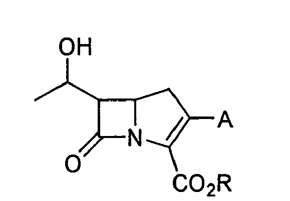 | | |

| No. | R | A |
|---|---|---|
| 36 | H | 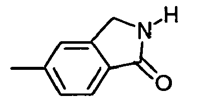 |
| 37 | H | 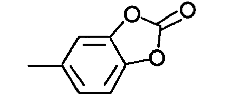 |
| 38 | H | 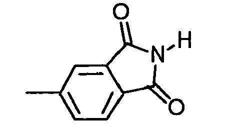 |
| 39 | H | 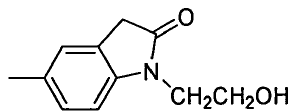 |
| 40 | H | 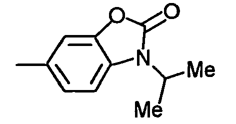 |
| 41 | H | 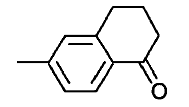 |
| 42 | H | 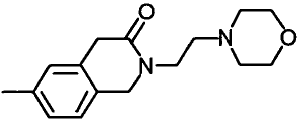 |

**Table 7**

| | | |
|---|---|---|
| 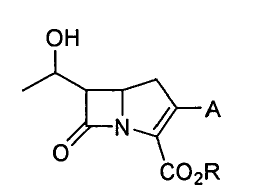 | | |

| No. | R | A |
|---|---|---|
| 43 | H | 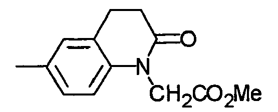 |
| 44 | H | 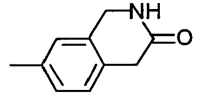 |
| 45 | H | 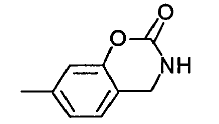 |
| 46 | H | 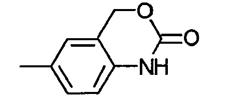 |
| 47 | H | 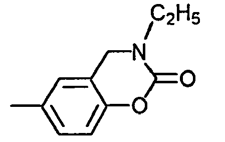 |
| 48 | H | 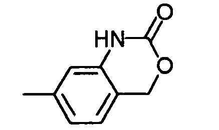 |

The present invention is explained by illustrating Examples, but is not limited by such Examples.

The abbreviated terms used in Examples mean as follows.
Ac: acetyl group
ALOC: allyloxycarbonyl group
br.: broad
t-Bu: tert-butyl group
DMF: N,N-dimethylformamide
Et: ethyl group
Me: methyl group
Ph: phenyl group
PNB: p-nitrobenzyl group
TBDMS: tert-butyl(dimethyl)silyl group
THF: tetrahydrofuran
TMS: trimethylsilyl group

### Example 1

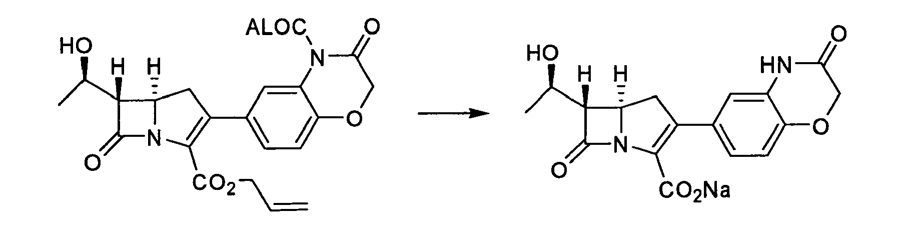

Allyl (5R,6S)-3-(4-allyloxycarbonyl-3-oxo-3,4-dihydro-2H-1,4-benzoxazin-6-yl)-7-oxo-6-{(1R)-1-hydroxyethyl}-1-azabicyclo[3.2.0]hept-2-ene-2-carboxylate (0.36g, 0.67mmol) obtained by referential example 3 and dichloro bis(triphenylphosphine)palladium(II) (23mg, 0.034mmol) were dissolved in methylene chloride (7ml), and thereto was added at 0°C tri n-butyltin hydride (2.7ml, 10mmol), followed by stirring for 30 minutes. To the reaction mixture was dropped aqueous hydrogen sodium solution (0.13M, 10ml), and the aqueous layer was washed with diethyl ether and separated with a separating funnel. After part of the aqueous layer was concentrated in vacuo at 0°C, the residue was purified with C18 reverse column chromatography (filler: Wako Pure Chemical: Wakosil 40C18, mobile phase; 0 to 3%THF/ice cooled ion exchanged water). The combined fraction containing the object compound was stirred at room temperature in vacuo for 1 hour. After removal of THF the residue was lyophilized to give sodium (5R,6S)-3-(3-oxo-3,4-dihydro-2H-1,4-benzoxazin-6-yl)-6-[(1R)-1-hydroxyethyl]-7-oxo-1-azabicyclo[3.2.0]hept-2-ene-2-caroxylate (170mg, yield 69%).
¹H NMR (400 MHz, D₂O) δ 1.12 (d, 3 H, J = 6.4 Hz), 2.86 (dd, 1 H, J = 16.9, 9.8 Hz), 3.20 (dd, 1 H, J = 17.0, 8.6 Hz), 3.30 (dd, 1 H, J = 6.0, 2.8 Hz), 4.02-4.15 (m, 2 H), 6.74-6.86 (m, 3 H).

### Example 2

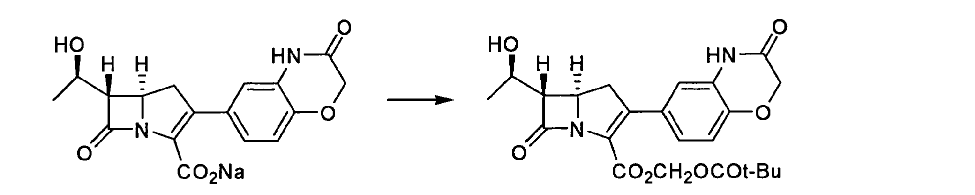

Sodium (5R,6S)-3-(3-oxo-3,4-dihydro-2H-1,4-benzoxazin-6-yl)-6-[(1R)-1-hydroxyethyl]-7-oxo-1-azabicyclo[3.2.0]hept-2-ene-2-carboxylate (0.14g) obtained by Example 1 was dissolved in dry DMF (1.4ml), and to the solution was gradually dropped under ice-cooling pivaloyloxymethyl iodide (98 mg), followed by stirring. One hour later, ethyl acetate was added thereto and the solution was washed with sodium hydrogen carbonate, water and brine. The organic layer was dried over anhydrous sodium sulfate and concentrated. The residue was purified by silica gel column chromatography (hexane : ethyl acetate = 1:1→1:3) to give (2,2-dimethylpropanoyl)oxymethyl (5R,6S)-3-(3-oxo-3,4-dihydro-2H-1,4-benzoxazin-6-yl)-6-[(1R)-1-hydroxyethyl]-7-oxo-1-azabicyclo[3.2.0]hept-2-ene-2-carboxylate (0.11g, yield 68%).
¹H NMR (400 MHz, CDCl₃) δ1.19 (s, 9 H), 1.37 (d, 3 H, J = 6.3 Hz), 1.80 (d, 1 H, J = 4.8 Hz), 3.18-3.35 (m, 3 H), 4.22-4.30 (m, 2 H), 4.63 (s, 2 H), 5.78 (d, 1 H, J = 5.5 Hz), 5.85 (d, 1 H, J = 5.5 Hz), 6.88-7.02 (m, 3 H), 8.07 (s, 1 H).

### Example 3

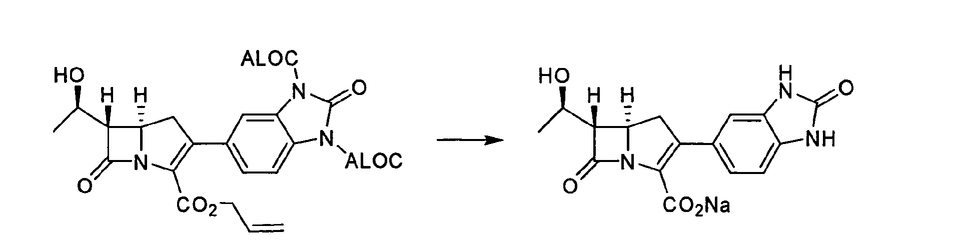

Allyl (5R,6S)-3-(1,3-diallyloxycarbonyl-2-oxo-2,3-dihydro-2H-benzimidazol-5-yl)-7-oxo-6-{(1R)-1-hydroxyethyl}-1-azabicyclo[3.2.0]hept-2-ene-2-carboxylate (0.56g, 1.0mmol) obtained by referential example 7 and dichloro bis(triphenylphosphine)palladium(II) (18mg, 0.025mmol) were dissolved in methylene chloride (35ml) and thereto was added at 0°C tri n-butyltin hydride (4.4 g,15mmol), followed by stirring for 20 minutes. Thereto was dropped aqueous sodium hydrogen carbonate solution (0.20M, 10ml). The aqueous layer was washed with diethyl ether and separated with a separating funnel. After part of the aqueous layer was concentrated in vacuo at 0°C, the residue was purified by C18 reverse column chromatography (filler: Wako Pure Chemical: Wakosil 40C 18, mobile phase; 0 to 2%THF/ice-cooled ion-exchange water). The combined fraction containing the object compound was stirred at room temperature in vacuo for 1 hour. After removal of THF the residue was lyophilized to give sodium (5R,6S)-6-[(1R)-1-hydroxyethyl]-3-(2-oxo-2,3-dihydro-2H-benzimidazol-5-yl)-7-oxo-1-azabicyclo[3.2.0]hept-2-ene-2-carboxylate (181mg, yield 51%).
¹H NMR (400 MHz, D₂O) δ 1.10 (d, 3 H, J = 6.4 Hz), 2.87 (dd, 1 H, J = 16.9, 9.8 Hz), 3.23 (dd, 1 H, J = 17.0, 8.6 Hz), 3.29 (dd, 1 H, J = 6.0, 2.8 Hz), 3.98-4.13 (m, 2 H), 6.86-6.95 (m, 3 H).

### Example 4

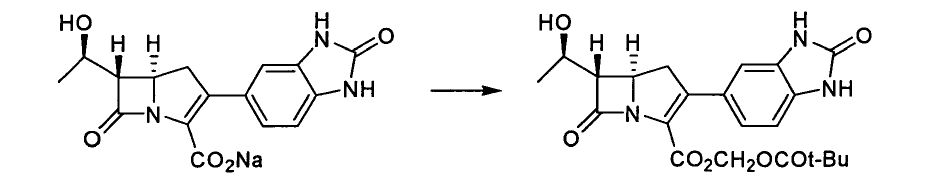

Sodium (5R,6S)-6-[(1R)-1-hydroxyethyl]-3-(2-oxo-2,3-dihydro-2H-benzimidazol-5-yl)-7-oxo-l-azabicyclo[3.2.0]hept-2-ene-2-carboxylate (70 mg) obtained by Example 3 was dissolved in dry DMF (5ml) and thereto was gradually dropped pivaloyloxymethyl iodide (53mg), followed by stirring. One hour later, ethyl acetate was added thereto and the solution was washed with sodium hydrogen carbonate, water and brine. The organic layer was dried over anhydrous sodium sulfate and concentrated. The residue was purified by silica gel column chromatography (hexane : ethyl acetate = 2:1→ethyl acetate only) to give [(2,2-dimethylpropanoyl)oxymethyl (5R,6S)-6-[(1R)-1-hydroxyethyl]-3-(2-oxo-2,3-dihydro-2H-benzimidazol-5-yl)-7-oxo-1-azabicyclo[3.2.0]hept-2-ene-2-carboxylate (55mg, yield 62%).
¹H NMR (400 MHz, CDCl₃) δ1.19 (s, 9 H), 1.37 (d, 3 H, J = 6.3 Hz), 1.93 (br.s, 1 H), 3.16-3.38 (m, 3 H), 4.22-4.31 (m, 2 H), 5.77 (d, 1 H, J = 5.5 Hz), 5.89 (d, 1 H, J = 5.5 Hz), 6.92-7.09 (m, 3 H), 8.28 (br.s, 1 H), 8.35 (br.s, 1 H).

### Example 5

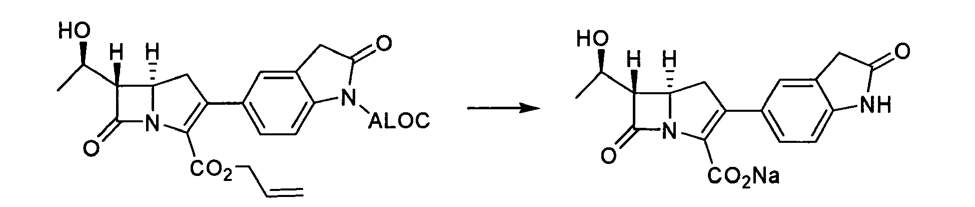

Allyl (5R,6S)-3-(1-allyloxycarbonyl-2-oxo-2,3-dihydro-1H-indol-5-yl)-7-oxo-6-{(1R)-1-hydroxyethyl}-1-azabicyclo[3.2.0]hept-2-ene-2-carboxylate (0.39 g, 0.87mmol) obtained by referential example 11 and dichloro bis(triphenylphosphine)palladium(II) (15mg, 0.022mmol) were dissolved in methylene chloride (25ml) and thereto was added at 0°C tri n-butyltin hydride (3.8 g, 13mmol), followed by 20 minutes. Thereto was dropped aqueous sodium hydrogen solution (0.17M, 10ml). The aqueous layer was washed with diethyl ether and separated with a separating funnel. After part of the aqueous layer was concentrated in vacuo at 0°C, the residue was purified by C 18 reverse column chromatography (filler: Wako Pure Chemical: Wakosil 40C18, mobile phase; 0 to 1%THF/ice-cooled ion exchange water). The combined fraction containing the object compound was stirred at room temperature in vacuo for 1 hour to remove THF. The residue was lyophilized to give sodium (5R,6S)-6-[(1R)-1-hydroxyethyl]-3-(2-oxo-2,3-dihydro-1H-indol-5-yl)-7-oxo-1-azabicyclo[3.2.0]hept-2-ene-2-carboxylate (219mg, yield 72%).
¹H NMR (400 MHz, D₂O) δ 1.11 (d, 3 H, J = 6.2 Hz), 2.82-2.90 (m, 1 H), 3.23 (dd, 1H, J = 17.0, 8.6 Hz), 3.28 (dd, 1 H, J = 6.0, 2.7 Hz), 3.38-3.44 (m, 2 H), 4.02-4.13 (m, 2 H), 6.76 (d, 1 H, 11.8 Hz), 7.03 (d, 1 H, 8.2 Hz), 7.09 (s, 1 H).

### Example 6

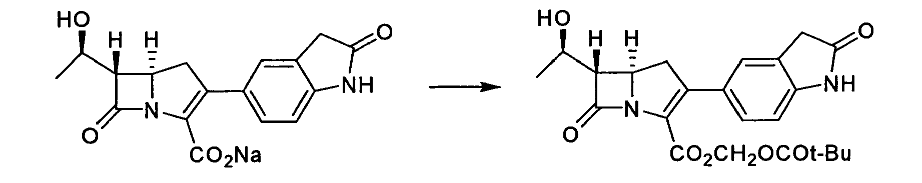

Sodium (5R,6S)-6-[(1R)-1-hydroxyethyl]-3-(2-oxo-2,3-dihydro-1H-indol-5-yl)-7-oxo-1-azabicyclo[3.2.0]hept-2-ene-2-carboxylate (83mg) obtained by Example 5 was dissolved in dry DMF (6ml) and thereto was gradually dropped pivaloyloxymethyl iodide (91mg), followed by stirring. One hour later, ethyl acetate was added thereto and the solution was washed with sodium hydrogen carbonate, water and brine. The organic layer was dried over anhydrous sodium sulfate and concentrated. The residue was purified by silica gel column chromatography (hexane : ethyl acetate = 2:1→ethyl acetate only) to give (2,2-dimethylpropanoyl)oxymethyl (5R,6S)-6-[(1R)-1-hydroxyethyl]-3-(2-oxo-2,3-dihydro-1H-indol-5-yl)-7-oxo-1-azabicyclo[3.2.0]hept-2-ene-2-carboxylate (72mg, yield 69%). ¹H NMR (400 MHz, CDCl₃) δ1.19 (s, 9 H), 1.35 (d, 3 H, J = 6.3 Hz), 1.93 (br.s, 1 H), 3.14-3.33 (m, 3 H), 3.56 (s, 2 H), 4.21-4.33 (m, 2 H), 5.77 (d, 1 H, J = 5.5 Hz), 5.87 (d, 1 H, J = 5.5 Hz), 6.84 (d, 1 H, J = 8.2 Hz), 7.22-7.36 (m, 2 H), 7.82 (s, 1 H).

### Example 7

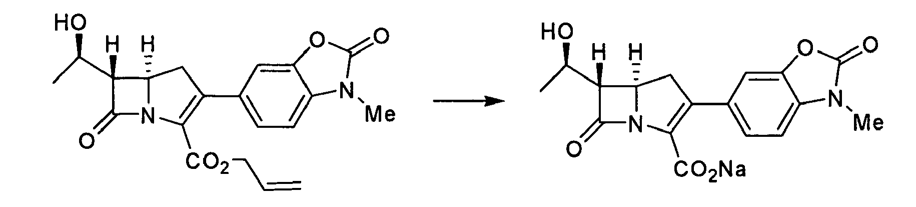

Allyl (5R,6S)-6-[(1R)-1-hydroxyethyl]-3-(2-oxo-3-methyl-2,3-dihydro-1,3-benzoxazol-6-yl)-7-oxo-1-azabicyclo[3.2.0]hept-2-ene-2-carboxylate (0.767g, 2.0mmol) obtained by referential example 20 and triphenylphosphine (52mg) were dissolved in THF (15ml) and thereto were added at 0°C sodium 2-ethylhexanoate (0.33g, 2.0mmol) and tetrakis(triphenylphosphine)palladium(0) (0.12g, 0.1mmol), followed by stirring for 20 minutes. A white solid occurred by addition of hexane (10ml) was filtered under nitrogen atmosphere, washed with hexane, and dried at room temperature in vacuo to give a crude product. The product was dissolved in a small amount of ice water and purified by 18 reverse column chromatography (filler: Wako Pure Chemical: Wakosil 40C 18, mobile phase; 0 to 3% THF/ice-cooled ion-exchange water). The combined fraction containing the object compound was stirred for 1 hour at room temperature in vacuo to remove hexane. The residue was lyophilized to give sodium (5R,6S)-6-[(1R)-1-hydroxyethyl]-3-(2-oxo-3-methyl-2,3-dihydro-1,3-benzoxazol-6-yl)-7-oxo-1-azabicyclo[3.2.0]hept-2-ene-2-carboxylate (310mg, yield 58%).
¹H NMR (400 MHz, D₂O) δ 1.07 (d, 3 H, J = 6.0 Hz), 2.89 (dd, 1 H, J = 16.9, 9.8 Hz), 3.19 (s, 3 H), 3.24 (dd, 1 H, J = 17.0, 8.6 Hz), 3.30 (dd, 1 H, J = 6.0, 2.7 Hz), 4.01-4.14 (m, 2 H), 6.87-6.92 (m, 1 H), 7.02-7.09 (m, 2 H).

### Example 8

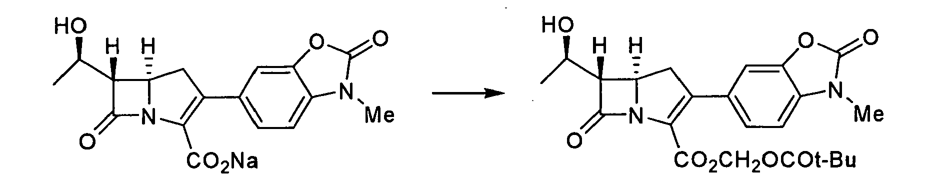

Sodium (5R,6S)-6-[(1R)-1-hydroxyethyl]-3-(2-oxo-3-methyl-2,3-dihydro-1,3-benzoxazol-6-yl)-7-oxo-1-azabicyclo[3.2.0]hept-2-ene-2-carboxylate (0.20g) obtained by Example 7 was dissolved in dry DMF (4ml) and thereto was gradually dropped under ice-cooling pivaloyloxymethyl iodide (145mg), followed by stirring. One hour later, ethyl acetate was added thereto and the solution was washed with sodium hydrogen carbonate, water and brine. The organic layer was dried over anhydrous sodium sulfate and concentrated. The residue was purified by silica gel column chromatography (hexane : ethyl acetate = 1:1→1:3) to give (2,2-dimethylpropanoyl)oxymethyl(5R,6S)-6-[(1R)-1-hydroxyethyl]-3-(2-oxo-3-methyl-2,3-dihydro-1,3-benzoxazol-6-yl)-7-oxo-1-azabicyclo[3.2.0]hept-2-ene-2-carboxylate (0.19g, yield 76%).
¹H NMR (400 MHz, CDCl₃) δ1.19 (s, 9 H), 1.37 (d, 3 H, J = 6.3 Hz), 1.80 (d, 1 H, J = 4.8 Hz), 3.18-3.37 (m, 3 H), 3.41 (s, 3H), 4.21-4.35 (m, 2 H), 5.77 (d, 1 H, J = 5.5 Hz), 5.86 (d, 1 H, J = 5.5 Hz), 6.89-6.94 (m, 1 H), 7.22-7.28 (m, 2 H).

### Example 9

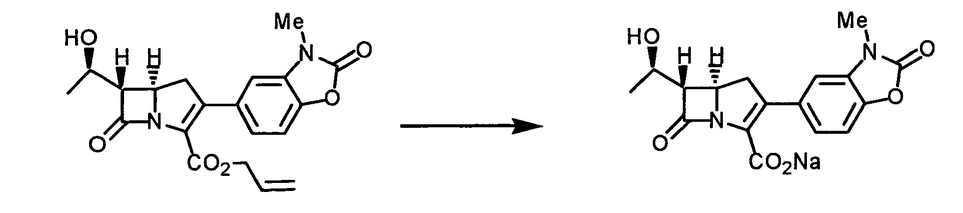

Allyl (5R,6S)-6-[(1R)-1-hydroxyethyl]-3-(2-oxo-3-methyl-2,3-dihydro-1,3-benzoxazol-6-yl)-7-oxo-1-azabicyclo[3.2.0]hept-2-ene-2-carboxylate (0.21g, 0.56mmol) obtained by referential example 28 and triphenylphosphine (15mg) were dissolved in THF (5ml) and thereto were added at 0°C sodium 2-ethylhexanoate (93mg, 0.56mmol) and tetrakis(triphenylphosphine)palladium(0) (32mg, 0.029mmol), followed by stirring for 20 minutes. A white solid occurred by addition of hexane (5ml) was filtered under nitrogen atmosphere, washed with hexane, and dried at room temperature in vacuo to give a crude product. The product was dissolved in a small amount of ice water and purified by 18 reverse column chromatography (filler: Wako Pure Chemical: Wakosil 40C18, mobile phase; 0 to 3% THF/ice-cooled ion-exchange water). The combined fraction containing the object compound was stirred for 1 hour at room temperature in vacuo to remove hexane. The residue was lyophilized to give sodium (5R,6S)-6-[(1R)-1-hydroxyethyl]-3-(2-oxo-3-methyl-2,3-dihydro-1,3-benzoxazol-6-yl)-7-oxo-1-azabicyclo[3.2.0]hept-2-ene-2-carboxylate (76mg, yield 37%).
¹H NMR (400 MHz, D₂O) δ 1.07 (d, 3 H, J = 6.0 Hz), 2.93 (dd, 1 H, J = 17.0, 9.8 Hz), 3.21 (s, 3H), 3.22-3.37 (m, 2 H), 4.03-4.18 (m, 2 H), 6.95-7.01 (m, 2 H), 7.04-7.10 (m, 1 H).

### Example 10

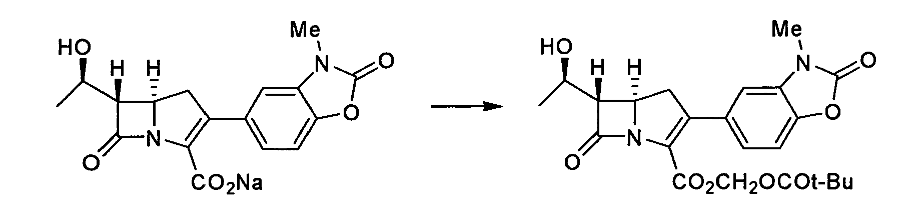

Sodium (5R,6S)-6-[(1R)-1-hydroxyethyl]-3-(2-oxo-3-methyl-2,3-dihydro-1,3-benzoxazol-6-yl)-7-oxo-1-azabicyclo[3.2.0]hept-2-ene-2-carboxylate (43mg) obtained by Example 9 was dissolved in dry DMF (1ml) and thereto was gradually dropped under ice-cooling pivaloyloxymethyl iodide (31mg), followed by stirring. One hour later, ethyl acetate was added thereto and the solution was washed with sodium hydrogen carbonate, water and brine. The organic layer was dried over anhydrous sodium sulfate and concentrated. The residue was purified by silica gel column chromatography (hexane : ethyl acetate = 2:1→1:2) to give (2,2-dimethylpropanoyl)oxymethyl(5R,6S)-6-[(1R)-1-hydroxyethyl]-3-(2-oxo-3-methyl-2,3-dihydro-1,3-benzoxazol-6-yl)-7-oxo-1-azabicyclo[3.2.0]hept-2-ene-2-carboxylate (33mg, yield 62%).
¹H NMR (400 MHz, CDCl₃) δ 1.15 (s, 9 H), 1.37 (d, 3 H, J = 6.3 Hz), 1.77 (d, 1 H, J = 4.8 Hz), 3.19-3.37 (m, 3 H), 3.42 (s, 3H), 4.23-4.37 (m, 2 H), 5.77 (d, 1 H, J = 5.5 Hz), 5.84 (d, 1 H, J = 5.5 Hz), 6.89-7.00 (m, 2 H), 7.17 (d, 1 H, J = 8.4 Hz).

### Example 11

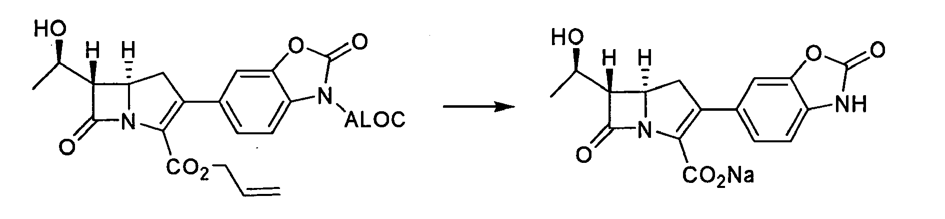

In accordance with the same method as Example 7 using allyl 6-{(5R,6S)-2-[(allyloxy)carbonyl]-6-[(1R)-1-hydroxyethyl]-7-oxo-1-azabicyclo[3.2.0]hept-2-ene-3-yl}-2-oxo-1,3-benzoxazol-3(2H)-carboxylate (0.69g) obtained by referential example 15, there was obtained sodium (5R,6S)-6-[(1R)-1-hydroxyethyl]-7-oxo-3-(2-oxo-2,3-dihydro-1,3-benzoxazol-6-yl)-1-azabicyclo[3.2.0]hept-2-ene-2-carboxylate (0.19g) as a white solid. ¹H NMR (400 MHz, DMSO- d₆) δ 1.09 (d, 3 H, J = 6.3 Hz), 2.82 (dd, 1 H, J = 9.8, 15.6 Hz), 3.00 (dd, 1 H, J = 8.5, 15.6 Hz), 3.05 (dd, 1 H, J = 2.8, 6.6 Hz), 3.80-3.88 (m, 1 H), 3.89-3.95 (m, 1 H), 4.92 (d, 1 H, J = 5.0 Hz), 6.84 (d, 1 H, J = 8.2 Hz), 7.10 (dd, 1 H, J = 1.6, 8.2 Hz), 7.49 (br.s, 1 H), 11.52 (br.s, 1 H). LCMS 331 (M-Na+1+). IR 1751, 1585, 1497, 1446, 1392, 1300, 1257, 1219, 1153, 1134, 1091, 933, 810, 756, 710 cm-1.

### Example 12

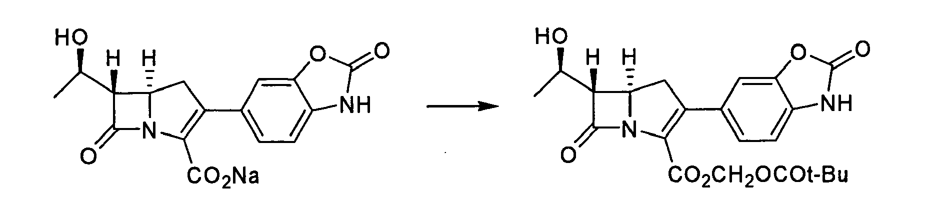

In accordance with the same method as Example 8 using sodium (5R,6S)-6-[(1R)-1-hydroxyethyl]-7-oxo-3-(2-oxo-2,3-dihydro-1,3-benzoxazol-6-yl)-1-azabicyclo[3.2.0]hept-2-ene-2-carboxylate (0.12g) obtained by Example 11, there was obtained [(2,2-dimethylpropanoyl)oxy]methyl (5R,6S)-6-[(1R)-1-hydroxyethyl]-7-oxo-3-(2-oxo-2,3-dihydro-1,3-benzoxazol-6-yl)-1-azabicyclo[3.2.0]hept-2-ene-2-carboxylate (0.10g) as a pale yellow amorphous.
¹H NMR (300 MHz, CDCl₃) δ 1.20 (s, 9 H), 1.38 (d, 3 H, J = 6.0 Hz), 1.92 (d, 1 H, J = 4.4 Hz), 3.18-3.38 (m, 3 H), 4.25-4.35 (m, 2 H), 5.79 (d, 1 H, J = 5.5 Hz), 5.88 (d, 1 H, J = 5.5 Hz), 7.03 (d, 1 H, J = 8.2 Hz), 7.19 (dd, 1 H, J = 1.6, 8.2 Hz), 7.26 (d, 1 H, J = 1.6 H), 8.35 (br.d, 1 H).

### Example 13

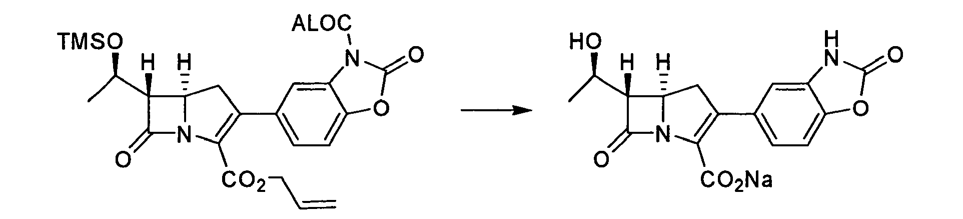

Allyl 5-((5R,6S)-2-[(allyloxy)carbonyl]-7-oxo-6-{(1R)-1-[(trimethylsilyl)oxy]ethyl}-1-azabicyclo[3.2.0]hept-2-ene-3-yl)-2-oxo-1,3-benzoxazol-3(2H)-carboxylate (0.79g) obtained by referential example 26 was dissolved in THF (13ml) and water (4ml) and to the solution in an ice bath was gradually dropped 1N hydrochloric acid with using a pH meter to adjust pH 2.5. Fifteen minutes later, thereto were added aqueous saturated sodium hydrogen carbonate solution (20ml) and saturated brine (30ml). The mixture was extracted with chloroform (30ml x 3). The organic layers were combined and dried over anhydrous sodium sulfate, filtered and concentrated to give allyl 5-((5R,6S)-2-[(allyloxy)carbonyl]-7-oxo-6-[(1R)-1-hydroxyethyl]-1-azabicyclo[3.2.0]hept-2-ene-3-yl)-2-oxo-1,3-benzoxazol-3(2H)-carboxylate (0.67g) as a pale yellow amorphous. By using this product and in accordance with the same method of Example 4, there was obtained sodium (5R,6S)-6-[(1R)-1-hydroxyethyl]-7-oxo-3-(2-oxo-2,3-dihydro-1,3-benzoxazol-5-yl)-1-azabicyclo[3.2.0]hept-2-ene-2-carboxylate (0.33g, yield 62%) as a white solid.
¹H NMR (400 MHz, DMSO- d₆) δ 1.09 (d, 3 H, J = 6.2 Hz), 2.82 (dd, 1 H, J = 9.9, 15.7 Hz), 3.03-3.10 (m, 2 H), 3.83-3.87 (m, 1 H), 3.92-3.98 (m, 1 H), 4.95 (br.s, 1 H), 6.92 (dd, 1 H, J = 1.8, 8.4 Hz), 7.00 (d, 1 H, J = 8.4 Hz), 7.37 (d, 1 H, J = 1.8 Hz), 12.32 (br.s, 1 H). LCMS 331 (M-Na+1+).

### Example 14

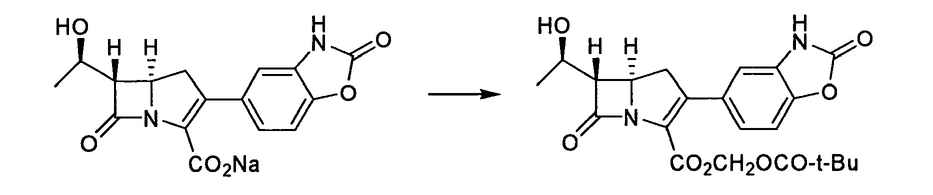

In accordance with the same method as Example 8 using sodium (5R,6S)-6-[(1R)-1-hydroxyethyl]-7-oxo-3-(2-oxo-2,3-dihydro-1,3-benzoxazol-5-yl)-1-azabicyclo[3.2.0]hept-2-ene-2-carboxylate (0.12g) obtained by Example 13, there was obtained [(2,2-dimethylpropanoyl)oxy]methyl (5R,6S)-6-[(1R)-1-hydroxyethyl]-7-oxo-3-(2-oxo2,3-dihydro-1,3-benzoxazol-5-yl-1-azabicyclo[3.2.0]hept-2-ene-2-carboxylic acid (0.10g) as a white amorphous.
¹H NMR (400 MHz, CDCl₃) δ 1.16 (s, 9 H), 1.36 (d, 3 H, J = 6.3 Hz), 2.67 (br.s, 1 H), 3.19-3.39 (m, 3 H), 4.24-4.35 (m, 2 H), 5.74 (d, 1 H, J = 5.6 Hz), 5.85 (d, 1 H, J = 5.6 Hz), 7.08 (dd, 1 H, J = 1.7, 8.4 Hz), 7.15 (d, 1 H, J = 8.4 Hz), 7.16 (d, 1 H, J = 1.7 H), 9.23 (br.d, 1 H). LCMS 445 (M+1+).

### Example 15

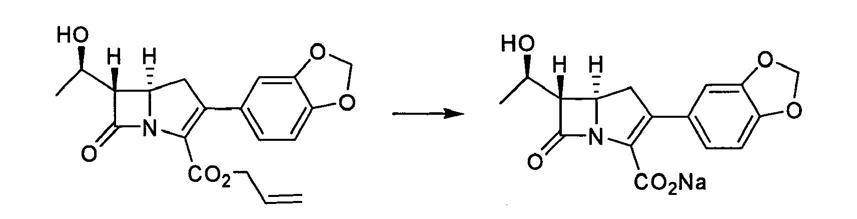

In accordance with the same method as Example 7 using allyl (5R,6S)-3-(1,3-benzodioxol-5-yl)-6-[(1R)-1-hydroxyethyl]-7-oxo-1-azabicyclo[3.2.0]hept-2-ene-2-carboxylate (0.77g) obtained by referential example 30, there was obtained sodium (5R,6S)-3-(1,3-benzoxol-5-yl)-6-[(1R)-1-hydroxyethyl]-7-oxo-1-azabicyclo[3.2.0]hept-2-ene-2-carboxylate (0.50g, yield 73%) as a white solid.
¹H NMR (400 MHz, DMSO- d₆) δ1.15 (d, 3 H, J = 6.2 Hz), 2.88 (dd, 1 H, J = 9.8, 15.8 Hz), 3.04 (dd, 1 H, J = 8.5, 15.8 Hz), 3.12 (dd, 1 H, J = 2.8, 6.9 Hz), 3.89-3.95 (m, 1 H), 3.98-4.01 (m, 1 H), 5.95-5.96 (m, 2 H), 6.79 (d, 1 H, J = 8.1 Hz), 6.86 (dd, 1 H, J = 1.7, 8.1 Hz), 7.33 (d, 1 H, J = 1.7 Hz).

### Example 16

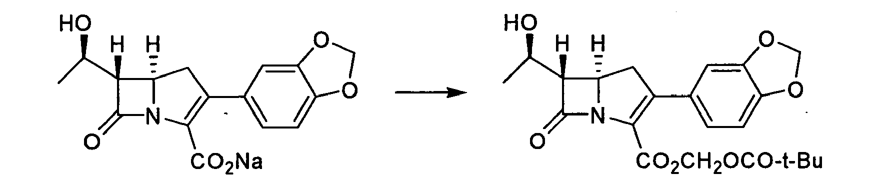

In accordance with the same method as Example 8 using sodium (5R,6S)-3-(1,3-benzodioxol-5-yl)-6-[(1R)-1-hydroxyethyl]-7-oxo-1-azabicyclo[3.2.0]hept-2-ene-2-carboxylate (0.13 g) obtained by Example 15, there was obtained [(2,2-dimethylpropanoyl)oxy]methyl (5R,6S)-3-(1,3-benzodioxol-5-yl)-6-[(1R)-1-hydroxyethyl]-7-oxo-1-azabicyclo[3.2.0]hept-2-ene-2-carboxylate (0.08g, yield 48%) as a pale yellow amorphous.
¹H NMR (400 MHz, CDCl₃) δ 1.20 (s, 9 H), 1.36 (d, 3 H, J = 6.3 Hz), 3.15-3.31 (m, 3 H), 4.22-4.29 (m, 2 H), 5.79 (d, 1 H, J = 5.5 Hz), 5.88 (d, 1 H, J = 5.5 Hz), 5.99 (s, 2 H), 6.78 (d, 1 H, J = 8.0 Hz), 6.86-6.89 (m, 2 H). LCMS 432.

### Example 17

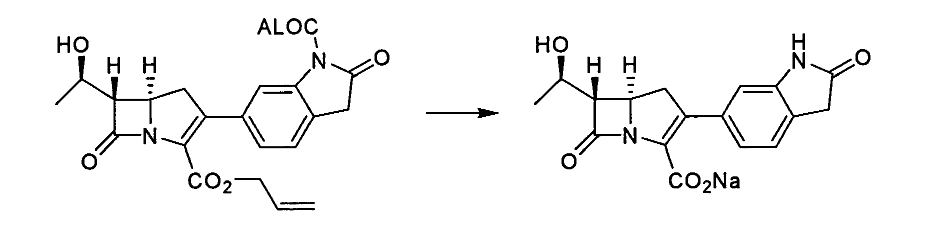

In accordance with the same method as Example 7 using allyl 6-{(5R,6S)-2-[(allyloxy)carbonyl]-6-[(1R)-1-hydroxyethyl]-7-oxo-1-azabicyclo[3.2.0]hept-2-ene-3-yl}-2-oxo-indoline-1-carboxylate (0.26g) obtained by referential example 33, there was obtained sodium (5R,6S)-6-[(1R)-1-hydroxyethyl]-7-oxo-3-(2-oxo-2,3-dihydro-1H-indol-6-yl)-1-azabicyclo[3.2.0]hept-2-ene-2-carboxylate (0.11g, yield 64%) as a white solid.
¹H NMR (400 MHz, DMSO- d₆) δ 1.09 (d, 3 H, J = 6.3 Hz), 2.76 (dd, 1 H, J = 9.8, 15.5 Hz), 2.99 (dd, 1 H, J = 8.5, 15.5 Hz), 3.07 (dd, 1 H, J = 2.8, 6.6 Hz), 3.31 (s, 2 H), 3.80-3.88 (m, 1 H), 3.90-3.95 (m, 1 H), 4.91 (d, 1 H, J = 5.0 Hz), 6.87 (dd, 1 H, J = 1.5, 7.7 Hz), 6.96 (d, 1 H, J = 7.7 Hz), 7.02 (d, 1 H, J = 1.5 Hz), 10.27 (br.s, 1 H).

### Example 18

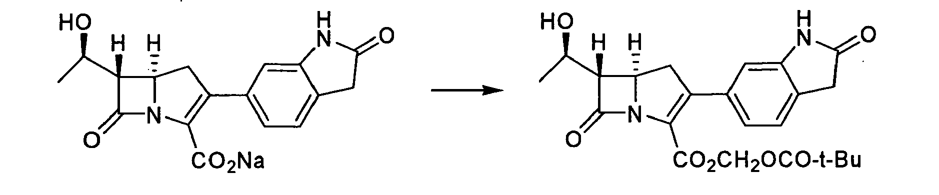

In accordance with the same method as Example 8 using sodium (5R,6S)-6-[(1R)-1-hydroxyethyl]-7-oxo-3-(2-oxo-2,3-dihydro-1H-indol-6-yl)-1-azabicyclo[3.2.0]hept-2-ene-2-carboxylate (0.07g) obtained by Example 17, there was obtained [(2,2-dimethylpropanoyl)oxy]methyl (5R,6S)-6-[(1R)-1-hydroxyethyl]-7-oxo-3-(2-oxo-2,3-dihydro-1H-indol-6-yl)-1-azabicyclo[3.2.0]hept-2-ene-2-carboxylate (0.06g, yield 63%) as a pale yellow oil.
¹H NMR (400 MHz, CDCl₃) δ 1.18 (s, 9 H), 1.37 (d, 3 H, J = 6.3 Hz), 3.17-3.36 (m, 3 H), 3.53 (s, 2 H), 4.23-4.33 (m, 2 H), 5.76 (d, 1 H, J = 5.5 Hz), 5.86 (d, 1 H, J = 5.5 Hz), 6.92 (d, 1 H, J = 1.6 Hz), 6.97 (dd, 1 H, J = 1.6, 7.7 Hz), 7.20 (d, 1 H, J = 7.7 Hz), 8.22 (br.s, 1 H).

### Referential example 1

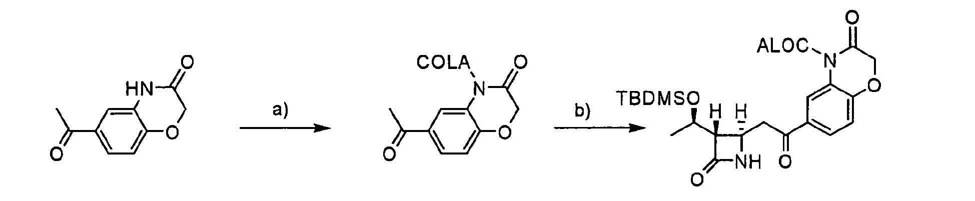

### Step a)

To a solution of 6-acetyl-2H-1,4-benzoxazin-3(4H)-one (1.91g, 10mmol) in DMF (30ml) was added under ice-cooling 60% sodium hydride (480mg, 12mmol). Thereto was dropped under ice-cooling allyl chhloroformate (1.45g, 12mmol) and the mixture was stirred at room temperature for 1 hour. After the reaction was quenched by addition of acetic acid (601mg, 10mmol), thereto was added a previously cooled 5% potassium hydrogen sulfate, and the mixture was extracted with ethyl acetate and separated by a separating funnel. The organic layer was washed by saturated brine, saturated sodium hydrogen carbonate solution and saturated brine in three times in that order and dried over anhydrous magnesium sulfate, filtered and concentrated. The residue was purified by silica gel column chromatography (silica gel 60g, hexane/ethyl acetate) to give allyl 6-acetyl-3-oxo-2,3-dihydro-4H-1,4-benzoxazin-4-carboxylate (2.057g, yield 74.7%) as an oil.
¹H NMR (400 MHz, CDCl₃) δ 2.57 (s, 3 H), 4.65 (s, 2 H), 4.90-4.95 (m, 2H), 5.36-5.42 (m, 1 H), 5.47-5.55 (m, 1 H), 5.98-6.09 (m, 1 H), 7.12 (d, 1 H, J = 8.4Hz), 7.74 (dd, 1 H, J = 2.0, 8.4 Hz), 7.97 (d, 1 H, J = 1.9Hz).

### Step b)

To a solution of allyl 6-acetyl-3-oxo-2,3-dihydro-4H-1,4-benzoxazin-4-carboxylate (2.04g, 7mmol) obtained by step b and triethylamine (1.14ml, 8mmol) in methylene chloride (30ml) was dropped at 40°C trifluoromethane sulfonic acid trimethylsilyl ester (1.34ml, 8mmol) and the mixture was at ice-cooling for 20 minutes stirred. Thereto were added at ice-cooling (2R,3R)-3-((1R)-1-{[tert-butyl(dimethyl)silyl]oxy}ethyl)-4-oxo-2-azetidinylacetate (2.13g, 7mmol) and zinc iodide (1.42g, 4mmol), followed by stirring for 1 hour and 20 minutes. The reaction mixture was extracted with ethyl acetate and saturated brine, separated by a separating funnel, dried over anhydrous sodium sulfate, filtered and concentrated. The residue was purified by silica gel column chromatography (silica gel 50g, ethyl acetate : hexane = 1:2→1:1) to give allyl 6-{[(2R,3S)-3-((1R)-1-{[tert-butyl(dimethyl)silyl]oxy}ethyl)-4-oxoazetidin-2-yl]acetyl}-3-oxo-2,3-dihydro-4H-1,4-benzoxazin-4-carboxylate (3.6g, yield 96%).
¹H NMR (400 MHz, CDCl₃) δ 0.02-0.13 (m, 6 H), 0.87 (s, 9 H), 1.25 (d, 3 H, J = 5.4 Hz), 2.85-2.91 (m, 1 H), 3.07-3.16 (m, 1 H), 3.36-3.44 (m, 1 H), 4.07-4.14 (m, 1 H), 4.18-4.25 (m, 1 H), 4.66 (s, 2 H), 4.90-4.95 (m, 2 H), 5.34-5.41 (m, 1 H), 5.47-5.54 (m, 1 H), 5.94-6.12 (m, 2 H), 7.14 (d, 1 H, J = 8.5 Hz), 7.72 (d, 1 H, J = 8.5 Hz), 8.00 (s, 1 H).

### Referential example 2

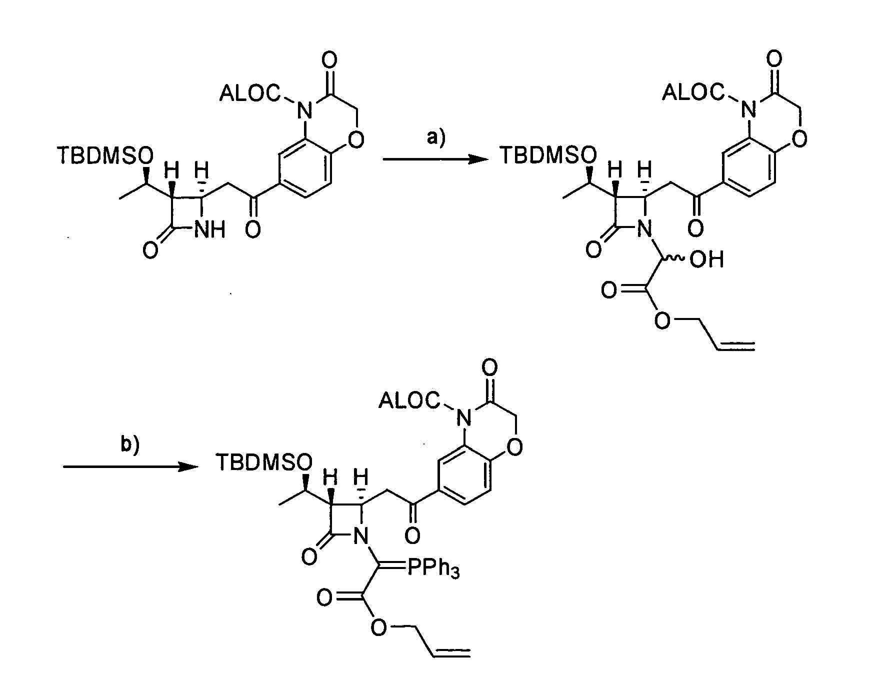

### Step a)

After to a solution of allyl 6-{[(2R,3S)-3-((1R)-1-{[tert-butyl(dimethyl)silyl]oxy}ethyl)-4-oxoazetidin-2-yl]acetyl}-3-oxo-2,3-dihydro-4H-1,4-benzoxazin-4-carboxylate (3.6g, 7mmol) obtained by referential example 1 in toluene (40ml) was dropped at room temperature allyl dihydroxyacetate (1.41g, 11mmol), the mixture was stirred for 2 hours while azeotropically dehydrating at 120°C with a Dean-Stark evaporator. The reaction mixture was concentrated to give allyl {(2R,3S)-2-[2-(4-allyloxycarbonyl-3-oxo-2,3-dihydro-4H-1,4-benzoxazin-6-yl)-2-oxoethyl]-3-(1R)-1-{[tert-butyl(dimethyl)silyl]oxy}ethyl-4-oxoazetidin-1-yl}(hydroxyl)acetate (5.33g). This product was used in the following reaction without purification.

### Step b)

To a solution of allyl {(2R,3S)- 2-[2-(4-allyloxycarbonyl-3-oxo-2,3-dihydro-4H-1,4-benzoxazin-6-yl)-2-oxoethyl]-3-(1R)-1-{[tert-butyl(dimethyl)silyl]oxy}ethyl-4-oxoazetidin-1-yl}(hydroxyl)acetate (5.33g) obtained by step a) in THF (40ml) were added at -20°C 2,6-lutidine (1.32ml, 11mmol) and thionyl chloride (0.73ml, 10mmol), and thereto was added at room temperature THF (20ml), followed by stirring for 1 hour. The reaction mixture was filtered, concentrated and the residue was dissolved in 1,4-dioxane (40ml). To the solution were added at room temperature 2,6-lutidine (1.82ml, 16mmol) and triphenylphosphine (3.35g, 13mmol), followed by stirring at 50°C for 3 hours. To the reaction mixture was added a saturated sodium hydrogen carbonate solution and the mixture was extracted with ethyl acetate, and separated by a separating a funnel. The organic layer was washed with saturated brine (three times), dried over anhydrous sodium sulfate, filtered and concentrated. The residue was purified by silica gel column chromatography (silica gel 150g, ethyl acetate : hexane = 1:5→3:1) to give allyl {(2R,3S)- 2-[2-(4-allyloxycarbonyl-3-oxo-2,3-dihydro-4H-1,4-benzoxazin-6-yl)-2-oxoethyl]-3-(1R)-1-{[tert-butyl(dimethyl)silyl]oxy}ethyl-4-oxoazetidin-1-yl}(triphenylphosphoanilidene)acetate (2.56g, yield 42%).
¹H NMR (400 MHz, CDCl₃) δ -0.15-0.11 (m, 6 H), 0.70-0.85 (m, 9 H), 1.10 (d, 3 H, J = 6.0 Hz), 2.85-3.01 (m, 2 H), 3.51-3.59 (m, 1 H), 3.78-4.05 (m, 2 H), 4.37-4.48 (m, 1 H), 4.62-4.71 (m, 1 H), 4.88-5.04 (m, 3 H), 5.06-5.56 (m, 4 H), 5.73-6.11 (m, 2 H), 7.11-7.19 (m, 1 H), 7.38-7.88 (m, 15 H), 7.89-8.05 (m, 2 H)

### Referential example 3

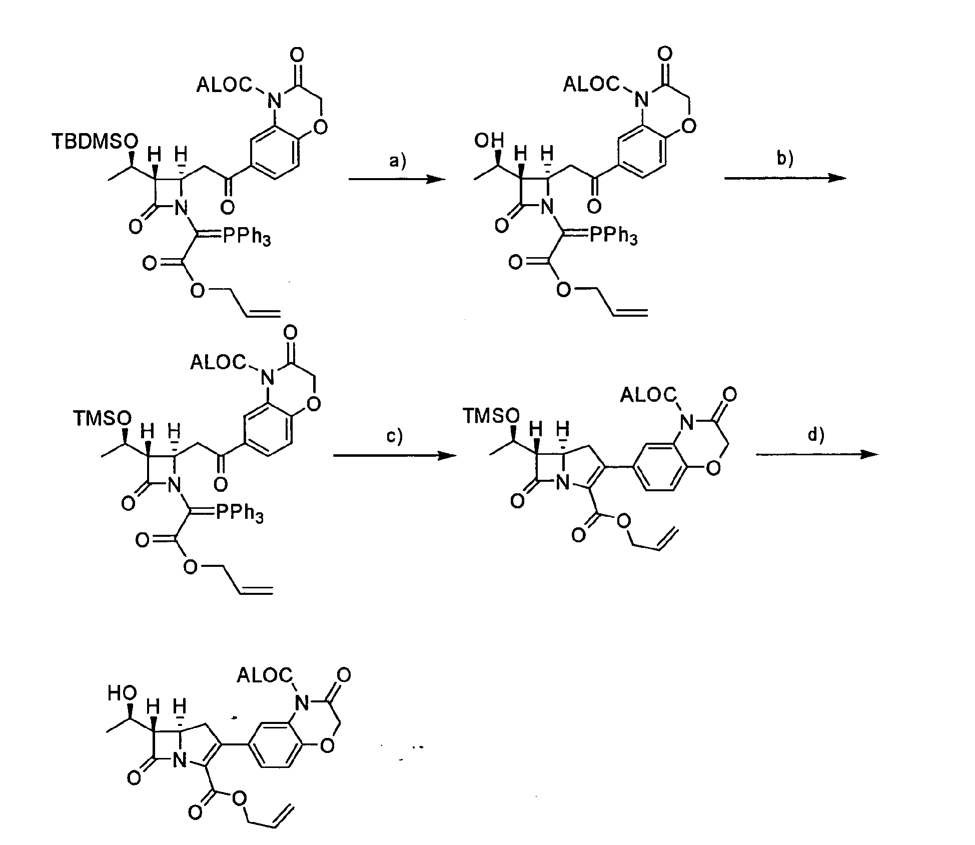

### Step a)

Allyl {(2R,3S)- 2-[2-(4-allyloxycarbonyl-3-oxo-2,3-dihydro-4H-1,4-benzoxazin-6-yl)-2-oxoethyl]-3-(1R)-1-{[tert-butyl(dimethyl)silyl]oxy}ethyl-4-oxoazetidin-1-yl}(triphenylphosphoanilidene)acetate (2.56g) obtained by referential example 2 was dissolved at room temperature in 70% aqueous trifluoroacetic acid solution (20ml). After addition of ethyl acetate (100ml), the mixture was washed with saturated brine (100ml x 2) and an aqueous saturated sodium hydrogen carbonate (100ml x 2), dried over anhydrous sodium sulfate, filtered and concentrated to give allyl {(2R,3S)-2-[2-(4-allyloxycarbonyl-3-oxo-2,3-dihydro-4H-1,4-benzoxazin-6-yl)-2-oxoethyl]-3-((1R)-1-hydroxyethyl)-4-oxoazetidin-1-yl}(triphenylphosphoanilidene)acetate (2.35g) as a pale yellow amorphous. This product was used in the following reaction without purification.

### Step b)

Allyl {(2R,3S)- 2-[2-(4-allyloxycarbonyl-3-oxo-2,3-dihydro-4H-1,4-benzoxazin-6-yl)-2-oxoethyl]-3-((1R)-1-hydroxyethyl)-4-oxoazetidin-1-yl}(triphenylphosphoanilidene)acetate (2.35g) obtained by step a) and triethylamine (2.15ml, 15mmol) were dissolved in THF (20ml). Thereto was added at 0°C chlorotrimethyl silane (1.17ml, 8.9mmol), followed by stirring for 30 minutes. After addition of ethyl acetate, the mixture was washed with an aqueous saturated sodium hydrogen carbonate solution (50ml) and saturated brine (100ml), dried over anhydrous sodium sulfate, filtered and concentrated to give allyl {(2R,3S)- 2-[2-(4-allyloxyoxycarbonyl-3-oxo-2,3-dihydro-4H-1,4-benzoxazin-6-yl)-2-oxoethyl]-3-(1R)-1-[(trimethylsilyl)oxy]ethyl-4-oxoazetidin-1-yl}(triphenylphosphoanilidene)acetate (2.46 g) as a yellow oil. This product was used in the following reaction without purification.

### Step c)

Allyl (2R,3S)- 2-[2-(4-allyloxycarbonyl-3-oxo-2,3-dihydro-4H-1,4-benzoxazin-6-yl)-2-oxoethyl]-3-{(1R)-1-[(trimethylsilyl)oxy]ethyl}-4-oxoazetidin-1-yl}(triphenylphosphoanilidene)acetate obtained by step b) was dissolved in toluene (50ml), and thereto was added N,O-bistrimethylsilylacetamide (1.5ml), followed by refluxing for 4 hours. After being cooled, the reaction mixture was concentrated and the residue was purified by silica gel column chromatography (silica gel 100g, ethyl acetate : hexane = 1:1→2:1) to give allyl (5R,6S)-3-(4-allyloxycarbonyl-3-oxo-2,3-dihydro-4H-1,4-benzoxazin-6-yl)-7-oxo-6-{(1R)-1-[(trimethylsilyl)oxy]ethyl}-1-azabicyclo[3.2.0]hept-2-ene-2-carboxylate (0.45 g, yield 28%) as a pale yellow oil.
¹H NMR (400 MHz, CDCl₃) δ 0.14 (s, 9 H), 1.29 (d, 3 H, J = 6.2 Hz), 3.12-3.31 (m, 3 H), 4.15-4.25 (m, 2 H), 4.56-4.75 (m, 4 H), 4.88-4.93 (m, 2 H), 5.16-5.21 (m, 1 H), 5.29-5.39 (m, 2 H), 5.44-5.52 (m, 1 H), 5.82-6.08 (m, 2 H), 7.24 (d, 1 H, J = 8.4 Hz), 7.15 (d, 1 H, J = 8.4 Hz) 7.45 (s, 1 H).

### Step d)

Allyl (5R,6S)-3-(4-allyloxycarbonyl-3-oxo-2,3-dihydro-4H-1,4-benzoxazin-6-yl)-7-oxo-6-{(1R)-1-[(trimethylsilyl)oxy]ethyl}-1-azabicyclo[3.2.0]hept-2-ene-2-carboxylate (0.45g, 0.83mmol) obtained by step c) was dissolved in THF (20ml) and water (2ml). To the solution was gradually dropped 1N hydrochloric acid using a pH meter to adjust pH 2.5. Ten minutes later, a saturated sodium hydrogen carbonate solution (20ml) and saturated brine (20ml) were added thereto. After extracted with ethyl acetate (20ml) the organic layer was dried over anhydrous sodium sulfate, filtered and concentrated to give allyl (5R,6S)-3-(4-allyloxycarbonyl-3-oxo-2,3-dihydro-4H-1,4-benzoxazin-6-yl)-7-oxo-6-{(1R)-1-hydroxyethyl}-1-azabicyclo[3.2.0]hept-2-ene-2-carboxylate (0.36 g, 0.67mmol, yield 93%) as a pale yellow oil.
¹H NMR (400 MHz, CDCl₃) δ 1.37 (d, 3 H, J = 6.3 Hz), 1.74 (br.s, 1 H), 3.17-3.31 (m, 3 H), 4.22-4.34 (m, 2 H), 4.54-4.78 (m, 3 H), 4.86-4.91 (m, 2 H), 5.20-5.54 (m, 4 H), 5.82-6.07 (m, 2 H), 7.03 (d, 1 H, J = 8.4 Hz), 7.15 (d, 1 H, J = 8.4 Hz), 7.51 (s, 1 H).

### Referential example 4

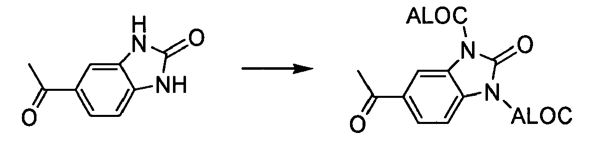

To a solution of 5-acetyl-1,3-dihydro-2H-benzimidazole-2-one (0.785 g, 4.5mmol) in DMF (30ml) was added at ice cooling 60% sodium hydride (374mg, 9.4mmol) and thereto was dropped under ice cooling allyl chloroformate (1.07g, 8.9mmol). The mixture was stirred at room temperature for 1 hour. To the reaction mixture was added a previously cooled 5% aqueous potassium hydrogen sulfate solution and the mixture was extracted with ethyl acetate and separated by a separating funnel.

The organic layer was washed with brine (3 times), dried over anhydrous sodium sulfate, filtered and concentrated. The residue was purified by silica gel column chromatography (silica gel 40g, ethyl acetate : hexane = 1:3→1:1) to give diallyl 5-acetyl-2-oxo-1H-benzimidazole-1,3(2H)-dicarboxylate (1.26g, yield 83%).
¹H NMR (400 MHz, CDCl₃) δ 2.63 (s, 3 H), 4.94-5.02 (m, 4 H), 5.38-5.44 (m, 2 H), 5.54-5.63 (m, 2 H), 6.03-6.14 (m, 2 H), 7.92 (d, 1 H, J = 8.6 Hz), 8.03 (d, 1 H, J = 8.6 Hz), 8.56 (s, 1H).

### Referential example 5

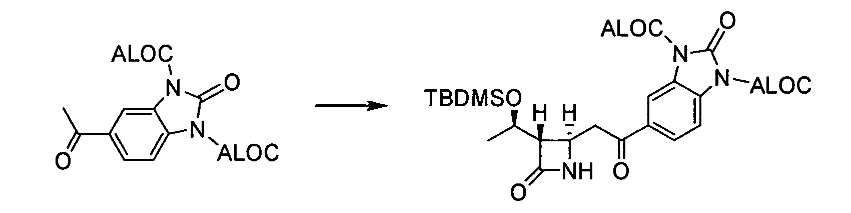

To a solution of diallyl 5-acetyl-2-oxo-1H-benzimidazole-1,3(2H)-dicarboxylate (1.16g, 3.4mmol) obtained by referential example 4 and triethylamine (0.38g, 3.7mmol) in methylene chloride (20ml) was dropped at 0°C trifluoromethane sulfonic acid trimethylsilyl ester (0.75g, 3.4mmol), followed by stirring for 20 minutes. Thereto were dropped at 0°C triethylamine (0.38g, 3.7mmol) and trifluoromethane sulfonic acid trimethylsilyl ester (0.75g, 3.4mmol), followed by stirring for 15 minutes. To the reaction mixture were added under ice-cooling (2R,3R)-3-((1R)-1-{[tert-butyl(dimethyl)silyl]oxy}ethyl)-4-oxo-2-azetidinyl acetate (0.97 g, 3.4mmol) and zinc iodide (1.3g, 4.0mmol), followed by stirring at room temperature for 5 hours. The reaction mixture was extracted with ethyl acetate and saturated brine, and separated, dried over anhydrous sodium sulfate, filtered and concentrated. The residue was purified by silica gel column chromatography (silica gel 50g, ethyl acetate : hexane = 1:4→1:1) to give diallyl 5-{[(2R,3S)-3-((1R)-1-{[tert-butyl(dimethyl)silyl]oxy}ethyl)-4-oxoazetidin-2-yl]acetyl}-2-oxo-1H-benzimidazole-1,3(2H)-dicarboxylate (1.5g, yield 73%).
¹H NMR (400 MHz, CDCl₃) δ 0.08 (s, 6 H), 0.88 (s, 9 H), 1.25 (d, 3 H, J = 6.2 Hz), 2.85-2.92 (m, 1 H), 3.11-3.23 (m, 1 H), 3.43-3.54 (m, 1 H), 4.07-4.16 (m, 1 H), 4.19-4.27 (m, 1 H), 4.91-4.97 (m, 4 H), 5.33-5.42 (m, 2 H), 5.54-5.62 (m, 2 H), 5.95-6.12 (m, 3 H), 7.91 (d, 1 H, J = 8.6 Hz), 8.06 (d, 1 H, J = 8.6 Hz), 8.55 (s, 1 H).

### Referential example 6

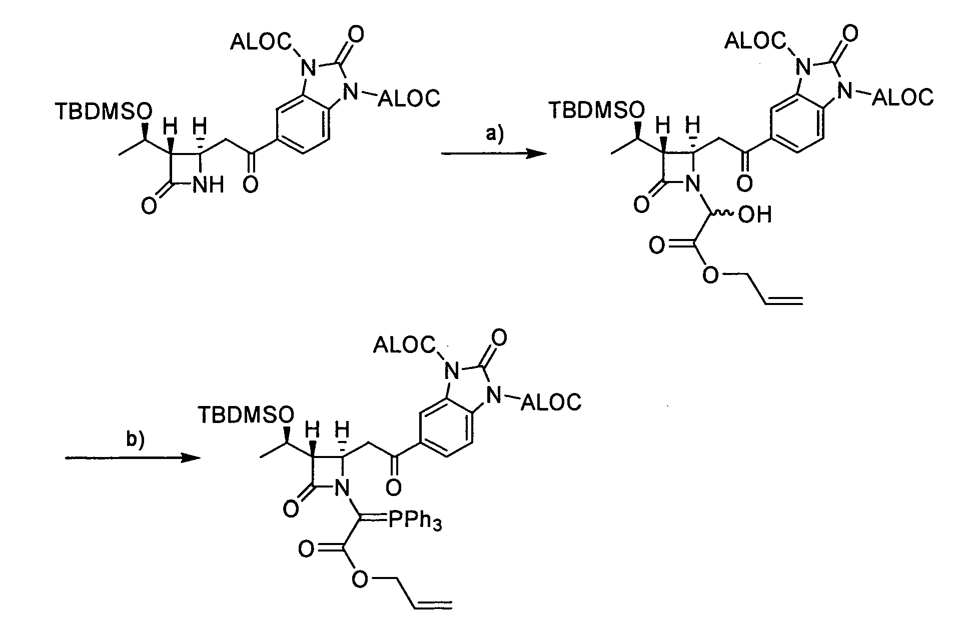

### Step a)

To a solution of diallyl 5-{[(2R,3S)-3-((1R)-1-{[tert-butyl(dimethyl)silyl]oxy}ethyl)-4-oxoazetidin-2-yl]acetyl}-2-oxo-1H-benzimidazole-1,3(2H)-dicarboxylate (1.53g, 2.7mmol) obtained by referential example 5 in toluene (35ml) was dropped at room temperature allyl dihydroxyacetate (0.53g, 4.0mmol), and the reaction mixture was stirred at 120°C for 2 hours while azeotropically dehydrating using Dean-Stark evaporator. The reaction mixture was concentrated to give allyl (2R,3S)- 2-{2-(1,3-diallyloxydicarbonyl-2-oxo-2,3-dihydro-1H-benzimidazol-5-yl)-2-oxoethyl}-3-((1R)-1-{[tert-butyl(dimethyl)silyl]oxy}ethyl)-4-oxoazetidin-1-yl}(hydroxyl)acetate. This product was used in following reaction without purification.

### Step b)

To a solution of allyl {(2R,3S)-2-{2-(1,3-diallyloxycarbonyl-2-oxo-2,3-dihydro-1H-benzimidazol-5-yl)-2-oxoethyl}-3-((1R)-1-{[tert-butyl(dimethyl)silyl]oxy}ethyl)-4-oxoazetidin-1-yl}(hydroxyl)acetate obtained in step a) in THF (30ml) were added at -20°C 2,6-lutidine (0.46 g, 4.3mmol) and thionyl chloride (0.45 g, 3.8mmol), and thereto was added at room temperature THF (15ml), followed by stirring for 1 hour. The reaction mixture was filtered, the filtrate was concentrated and the residue was dissolved in 1,4-dioxane (30ml). Thereto were added at room temperature 2,6-lutidine (0.63g, 5.9mmol) and triphenylphosphine (1.27g, 4.8mmol), followed by stirring at 50°C for 3 hours. After addition of an aqueous saturated sodium hydrogen carbonate solution, the mixture was extracted with ethyl acetate, and separated by a separating funnel. The organic layer was washed with saturated brine (3 times), dried over anhydrous sodium sulfate, filtered and concentrated. The residue was purified by silica gel column chromatography (silica gel 150g, ethyl acetate : hexane = 1:5→1:1) to give allyl {(2R,3S)-2-{2-(1,3-diallyloxycarbonyl-2-oxo-2,3-dihydro-1H-benzimidazol-5-yl)-2-oxoethyl}-3-((1R)-1-{[tert-butyl(dimethyl)silyl]oxy}ethyl)-4-oxoazetidin-1-yl}(triphenylphosphoanilidene)acetate (1.48 g, yield 59%).
¹H NMR (400 MHz, CDCl₃) δ -0.15-0.08 (m, 6 H), 0.72-0.79 (m, 9 H), 1.06-1.12 (m, 3 H), 2.62-2.79 (m, 1 H), 2.86-3.04 (m, 2 H), 3.41-3.57 (m, 1 H), 3.79-3.98 (m, 1 H), 4.88-4.99 (m, 6 H), 5.31-5.39 (m, 3 H), 5.52-5.62 (m, 3 H), 5.96-6.13 (m, 3 H), 7.35-7.80 (m, 15 H), 7.98-8.05 (m, 2 H), 8.58-8.64 (m, 1 H)

### Referential example 7

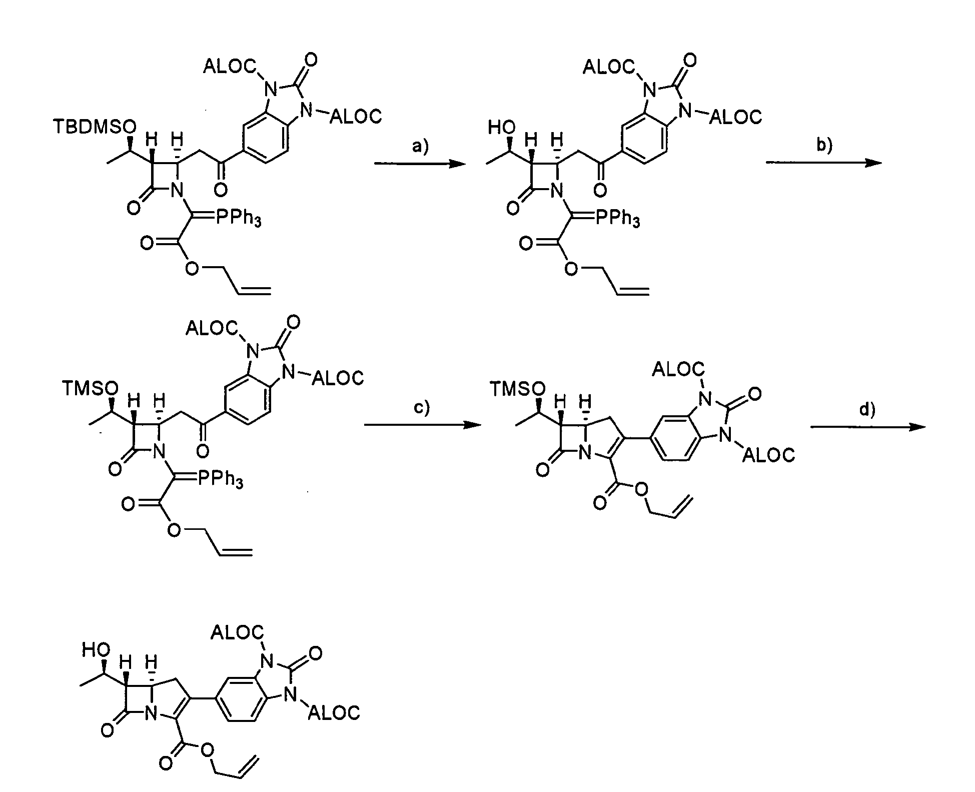

### Step a)

Allyl {(2R,3S)-2-{2-(1,3-diallyloxycarbonyl-2-oxo-2,3-dihydro-1H-benzimidazol-5-yl)-2-oxoethyl}-3-((1R)-1-{[tert-butyl(dimethyl)silyl]oxy}ethyl)-4-oxoazetidin-1-yl}(triphenylphosphoanilidene)acetate (1.48 g) obtained by referential example 6 was dissolved at room temperature in 70% aqueous trifluoroacetic acid solution (15ml). After addition of ethyl acetate (100ml) the reaction mixture was washed with saturated brine (100ml x 2) and an aqueous sodium hydrogen carbonate solution (100ml x 2), dried over anhydrous sodium sulfate, filtered and concentrated to give allyl {(2R,3S)-2-{2-(1,3-diallyloxycarbonyl-2-oxo-2,3-dihydro-1H-benzimidazol-5-yl)-2-oxoethyl}-3-((1R)-1-hydroxyethyl)-4-oxoazetidin-1-yl}(triphenylphosphoanilidene)acetate as a pale yellow amorphous. This product was used in the following reaction without purification.

### Step b)

To a solution of allyl {(2R,3S)-2-{2-(1,3-diallyloxycarbonyl-2-oxo-2,3-dihydro-1H-benzimidazol-5-yl)-2-oxoethyl}-3-((1R)-1-hydroxyethyl)-4-oxoazetidin-1-yl}(triphenylphosphoanilidene)acetate obtained in step b) and triethylamine (1.6g, 16mmol) in THF (50ml) was added at 0°C chlorotrimethyl silane (1.2g, 1 1mmol), followed by stirring for 30 minutes. After addition of ethyl acetate, the reaction mixture was washed with an aqueous saturated sodium hydrogen carbonate solution (50ml) and saturated brine (100ml), dried over anhydrous sodium sulfate, filtered and concentrated. The residue was purified by silica gel column chromatography (silica gel 30g, ethyl acetate : hexane = 1:4→2:1) to give allyl {(2R,3S)-2-{2-(1,3-diallyloxycarbonyl-2-oxo-2,3-dihydro-1H-benzimidazol-5-yl)-2-oxoethyl}-3-((1R)-1-[(trimethylsilyl)oxy]ethyl)-4-oxoazetidin-1-yl}(triphenylphosphoanilidene)acetate (1.1 g, yield 78%). Step c)

To a solution of allyl {(2R,3S)-2-{2-(1,3-diallyloxycarbonyl-2-oxo-2,3-dihydro-1H-benzimidazol-5-yl)-2-oxoethyl}-3-((1R)-1-[(trimethylsilyl)oxy]ethyl)-4-oxoazetidin-1-yl}(triphenylphosphoanilidene)acetate (1.1 g) obtained in step b) in toluene (50ml) was added N,O-bis trimethylsilylacetamide (0.50 g), followed by refluxing for 10 hours. After being cooled the reaction mixture was concentrated and the residue was purified by silica gel column chromatography (silica gel 30g, ethyl acetate hexane = 1:4→ 1:3) to give allyl (5R,6S)-3-(1,3-diallyloxycarbonyl-2-oxo-2,3-dihydro-1H-benzimidazol-5-yl)-7-oxo-6-{(1R)-1-[(trimethylsilyl)oxy]ethyl}-1-azabicyclo[3.2.0]hept-2-ene-2-carboxylate (0.62 g, yield 82%) as a pale yellow oil.
¹H NMR (400 MHz, CDCl₃) δ 0.14 (s, 9 H), 1.28 (d, 3 H, J = 7.0 Hz), 3.14-3.35 (m, 3 H), 4.17-4.24 (m, 2 H), 4.59-4.75 (m, 2 H), 4.91-4.98 (m, 4 H), 5.13-5.18 (m, 1 H), 5.26-5.41 (m, 3 H), 5.49-5.58 (m, 2 H), 5.81-5.90 (m, 1 H), 5.97-6.07 (m, 2 H), 7.28-7.33 (m, 1 H), 7.90 (d, 1 H, J = 8.5 Hz) 8.02 (s, 1 H).

### Step d)

Allyl (5R,6S)-3-(1,3-diallyloxycarbonyl-2-oxo-2,3-dihydro-1H-benzimidazol-5-yl)-7-oxo-6-{(1R)-1-[(trimethylsilyl)oxy]ethyl}-1-azabicyclo[3.2.0]hept-2-ene-2-carboxylate (0.62 g, 1.0mmol) obtained in step c) was dissolved in THF (45ml) and water (5ml), and thereto was gradually dropped 1N hydrochloric acid under cooling in an ice bath using a pH meter to adjust pH 2.5. Ten minutes later, an aqueous saturated sodium hydrogen carbonate solution (30ml) and saturated brine (30ml) were added thereto, and the reaction mixture was extracted with ethyl acetate (30ml). The organic layer was dried over anhydrous sodium sulfate, filtered and concentrated to give allyl (5R,6S)-3-(1,3-diallyloxycarbonyl-2-oxo-2,3-dihydro-1H-benzimidazol-5-yl)-7-oxo-6-{(1R)-1-hydroxyethyl}-1-azabicyclo[3.2.0]hept-2-ene}-2-carboxylate (0.56g, 1.0mmol, quantitative yield) as a pale yellow oil.
¹H NMR (400 MHz, CDCl₃) δ 1.38 (d, 3 H, J = 6.3 Hz), 1.79 (br.s, 1 H), 3.19-3.38 (m, 3 H), 4.22-4.37 (m, 2 H), 4.55-4.67 (m, 1 H), 4.71-4.79 (m, 1 H), 4.91-4.98 (m, 4 H), 5.14-5.20 (m, 1 H), 5.24-5.44 (m, 3 H), 5.52-5.61 (m, 2 H), 5.81-5.91 (m, 1H), 5.97-6.08 (m, 2 H), 7.28-7.32 (m, 1 H), 7.91 (d, 1 H, J = 8.5 Hz), 8.03 (s, 1 H).

### Referential example 8

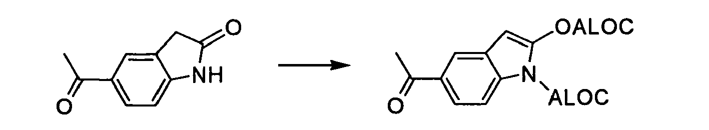

To a solution of 1,3-dihydro-2H-indole-2-one (6.30g, 36mmol) in DMF (100ml) was added under ice-cooling 60%sodium hydride (3.0g, 76mmol). Thereto was dropped under ice-cooling allyl chloroformate (8.7g, 72mmol), followed by stirring at room temperature for 3 hours. To the reaction mixture was added a previously cooled 5% aqueous potassium hydrogen sulfate solution and the mixture was extracted with ethyl acetate and separated by a separating funnel. The organic layer was washed with brine (3 times), dried over anhydrous sodium sulfate, filtered and concentrated. The residue was purified by silica gel column chromatography (silica gel 40g, ethyl acetate : hexane = 1:4→1:3) to give allyl 5-acetyl-2-(allyloxycarbonyloxy)-1H-indole-1-carboxylate (3.96g, yield 32%).
¹H NMR (400 MHz, CDCl₃) δ 2.67 (s, 3 H), 4.76-4.79 (m, 2 H), 4.91-4.93 (m, 2 H), 5.36-5.48 (m, 4 H), 5.97-6.04 (m, 2 H), 6.43 (s, 1 H), 7.94-7.97 (m, 1 H), 8.14-8.17 (m, 2 H).

### Referential example 9

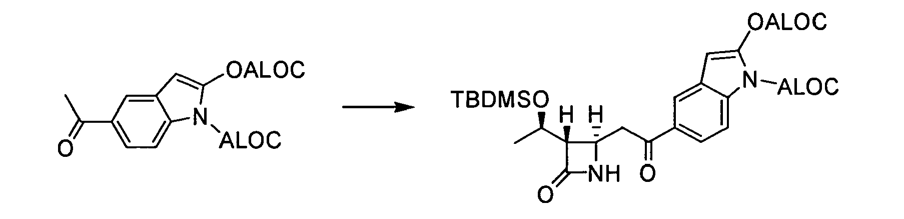

To a solution of allyl 5-acetyl-2-(allyloxycarbonyloxy)-1H-indole-1-carboxylate (3.76g, 11mmol) obtained by referential example 8 and triethylamine (1.23g, 12mmol) in methylene chloride (75ml) was dropped at 0°C trifluoromethane sulfonic acid trimethylsilyl (2.43g, 11mmol). After stirring for 20 minutes, thereto were dropped at 0°C triethylamine (0.60g, 6.0mmol) and trifluoromethanesulfonic acid trimethylsilyl (1.21g, 5.5mmol), followed by stirring for 15 minutes. To the reaction mixture were added under ice-cooling (2R,3R)-3-((1R)-1-{[tert-butyl(dimethyl)silyl]oxy}ethyl)-4-oxo-2-azetidinyl acetate (3.15g, 11mmol) and zinc iodide (3.2g, 9.9mmol), followed by stirring at room temperature for 5 hours. To the reaction mixture were added ethyl acetate and saturated brine, and the mixture was extracted with ethyl acetate, and separated by a separating funnel. The organic layer was washed with brine, dried over anhydrous sodium sulfate, filtered and concentrated. The residue was purified by silica gel column chromatography (silica gel 100g, ethyl acetate : hexane = 1:4→1:2) to give allyl 5-{[(2R,3S)-3-((1R)-1-{[tert-butyl(dimethyl)silyl]oxy}ethyl)-4-oxoazetidin-2-yl]acetyl}-2-(allyloxycarbonyloxy)-1H-indole-1-carboxylate (3.5 g, yield 56%).
¹H NMR (400 MHz, CDCl₃) δ 0.08 (s, 6 H), 0.86 (s, 9 H), 1.25 (d, 3 H, J = 6.2 Hz), 2.89-2.91 (m, 1 H), 3.17-3.26 (m, 1 H), 3.49-3.54 (m, 1 H), 4.12-4.16 (m, 1 H), 4.16-4.29 (m, 1 H), 4.76-4.79 (m, 2 H), 4.91-4.93 (m, 2 H), 5.36-5.42 (m, 2 H), 5.42-5.56 (m, 2 H), 5.92-6.12 (m, 2 H), 6.17 (s, 1 H), 6.43 (s, 1 H), 7.87-7.92 (m, 1 H), 8.12 (s, 1 H), 8.16-8.21 (m, 1 H).

### Referential example 10

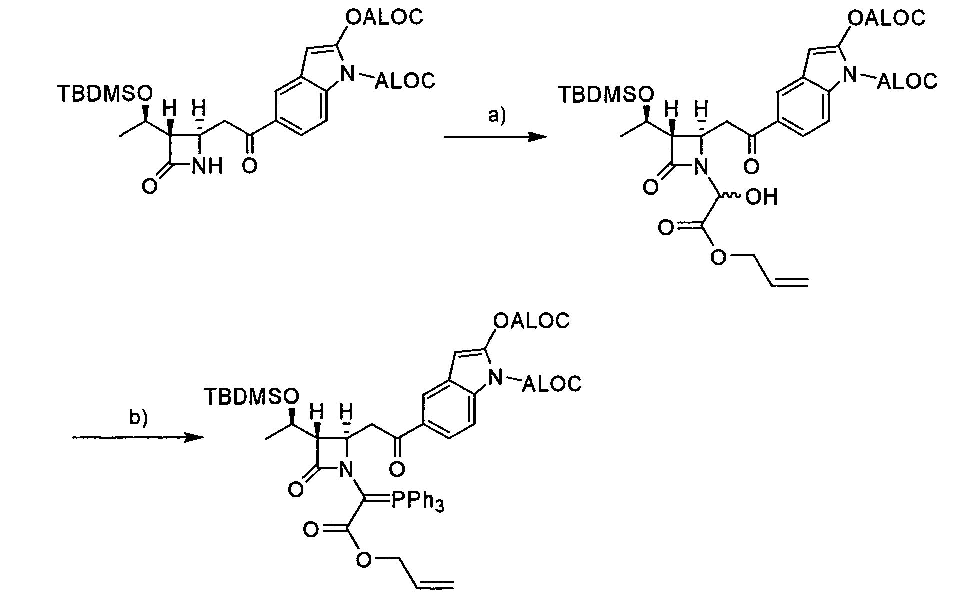

### Step a)

To a solution of allyl 5-{[(2R,3S)-3-((1R)-1-{[tert-butyl(dimethyl)silyl]oxy}ethyl)-4-oxoazetidin-2-yl]acetyl}-2-(allyloxycarbonyloxy)-1H-indole-1-carboxylate (3.52g, 6.2mmol) obtained by referential example 9 in toluene (100ml) was dropped at room temperature allyl dihydroxyacetate (1.2g, 9.3mmol). The reaction mixture was stirred at 120°C for 6 hours while azeotropically dehydrating using Dean-Stark evaporator. The reaction mixture was concentrated to give allyl {(2R,3S)- 2-[2-(1-allyloxycarbonyl-2-allyloxycarbonyloxy-1H-indol-5-yl)-2-oxoethyl]-3-((1R)-1-{[tert-butyl(dimethyl)silyl]oxy}ethyl)-4-oxoazetidin-1-yl}(hydroxyl)acetate. This product was used in the following reaction without purification.

### Step b)

To a solution of allyl {(2R,3S)- 2-[2-(1-allyloxycarbonyl-2-allyloxycarbonyloxy-1H-indol-5-yl)-2-oxoethyl]-3-((1R)-1-{[tert-butyl(dimethyl)silyl]oxy}ethyl)-4-oxoazetidin-1-yl}(hydroxyl)acetate obtained in step a) in THF (70ml) were added at -20°C 2,6-lutidine (1.05g, 9.9mmol) and thionyl chloride (1.03g, 8.6mmol). Thereto was added at room temperature THF (30ml), followed by stirring for 1 hour. The reaction mixture was filtered, concentrated and the residue was dissolved in 1,4-dioxane (70ml). Thereto were added at room temperature 2,6-lutidine (1.45g, 14mmol) and triphenylphosphine (2.91g, 11mmol), followed by stirring at 50°C for 3 hours. After addition of an aqueous saturated sodium hydrogen carbonate solution, the reaction mixture was extracted with ethyl acetate, and separated by a separating funnel. The organic layer was washed with saturated brine (3 times), dried over anhydrous sodium sulfate, filtered and concentrated. The residue was purified by silica gel column chromatography (silica gel 150g, ethyl acetate hexane = 1:5→1:1) to give allyl {(2R,3S)-2-[2-(1-allyloxycarbonyl-2-allyloxycarbonyloxy-1H-indol-5-yl)-2-oxoethyl]-3-((1R)-1-{[tert-butyl(dimethyl)silyl]oxy}ethyl)-4-oxoazetidin-1-yl}(triphenylphosphoanilidene)acetate (2.86g, yield 50%).
LC/MS (EI) 929 (M+1)

### Referential example 11

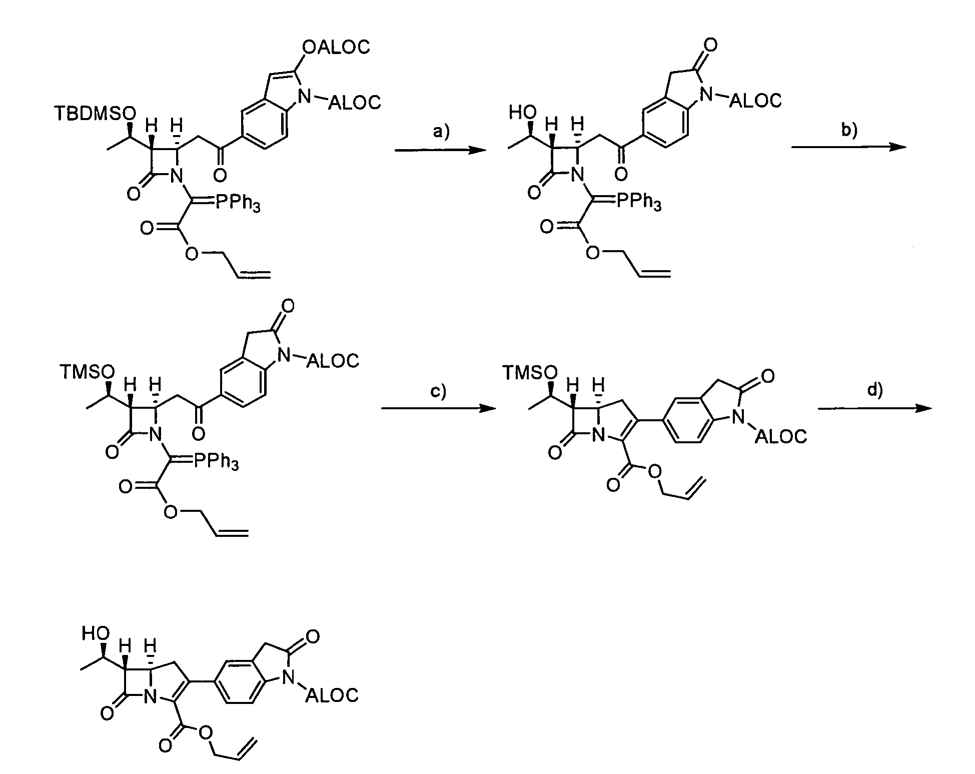

### Step a)

Allyl {(2R,3S)-2-[2-(1-allyloxycarbonyl-2-allyloxycarbonyloxy-1H-indol-5-yl)-2-oxoethyl]-3-((1R)-1-{[tert-butyl(dimethyl)silyl]oxy}ethyl)-4-oxoazetidin-1-yl}(triphenylphosphoanilidene)acetate (2.66 g) obtained by referential example 10 was dissolved at room temperature in 70% aqueous trifluoroacetic acid solution (30ml). After stirring for 3 days, to the reaction mixture was added ethyl acetate (100ml). The mixture was washed with saturated brine (100ml x 2) and aqueous saturated sodium hydrogen carbonate solution (100ml x 2), dried over anhydrous sodium sulfate, filtered and concentrated to give allyl [(2R,3S)-2-[2-(1-allyloxycarbonyl-2-oxo-2,3-dihydro-1H-indol-5-yl)-2-oxoethyl]-3-((1R)-1-hydroxyethyl)-4-oxoazetidin-1-yl(triphenylphosphoanilidene)acetate as a pale yellow amorphous. This product was used in the following reaction without purification.

### Step b)

To a solution of allyl [(2R,3S)-2-[2-(1-allyloxycarbonyl-2-oxo-2,3-dihydro-1H-indol-5-yl)-2-oxoethyl]-3-((1R)-1-hydroxyethyl)-4-oxoazetidin-1-yl(triphenylphosphoanilidene)acetate obtained in step a) and triethylamine (1.5g, 14mmol) in THF(80ml) was added at 0°C chlorotrimethylsilane (1.1g, 10mmol), followed by stirring for 30 minutes. After addition of ethyl acetate, the reaction mixture was washed with an aqueous saturated sodium hydrogen carbonate solution (50ml) and saturated brine (100ml), dried over anhydrous sodium sulfate, filtered, and concentrated. The residue was purified by silica gel column chromatography (silica gel 50 g, ethyl acetate : hexane = 1:3→2:1) to give allyl [(2R,3S)-2-[2-(1-allyloxycarbonyl-2-oxo-2,3-dihydro-1H-indol-5-yl)-2-oxoethyl]-4-oxo-3-((1R)-1-[(trimethylsilyl)oxy]ethyl)-azetidin-1-yl](triphenylphosphoanilidene)acetate (0.91g, yield 39%).
¹H NMR (400 MHz, CDCl₃) δ -0.05 (s, 9 H), 1.06-1.15 (m, 3 H), 2.62-2.75 (m, 1 H), 2.88-3.15 (m, 2 H), 3.41-3.52 (m, 1 H), 3.66-3.79 (m, 2 H), 3.82-4.01 (m, 1H), 4.42-4.61 (m, 2 H), 4.88-4.95 (m, 2 H), 5.03-5.28 (m, 2 H), 5.36-5.58 (m, 2 H), 5.78-6.13 (m, 2 H), 7.45-8.12 (m, 18 H).

### Step c)

To a solution of allyl [(2R,3S)-2-[2-(1-allyloxycarbonyl-2-oxo-2,3-dihydro-1H-indol-5-yl)-2-oxoethyl]-4-oxo-3-((1R)-1-[(trimethylsilyl)oxy]ethyl)-azetidin-1-yl](triphenylphosphoanilidene)acetate (0.91 g) obtained in step b) in toluene (45ml) was added N,O-bistrimethylsilylacetamide (0.46g), followed by refluxing for 10 minutes. After being cooled, the reaction mixture was concentrated and the residue was purified by silica gel column chromatography (silica gel 30g, ethyl acetate : hexane = 1:5→1:2) to give allyl (5R,6S)-3-(1-allyloxycarbonyl-2-oxo-2,3-dihydro-1H-indol-5-yl)-7-oxo-6-{(1R)-1-[(trimethylsilyl)oxy]ethyl}-1-azabicyclo[3.2.0]hept-2-ene-2-carboxylate (0.48g, yield 80%) as a pale yellow oil.
¹H NMR (400 MHz, CDCl₃) δ 0.14 (s, 9 H), 1.28 (d, 3 H, J = 6.2 Hz), 3.12-3.32 (m, 3 H), 3.69 (s, 2 H), 4.19-4.28 (m, 2 H), 4.59-4.67 (m, 1 H), 4.72-4.79 (m, 1 H), 4.88-4.95 (m, 2 H), 5.17-5.22 (m, 1 H), 5.29-5.39 (m, 2 H), 5.49-5.58 (m, 1 H), 5.83-6.12 (m, 2 H), 7.32-7.40 (m, 2 H), 7.86 (d, 1 H, J = 8.4 Hz).

### Step d)

Allyl (5R,6S)-3-(1-allyloxycarbonyl-2-oxo-2,3-dihydro-1H-indol-5-yl)-7-oxo-6-{(1R)-1-[(trimethylsilyl)oxy]ethyl}-1-azabicyclo[3.2.0]hept-2-ene-2-carboxylate (0.48g, 0.91mmol) obtained in step c) was dissolved in THF (45ml) and water (5ml), and thereto was gradually dropped 1N hydrochloric acid under cooling in an ice bath using a pH meter to adjust pH 3.

Twenty minutes later, an aqueous saturated sodium hydrogen carbonate solution (30ml) and saturated brine (30ml) were added thereto, and the reaction mixture was extracted with ethyl acetate (30ml). The organic layer was dried over anhydrous sodium sulfate, filtered and concentrated to give allyl [(5R,6S)-3-(1-allyloxycarbonyl-2-oxo-2,3-dihydro-1H-indol-5-yl)-7-oxo-6-{(1R)-1-hydroxyethyl}-1-azabicyclo[3.2.0]hept-2-ene]-2-carboxylate (0.39g, 0.87mmol, yield 96%) as a pale yellow oil.
¹H NMR (400 MHz, CDCl₃) δ 1.38 (d, 3 H, J = 6.2 Hz), 1.77 (br.s, 1 H), 3.16-3.38 (m, 3 H), 3.70 (s, 2 H), 4.21-4.35 (m, 2 H), 4.57-4.68 (m, 1 H), 4.72-4.79 (m, 1 H), 4.86-4.92 (m, 2 H), 5.19-5.27 (m, 1 H), 5.31-5.39 (m, 2 H), 5.48-5.59 (m, 1 H), 5.83-6.12 (m, 2 H), 7.33-7.40 (m, 2 H), 7.87 (d, 1 H, J = 8.4 Hz).

### Referential example 12

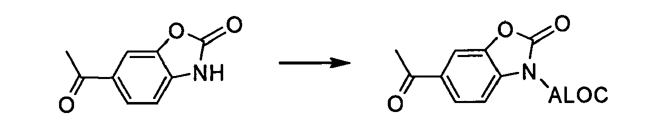

To a solution of 6-acetyl-1,3-benzoxazole-2(3H)-one (1.772g, 10mmol) in DMF (30ml) was added under ice-cooling 60% sodium hydride (480mg, 12mmol). Thereto was dropped under ice-cooling allyl chloroformate (1.45g, 12mmol), followed by stirring at room temperature for 1.5 hours. After addition of a previously cooled aqueous 5% potassium hydrogen sulfate solution, the reaction mixture was extracted with ethyl acetate and separated by a separating funnel. The organic layer was washed with brine (4 times) and partial solid was produced by adding THF. The solid was dissolved, dried over anhydrous magnesium sulfate, filtered and concentrated. The crude crystals were purified by suspending in ethyl acetate: hexane, followed by removal of the supernatant, and dried in vacuo to give allyl 6-acetyl-2-oxo-1,3-benzoxazole-3(2H)-carboxylate (2.207g, yield 84%) as a white solid.
¹H NMR (400 MHz, CDCl₃) δ 2.60 (s, 3 H), 4.90-4.98 (m, 2 H), 5.31-5.39 (m, 1 H), 5.48-5.58 (m, 1 H), 6.00-6.13 (m, 1 H), 7.77-7.83 (m, 1 H), 7.90-7.98 (m, 2 H).

### Referential example 13

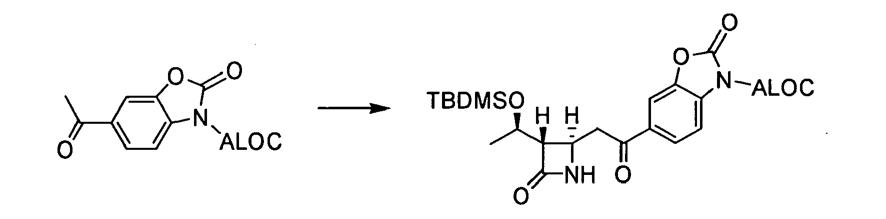

To a solution of ally 6-acetyl-2-oxo-1,3-benzoxazole-3(2H)-carboxylate (1.31g, 5mmol) and triethylamine (605mg, 6mmol) in methylene chloride (45ml) was dropped -50°C trifluoromethanesulfonic acid trimethylsilyl (1.22g, 5.5mmol), followed by stirring under ice-cooling for 20 minutes. To the reaction mixture were added under ice-cooling (2R,3R)-3-((1R)-1-{[tert-butyl(dimethyl)silyl]oxy}ethyl)-4-oxo-2-azetidinyl acetate (1.44g, 5mmol) and zinc iodide (1.60g, 5mmol), followed by stirring at room temperature for 2 hours and 20 minutes. After addition of ethyl acetate and saturated brine, the reaction mixture was extracted, separated by a separating funnel, dried over anhydrous magnesium sulfate, filtered and concentrated. The crude crystals were purified by suspended in ethyl acetate, followed by removal of the supernatant to give ally 6-{[(2R,3S)-3-((1R)-1-{[tert-butyl(dimethyl)silyl]oxy}ethyl)-4-oxoazetidin-2-yl]acetyl}-2-oxo-1,3-benzoxazole-3(2H)-carboxylate (1.675g) as a pale yellow. This product contained a starting material, allyl 6-acetyl-2-oxo-1,3-benzoxazole-3(2H)-carboxylate 7%, but the product was used in the following reaction without purification (yield 64%).
¹H NMR (400 MHz, CDCl₃) δ 0.03 (s, 3 H), 0.05 (s, 3 H), 0.83 (s, 9 H), 1.13 (d, 3 H, J = 6.2 Hz), 2.82-2.88 (m, 1 H), 3.37-3.44 (m, 1 H), 3.90-3.98 (m, 1 H), 4.07-4.17 (m, 1 H), 7.77-7.85 (m, 1 H), 7.91-8.00 (m, 1 H), 8.01-8.09 (m, 1 H).

### Referential example 14

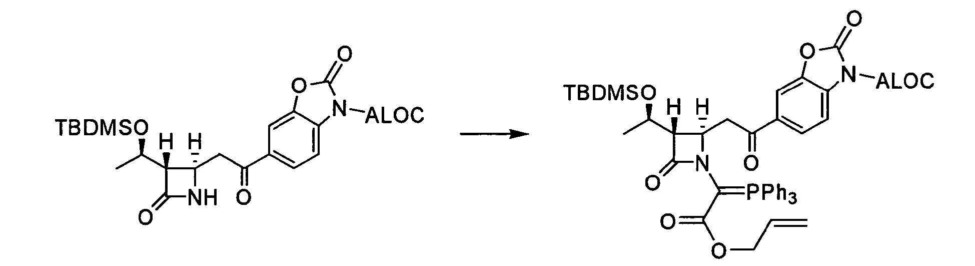

A solution of allyl 6-{[(2R,3S)-3-((1R)-1-{[tert-butyl(dimethyl)silyl]oxy}ethyl)-4-oxoazetidin-2-yl]acetyl}-2-oxo-1,3-benzoxazole-3(2H)-carboxylate (1.51g) obtained by referential example 13 and allyl dihydroxyacetate (0.49g) in toluene (31ml) was refluxed for 6 hours while azeotropically dehydrating using Dean-Stark evaporator. After being cooled toluene was removed by concentration in vacuo and the residue was dissolved in THF (15ml). Thereto was added 2,6-lutidine (0.50g). Thereto was dropped at -20°C thionyl chloride (0.55g), followed by stirring for 1 hour. After addition of THF (20ml), the insoluble material was taken by filtration and washed with THF. The filtrate and the washed solution were combined to be concentrated and the residue was dissolved in dioxane (50ml). Thereto were added 2,6-lutidine (0.73g) and triphenylphosphine (1.79g), followed by stirring at 60°C for 3 hours. After removal of dioxane in vacuo, the residue was dissolved in ethyl acetate (50ml), washed with saturated brine (50ml x 3), dried over magnesium sulfate, filtered, and concentrated in vacuo. The residue was purified by silica gel column chromatography (silica gel 50g, hexane : ethyl acetate = 4:1→1:1) to give allyl 6-{[(2R,3S)-1-[2-(allyloxy)-2-oxo-1-(triphenylphosphoanilidene)ethyl]-3-((1R)-1-{[tert-butyl(dimethyl)silyl]oxy}ethyl)-4-oxoazetidin-2-yl]acetyl}-2-oxo-1,3-benzoxazole-3(2H)-carboxylate (1.76g).
LCMS 847 (M+1+)

### Referential example 15

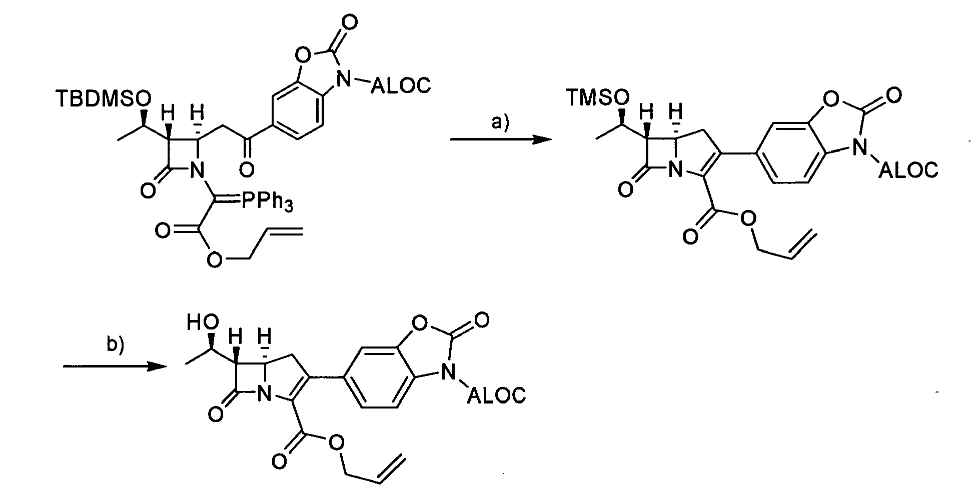

### Step a)

Allyl 6-{[(2R,3S)-1-[2-(allyloxy)-2-oxo-1-(triphenylphosphoanilidene)ethyl]-3-((1R)-1-{[tert-butyl(dimethyl)silyl]oxy}ethyl)-4-oxoazetidin-2-yl]acetyl}-2-oxo-1,3-benzoxazole-3(2H)-carboxylate (1.76g) obtained by referential example 14 was dissolved in 70%trifluoroacetic acid (7ml). After addition of chloroform (50ml) the reaction mixture was washed with saturated brine (50ml), and then thereto was added saturated brine (50ml). The mixture was neutralized with sodium hydrogen carbonate and the organic layer was separated by a separating funnel, dried over anhydrous magnesium sulfate, filtered and concentrated. The residue was dissolved in THF (10ml) and thereto was added triethylamine (0.35ml). To the mixture was dropped at 0°C chlorotrimethylsilane (0.32ml), followed by stirring for 30 minutes. To the reaction mixture were added saturated brine (10ml) and saturated sodium hydrogen carbonate solution (10ml) and the mixture was extracted with ethyl acetate (10ml x 3). The combined organic layer was dried over anhydrous magnesium sulfate, filtered and concentrated. After the residue was dissolved in toluene (40ml), to the solution was added N,O-bistrimethylsilylacetamide (0.5ml), followed by refluxing 2 hours. After being cooled toluene was removed in vacuo, and the residue was purified by silica gel column chromatography (silica gel 100g, hexane : ethyl acetate = 4:1→1:1) to give allyl 6-((5R,6S)-2-[(allyloxy)carbonyl]-7-oxo-6-{(1R)-1-[(trimethylsilyl)oxy]ethyl}-1-azabicyclo[3.2.0]hept-2-ene-3-yl)-2-oxo-1,3-benzoxazole-3(2H)-carboxylate (0.86g).
¹H NMR (400 MHz, CDCl₃) δ 0.15 (s, 9 H), 1.30 (d, 3 H, J = 6.2 Hz), 3.15-3.33 (m, 3 H), 4.20-4.27 (m, 2 H), 4.62-4.76 (m, 2 H), 4.96-4.97 (m, 2H), 5.21-5.24 (m, 1 H), 5.32-5.43 (m, 2 H), 5.53-5.57 (m, 1 H), 5.84-5.93 (m, 1 H), 6.01-6.11 (m, 1 H), 7.25-7.30 (m, 2 H), 7.75 (d, 1 H, J = 8.4 Hz).

### Step b)

Allyl 6-((5R,6S)-2-[(allyloxy)carbonyl]-7-oxo-6-{(1R)-1-[(trimethylsilyl)oxy]ethyl}-1-azabicyclo[3.2.0]hept-2-ene-3-yl)-2-oxo-1,3-benzoxazole-3(2H)-carboxylate (0.86g) obtained in step a) was dissolved in THF (13ml) and water (4ml), and thereto was gradually dropped 1N hydrochloric acid under cooling in an ice bath using a pH meter to adjust pH 2.5. Fifteen minutes later, an aqueous saturated sodium hydrogen carbonate solution (20ml) and saturated brine (50ml) were added thereto and the reaction mixture was extracted with chloroform (50ml x 3). The combined organic layer was dried over anhydrous sodium sulfate, filtered and concentrated to give allyl 6-{(5R,6S)-2-[(allyloxy)carbonyl]-6-[(1R)-1-hydroxyethyl]-7-oxo-1-azabicyclo[3.2.0]hept-2-ene-3-yl}-2-oxo-1,3-benzoxazole-3(2H)-carboxylate (0.69g, yield 93%) as a pale yellow amorphous.
¹H NMR (400 MHz, CDCl₃) δ 1.39 (d, 3 H, J = 6.3 Hz), 3.18-3.35 (m, 3 H), 3.75 (br.s, 1 H), 4.24-4.35 (m, 2 H), 4.62-4.78 (m, 2 H), 4.96-4.98 (m, 2H), 5.21-5.25 (m, 1 H), 5.31-5.42 (m, 2 H), 5.53-5.58 (m, 1 H), 5.84-5.94 (m, 1 H), 6.01-6.11 (m, 1 H), 7.25-7.32 (m, 2 H), 7.76 (d, 1 H, J = 8.4 Hz). LCMS 455 (M+1+1).

### Referential example 16

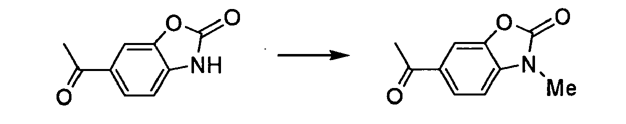

To a solution of 6-acetyl-1,3-benzoxazole-2(3H)-one (2.0g, 11.3mmol) in DMF (20ml) was added under ice-cooling 60% sodium hydride (540mg,13.6mmol), followed by stirring for 20 minutes. Thereto was dropped under ice-cooling methyl iodide (0.92ml, 14.7mmol), followed by stirring at room temperature for 1.5 hours. To the reaction mixture were added saturated brine (90ml) and 2M hydrochloric acid (10ml). The mixture was extracted with ethyl acetate, and separated by a separating funnel. The organic layer was dried over anhydrous magnesium sulfate, filtered and concentrated. The crude crystals were purified by silica gel column chromatography (silica gel 23g, chloroform : methanol = 100:0→ 95:5) to give 6-acetyl-3-methyl-1,3-benzoxazole-2-(3H)-one (2.11g, yield 98%) as a pale orange solid.
¹H NMR (400 MHz, CDCl₃) δ 2.61 (s, 3 H), 3.46 (s, 3 H), 7.03 (d, 1 H, J = 8.2 Hz), 7.82 (d, 1 H, J = 1.5 Hz), 7.90 (dd, 1 H, J = 1.5, 8.2 Hz). LCMS 192.

### Referential example 17

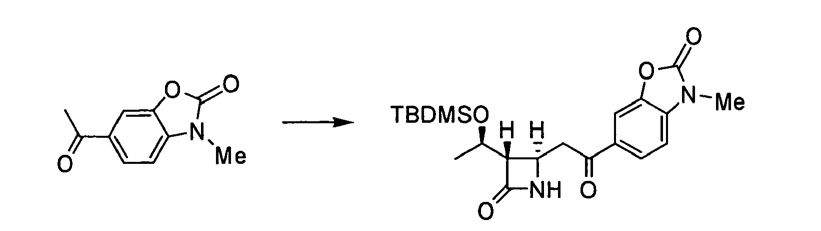

In accordance with the same method as referential example 13 using 6-acetyl-3-methyl-1,3-benzoxazole-2-(3H)-one (2.11g) obtained by referential example 16, there was obtained a crude product and the product was purified by silica gel column chromatography (silica gel 50g, hexane : ethyl acetate = 1:1→only ethyl acetate) to give 6-{[3-(1-{[tert-butyl(dimethyl)silyl]oxy}ethyl)-4-oxoazetidin-2-yl]acetyl}-3-methyl-1,3-benzoxazole-2(3H)-one (4.59g, quantitative yield) as a white solid.
¹H NMR (400 MHz, CDCl₃) δ 0.08 (s, 3 H), 0.09 (s, 3 H), 0.88 (s, 9 H), 1.26 (d, 3 H, J = 6.2 Hz), 2.90 (dd, 1 H, J = 2.3, 5.3 Hz), 3.16 (dd, 1 H, J = 10.2, 17.6 Hz), 3.43-3.48 (m, 1 H), 3.47 (s, 3 H), 4.11-4.15 (m, 1 H), 4.20-4.26 (m, 1 H), 6.11 (br.s, 1 H), 7.05 (d, 1 H, J = 8.3 Hz), 7.80 (d, 1 H, J = 1.5 Hz), 7.88 (dd, 1 H, J = 1.5, 8.3 Hz). LCMS 419 (M+1)+.

### Referential example 18

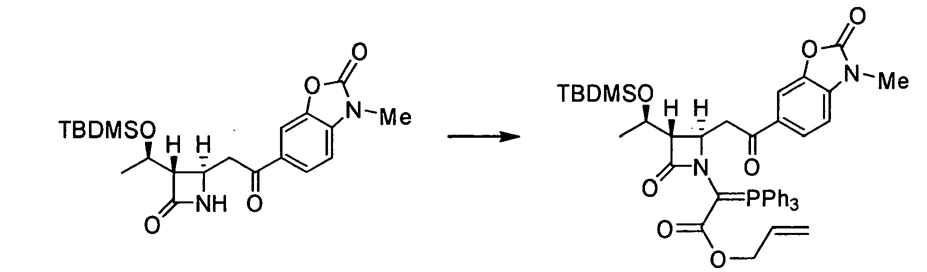

In accordance with the same method as referential example 14 using 6-{[3-(1-{[tert-butyl(dimethyl)silyl]oxy}ethyl)-4-oxoazetidin-2-yl]acetyl}-3-methyl-1,3-benzoxazole-2(3H)-one (4.59g) obtained by referential example 17, there was obtained allyl (2R,3S)-3-((1R)-1-{[tert-butyl(dimethyl)silyl]oxy}ethyl)-2-[2-(3-methyl-2-oxo-2,3-dihydro-1,3-benzoxazol-6-yl)-2-oxoethyl]-4-oxoazetidin-1-yl}(triphenylphosphoanilidene)acetate (5.24g, 61%) as a pale yellow amorphous.
LCMS 777 (M+1)+.

### Referential example 19

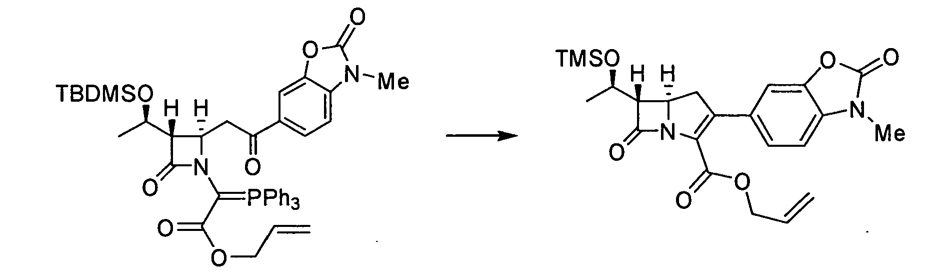

In accordance with the same method as step a) of referential example 15 using allyl {(2R,3S)-3-((1R)-1-{[tert-butyl(dimethyl)silyl]oxy}ethyl)-2-[2-(3-methyl-2-oxo-2,3-dihydro-1,3-benzoxazol-6-yl)-2-oxoethyl]-4-oxoazetidin-1-yl}(triphenylphosphoanilidene)acetate (3.0g) obtained by referential example 18, there was obtained allyl (5R,6S)-3-(3-methyl-2-oxo-2,3-dihydro-1,3-benzoxazol-6-yl)-7-oxo-6-{(1R)-1-[(trimethylsilyl)oxy]ethyl}-1-azabicyclo[3.2.0]hept-2-ene-2-carboxylate (1.8g, 100%) as a pale yellow oil. ¹H NMR (400 MHz, CDCl₃) δ 0.15 (s, 9H), 1.30 (d, 3 H, J = 6.2 Hz), 3.15-3.32 (m, 3 H), 3.41 (s, 3 H), 4.20-4.26 (m, 2 H), 4.62-4.76 (m, 2 H), 5.20-5.24 (m, 1 H), 5.32-5.37 (m, 1 H), 5.83-5.94 (m, 1 H), 6.93 (d, 1 H, J = 8.4 Hz), 7.25-7.28 (m, 2 H). LCMS 385, 457.

### Referential example 20

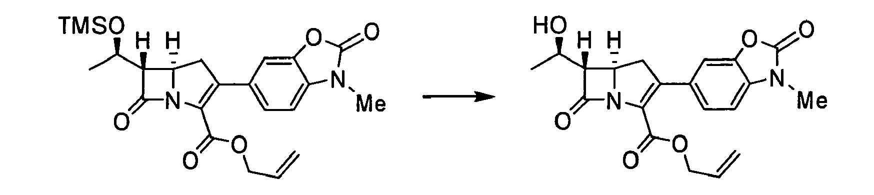

Allyl (5R,6S)-3-(3-methyl-2-oxo-1,3-benzoxazol-6-yl)-7-oxo-6-{(1R)-1-[(trimethylsilyl)oxy]ethyl}-1-azabicyclo[3.2.0]hept-2-ene-2-carboxylate (1.37g, 3.0mmol) was dissolved in THF (30ml) and water (3ml) and thereto was gradually dropped 1N hydrochloric acid under cooling in an ice bath using a pH meter to adjust pH 2.5. Ten minutes later, an aqueous saturated sodium hydrogen carbonate solution (20ml) and saturated brine (20ml) were added thereto, and the reaction mixture was extracted with ethyl acetate (20ml). The organic layer was dried over anhydrous sodium sulfate, filtered and concentrated to give allyl (5R,6S)-6-[(1R)-1-hydroxyethyl]-3-(3-methyl-2-oxo-1,3-benzoxazol-6-yl)-7-oxo-1-azabicyclo[3.2.0]hept-2-ene-2-carboxylate (0.77g, 2.0mmol, yield 66%) as a pale yellow oil.
¹H NMR (400 MHz, CDCl₃) δ 1.37 (d, 3 H, J = 6.3 Hz), 1.77 (d, 1 H, J = 4.6 Hz), 3.19-3.38 (m, 3 H), 3.41 (s, 3 H), 4.22-4.33 (m, 2 H), 4.61-4.79 (m, 2 H), 5.18-5.23 (m, 1 H), 5.29-5.35 (m, 1 H), 5.82-5.91 (m, 1 H), 6.93 (d, 1 H, J = 8.1 Hz), 7.23-7.29 (m, 2 H).

### Referential example 21

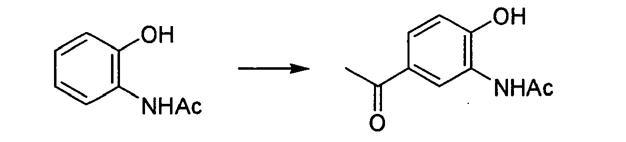

To a suspension of aluminum chloride (53.3g) in DMF (8.6g) was added at 35°C 2-acetamide phenol (7.6g) and then acetyl chloride (5.5g), and the mixture was heated at 85°C for 5 hours. After being cooled, to the reaction mixture was gradually added at 0°C ice and concentrated sulfuric acid. The reaction mixture was added to 6M hydrochloric acid (400ml) and the insoluble material was taken by filtration, washed with water and dried in vacuo to give N-(5-acetyl-2-hydroxyphenyl)acetamide (2.38g) as a pale green solid.
¹H NMR (400 MHz, DMSO-d₆) δ 2.10 (s, 3 H), 2.46 (s, 3 H), 6.94 (d, 1 H, J = 8.4), 7.61 (dd, 1 H, J = 2.1, 8.4), 8.41 (d, 1 H, J = 2.1 Hz), 9.34 (br.s, 1 H), 10.80 (br.s, 1 H). LCMS 194 (M+1+).

### Referential example 22

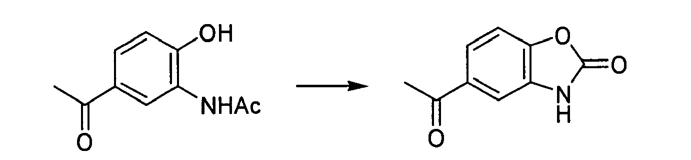

N-(5-Acetyl-2-hydroxyphenyl)acetamide (4.86g) obtained by referential example 21 was suspended in concentrated sulfuric acid (25ml) and the suspension was refluxed for 45 minutes. After being cooled hydrochloric acid was removed in vacuo. To the residue were added urea and concentrated hydrochloric acid (7ml), and the mixture was heated at 140°C for 1.5 hours, and at 170°C for 2.5 hours. After being cooled, thereto was added water (30ml), followed by stirring for 1 hour. The solid was taken by filtration, washed with water, and dried in vacuo to give a crude product (5.3g). By recrystallization from ethanol there was obtained 5-acetyl-1,3-benzoxazole-2-(3H)-one (2.08g) as an orange solid.
¹H NMR (400 MHz, DMSO-d₆) δ 2.47 (s, 3 H), 7.30 (d, 1 H, J = 8.4 Hz), 7.45 (d, 1 H, J = 1.8 Hz), 7.67 (dd, 1 H, J = 1.8, 8.4 Hz), 11.80 (br.s, 1 H).

### Referential example 23

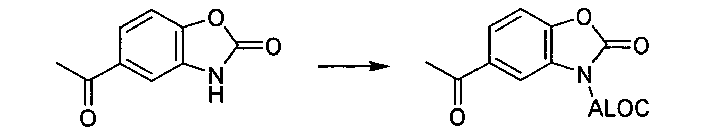

In accordance with the same method as referential example 12 using 5-acetyl-1,3-benzoxazole-2(3H)-one (2.08g) obtained by referential example 22 there was obtained a crude product. This product was purified by silica gel column chromatography (silica gel 30g, hexane : ethyl acetate = 4:1→1:1) to give allyl 5-acetyl-2-oxo-1,3-benzoxazole-3(2H)-carboxylate (2.61g) as a yellow solid.
¹H NMR (400 MHz, CDCl₃) δ 2.64 (s, 3 H), 4.99-5.01 (m, 1 H), 5.41-5.44 (m, 1 H), 5.55-5.60 (m, 1 H), 6.02-6.13 (m, 1 H), 7.30 (d, 1 H, J = 8.4 Hz), 7.94 (dd, 1 H, J = 1.8, 8.4 Hz), 8.41 (d, 1 H, J = 1.8 Hz).

### Referential example 24

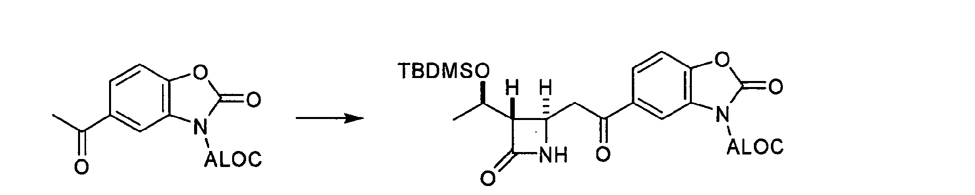

In accordance with the same method as referential example 13 using allyl 5-acetyl-2-oxo-1,3-benzoxazole-3(2H)-carbxylate (2.61g) obtained by referential example 23, there was obtained a crude product. The product was purified by silica gel column chromatography (silica gel 60g, hexane : ethyl acetate = 1:1→1:3) to allyl 5-{[3-(1-{[tert-butyl(dimethyl)silyl]oxy}ethyl)-4-oxoazetidin-2-yl]acetyl}-2-oxo-1,3-benzoxazole-3(2H)-carboxylate (4.31g) as a pale yellow solid.
¹H NMR (400 MHz, CDCl₃) δ 0.08 (s, 3 H), 0.09 (s, 3 H), 0.88 (s, 9 H), 1.26 (d, 3 H, J = 6.2 Hz), 2.91 (dd, 1 H, J = 2.3, 5.2 Hz), 3.19 (dd, 1 H, J = 10.2, 17.8), 3.47 (dd, 1 H, J = 3.0, 17.8 Hz), 4.13-4.17 (m, 1 H), 4.21-4.27 (m, 1 H), 4.99-5.01 (m, 1 H), 5.41-5.45 (m, 1 H), 5.55-5.61 (m, 1 H), 6.03-6.11 (m, 1 H), 6.12 (br.s, 1 H), 7.33 (d, 1 H, J = 8.4 Hz), 7.93 (dd, 1 H, J = 1.8, 8.4 Hz), 8.41 (d, 1 H, J = 1.8 Hz).

### Referential example 25

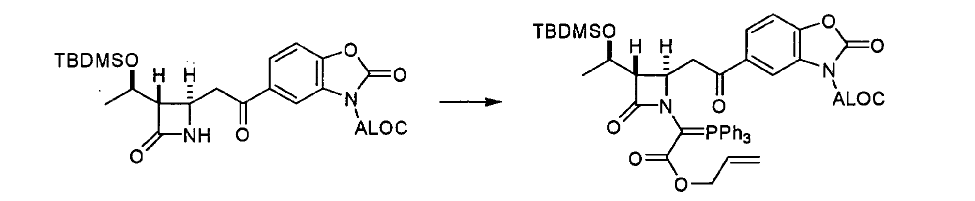

In accordance with the same method as referential example 14 using allyl 5-{[3-(1-{[tert-butyl(dimethyl)silyl]oxy}ethyl)-4-oxoazetidin-2-yl]acetyl}-2-oxo-1,3-benzoxazole-3(2H)-carboxylate (4.31g) obtained by referential example 24, there was obtained allyl 5-{[1-[2-(allyloxy)-2-oxo-1-(triphenylphosphoanilidene)ethyl]-3-(1-{[tert-butyl(dimethyl)silyl]oxy}ethyl)-4-oxoazetidin-2-yl]acetyl}-2-oxo-1,3-benzoxazole-3(2H)-carboxylate (5.58g) as a pale yellow amorphous.
LCMS 847 (M+1+).

### Referential example 26

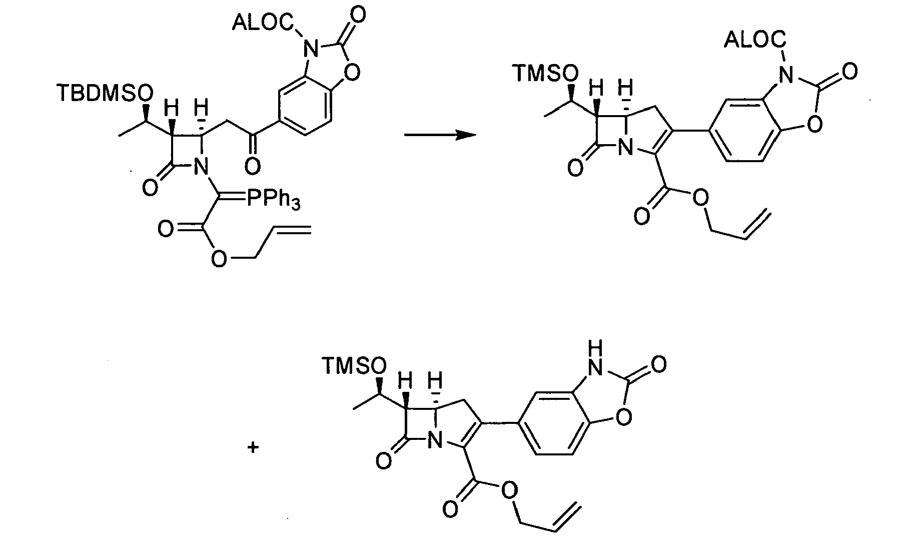

In accordance with the same method as step a) of referential example 15 using allyl 5-{[1-[2-(allyloxy)-2-oxo-1-(triphenylphosphoanilidene)ethyl]-3-(1-{[tert-butyl(dimethyl)silyl]oxy}ethyl)-4-oxoazetidin-2-yl]acetyl}-2-oxo-1,3-benzoxazole-3(2H)-carboxylate (2.5g) obtained by referential example 25, there were obtained allyl 5-((5R,6S)-2-[(allyloxy)carbonyl]-7-oxo-6-{(1R)-1-[(trimethylsilyl)oxy]ethyl}-1-azabicyclo[3.2.0]hept-2-ene-3-yl)-2-oxo-1,3-benzoxazole-3(2H)-carboxylate (0.79g) as a pale yellow amorphous, and allyl (5R,6S)-7-oxo-3-(2-oxo-2,3-dihydro-1,3-benzoxazol-5-yl)-6-{(1R)-1-[(trimethylsilyl)oxy]ethyl}-1-azabicyclo[3.2.0]hept-2-ene-2-carboxylate (0.43g) as a pale brown amorphous.

Allyl 5-((5R,6S)-2-[(allyloxy)carbonyl]-7-oxo-6-{(1R)-1-[(trimethylsilyl)oxy]ethyl}-1-azabicyclo[3.2.0]hept-2-ene-3-yl)-2-oxo-1,3-benzoxazole-3(2H)-carboxylate: ¹H NMR (300 MHz, CDCl₃) δ 0.15 (s, 9H), 1.31 (d, 3 H, J = 6.2 Hz), 3.14-3.34 (m, 3 H), 4.19-4.28 (m, 2 H), 4.60-4.76 (m, 2 H), 4.95-4.98 (m, 2 H), 5.18-5.22 (m, 1 H), 5.27-5.35 (m, 1 H), 5.37-5.42 (m, 1 H), 5.51-5.58 (m, 1 H), 5.80-5.93 (m, 1 H), 5.99-6.12 (m, 1 H), 7.19 (d, 1 H, J = 8.4 Hz), 7.25-7.29 (m, 1 H), 7.86 (d, 1 H, J = 1.8 Hz). LCMS 527 (M+1+), 455 (M-TMS+1).
Allyl (5R,6S)-7-oxo-3-(2-oxo-2,3-dihydro-1,3-benzoxazol-5-yl)-6-{(1R)-1-[(trimethylsilyl)oxy]ethyl}-1-azabicyclo[3.2.0]hept-2-ene-2-carboxylate: ¹H NMR (300 MHz, CDCl₃) δ 0.15 (s, 9 H), 1.31 (d, 3 H, J = 6.2 Hz), 3.13-3.33 (m, 3 H), 4.19-4.27 (m, 2 H), 4.60-4.76 (m, 2 H), 5.20-5.24 (m, 1 H), 5.30-5.36 (m, 1 H), 5.82-5.94 (m, 1 H), 7.09-7.19 (m, 3 H), 8.60 (br.s, 1 H).
LCMS 371 (M-TMS+1+).

### Referential example 27

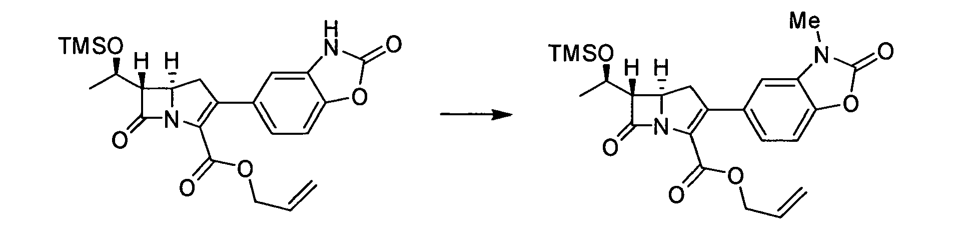

To a solution of allyl (5R,6S)-7-oxo-3-(2-oxo-2,3-dihydro-1,3-benzoxazol-5-yl)-6-{(1R)-1-[(trimethylsilyl)oxy]ethyl}-1-azabicyclo[3.2.0]hept-2-ene-2-carboxylate (0.28g) obtained by referential example 26 in DMF (2.5ml) was dropped at 0°C methyl iodide (47µl) and then added potassium carbonate (0.14g), followed by stirring for 1.5 hours. After addition of ether (100ml) the reaction mixture was washed with saturated brine (50ml x 3), dried over magnesium sulfate, filtered and concentrated to give a crude product. Further the same procedure was carried out using allyl (5R,6S)-7-oxo-3-(2-oxo-2,3-dihydro-1,3-benzoxazol-5-yl)-6-{(1R)-1-[(trimethylsilyl)oxy]ethyl}-1-azabicyclo[3.2.0]hept-2-ene-2-carboxylate (0.11g) to give a product. This product was combined with the previously obtained crude product and the combined product was purified by silica gel column chromatography (silica gel 15g, hexane : ethyl acetate = 4: 1) to give allyl (5R,6S)-3-(3-methyl-2-oxo-2,3-dihydro-1,3-benzoxazol-5-yl)-7-oxo-6-{(1R)-1-[(trimethylsilyl)oxy]ethyl}-1-azabicyclo[3.2.0]hept-2-ene-2-carboxylate (0.23g, yield 59%) as a pale yellow oil.
¹H NMR (400 MHz, CDCl₃) δ 0.16 (s, 9 H), 1.31 (d, 3 H, J = 6.2 Hz), 3.18-3.33 (m, 3 H), 3.39 (s, 3 H), 4.20-4.27 (m, 2 H), 4.62-4.74 (m, 2 H), 5.19-5.23 (m, 1 H), 5.30-5.35 (m, 1 H), 5.83-5.92 (m, 1 H), 7.08 (d, 1 H, J = 1.6 Hz), 7.11 (dd, 1 H, J = 1.6, 8.3 Hz), 7.17 (d, 1 H, J = 8.3 Hz).

### Referential example 28

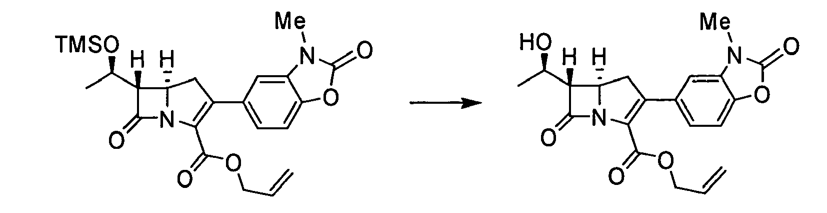

Allyl (5R,6S)-3-(3-methyl-2-oxo-1,3-benzoxazol-5-yl)-7-oxo-6-{(1R)-1-[(trimethylsilyl)oxy]ethyl}-1-azabicyclo[3.2.0]hept-2-ene-2-carboxylate (0.233g, 0.51mmol) was dissolved in THF (5.5ml) and water (0.5ml), and thereto was gradually dropped 1N hydrochloric acid under cooling in an ice bath using a pH meter to adjust pH 2.5. Ten minutes later, an aqueous saturated sodium hydrogen carbonate solution (20ml) and saturated brine (20ml) were added thereto and the reaction mixture was extracted with ethyl acetate (20ml). The organic layer was dried over anhydrous sodium sulfate, filtered and concentrated to give allyl (5R,6S)-6-[(1R)-1-hydroxyethyl]-3-(3-methyl-2-oxo-1,3-benzoxazole-5-yl)-7-oxo-1-azabicyclo[3.2.0]hept-2-ene-2-carboxylate (0.21g, 0.51mmol, yield 100%) as a pale yellow oil.
¹H NMR (400 MHz, CDCl₃) δ 1.37 (d, 3 H, J = 6.3 Hz), 1.77 (br.s, 1 H), 3.17-3.33 (m, 3 H), 3.40 (s, 3 H), 4.20-4.37 (m, 2 H), 4.60-4.78 (m, 2 H), 5.19-5.35 (m, 2 H), 5.81-5.93 (m, 1 H), 7.04-7.21 (m, 3 H).

### Referential example 29

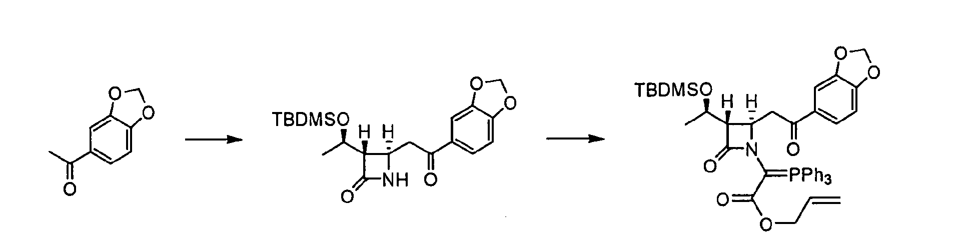

A crude product obtained in accordance with the same method as referential example 13 using 3',4'-(methylenedioxy)actophenone (10.0g) was purified by silica gel column chromatography (silica gel 500g, hexane : ethyl acetate = 2:1→1:1→0:1) to give (3S,4R)-4-[2-(1,3-benzodioxol-5-yl)-2-oxoethyl]-3-((1R)-1-{[tert-butyl(dimethyl)silyl]oxy}ethyl)azetidin-2-one (18.7g, 78%) as a pale yellow solid. LCMS 392. This product was treated in the same manner as referential example 14 to give allyl [(2R,3S)-2-[2-(1,3-benzodioxol-5-yl)-2-oxoethyl]-3-((1R)-1-{[tert-butyl(dimethyl)silyl]oxy}ethyl)-4-oxoazetidin-1-yl](triphenylphosphoanilidene)acetate (27.3g, 77%) as an ocher amorphous. LCMS 750 (M+1)+.

### Referential example 30

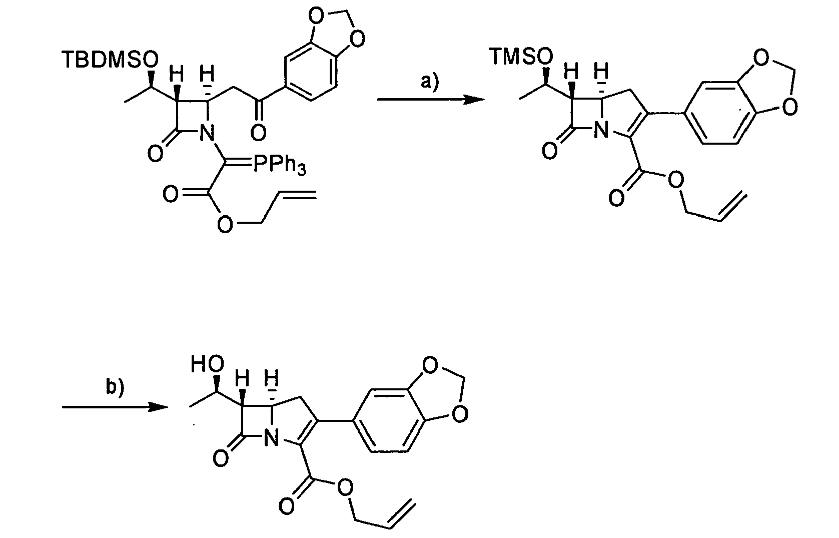

### Step a)

Allyl [(2R,3S)-2-[2-(1,3-benzodioxol-5-yl)-2-oxoethyl]-3-((1R)-1-{[tert-butyl(dimethyl)silyl]oxy}ethyl)-4-oxoazetidin-1-yl](triphenylphosphoanilidene)acetate (4.04g) obtained by referential example 29 was treated by the same method as step a) of referential example 15 to give allyl (5R,6S)-3-(1,3-benzoxol-5-yl)-7-oxo-6-{(1R)-1-[(trimethylsilyl)oxy]ethyl}-1-azabicyclo[3.2.0]hept-2-ene-2-carboxylate (2.07g, 89%) as a pale yellow oil.
¹H NMR (400 MHz, CDCl₃) δ 0.15 (s, 9 H), 1.30 (d, 3 H, J = 6.2 Hz), 3.11-3.27 (m, 3 H), 4.15-4.24 (m, 2 H), 4.62-4.82 (m, 2 H), 5.20-5.23 (m, 1 H), 5.31-5.37 (m, 1 H), 5.85-5.95 (m, 1 H), 5.97-5.98 (m, 2 H), 6.78 (d, 1 H, J = 8.1 Hz), 6.89 (dd, 1 H, J = 1.7, 8.1 Hz), 6.92 (d, 1 H, J = 1.7 Hz). LCMS 430, 358.

### Step b)

Allyl (5R,6S)-3-(1,3-benzodioxol-5-yl)-7-oxo-6-{(1R)-1-[(trimethylsilyl)oxy]ethyl}-1-azabicyclo[3.2.0]hept-2-ene-2-carboxylate (1.02 g) obtained by step a) was treated by the same method as referential example 15 to give allyl (5R,6S)-3-(1,3-benzodioxol-5-yl)-6-[(1R)-1-hydroxyethyl]-7-oxo-1-azabicyclo[3.2.0]hept-2-ene-2-carboxylate (0.77g, yield 91%) as a pale yellow oil.
¹H NMR (400 MHz, CDCl₃) δ 1.38 (d, 3 H, J = 6.3 Hz), 3.14-3.29 (m, 3 H), 4.23-4.32 (m, 2 H), 4.62-4.78 (m, 2 H), 5.20-5.24 (m, 1 H), 5.31-5.36 (m, 1 H), 5.86-5.95 (m, 1 H), 5.98-5.99 (m, 2 H), 6.79 (d, 1 H, J = 8.1 Hz), 6.89 (dd, 1 H, J = 1.7, 8.1 Hz), 6.92 (d, 1 H, J = 1.7 Hz). LCMS 358 (M+1+). Referential example 31

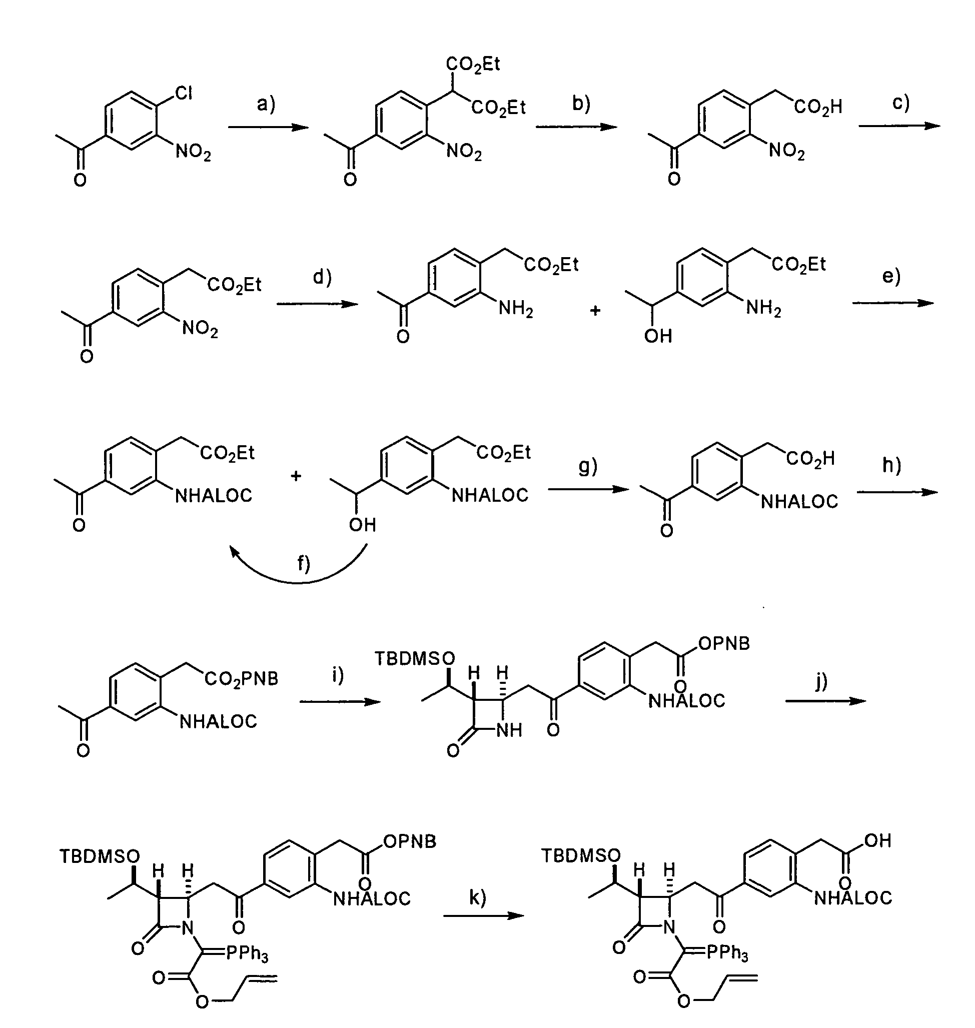

### Step a)

### Diethyl (4-acetyl-2-nitrophenyl)malonate

To a solution of sodium ethoxide in 20% ethanol (68.7 g) was dropped under stirring at 40-50°C diethyl malonate (32.4 g), followed by stirring for 10 minutes. To this mixture was added at room temperature 4-chloro-3-nitroacetophenone (20.2 g), followed by stirring for 3 hours. To the reaction mixture were added 2N hydrochloric acid (200ml) and chloroform (200ml) and an organic layer was separated. The aqueous layer was extracted with chloroform (100ml x 2), and the combined organic layer was dried over anhydrous magnesium sulfate, filtered and removed the solvent in vacuo to give a mixture of the object compound and diethyl malonate (54.1g, quantities yield). This product was used in the following reaction without purification.

The sample for analysis was obtained by purification by silica gel column chromatography (hexane/ethyl acetate).
¹H NMR (400 MHz, CDCl₃) δ 1.29 (t, 6 H, J = 7.1 Hz), 2.67 (s, 3 H), 4.280 (q, 2 H, J = 7.1 Hz), 4.283 (q, 2 H, J = 7.1 Hz), 5.33 (s, 1 H), 7.67 (d, 1 H, J = 8.1 Hz), 8.20 (dd, 1 H, J = 8.1, 1.8 Hz), 8.60 (d, 1 H, J = 1.8 Hz).

### Step b)

### (4-Acetyl-2-nitrophenyl)acetic acid

A mixture of diethyl (4-acetyl-2-nitrophenyl)malonate and diethyl malonate obtained in step a) (54.1g) was dissolved in 4M hydrochloric acid (800ml)/dioxane (800ml), followed by stirring at 100°C for 8 hours. After being cooled dioxane was removed in vacuo. The aqueous layer was extracted with chloroform (200ml, and then 100ml x 2). The combined organic layer was dried over anhydrous magnesium sulfate, filtered and the solvent was removed in vacuo to give the object compound (23.1g, quantitative yield) as a brown solid.
¹H NMR (400 MHz, CDCl₃) δ 2.68 (s, 3 H), 4.14 (s, 2 H), 7.50 (d, 1 H, J = 7.9 Hz), 8.18 (dd, 1 H, J = 1.7, 7.9Hz), 8.68 (d, 1 H, J = 1.7 Hz).

### Step c)

### Ethyl (4-acetyl-2-nitrophenyl)acetate

(4-Acetyl-2-nitrophenyl)acetic acid (23.1g) obtained in step b) was dissolved in ethanol (500ml) and thereto was added concentrated hydrochloric acid (50ml), followed by refluxing for 6 hours. After being cooled ethanol was removed in vacuo. To the aqueous layer were added ethyl acetate (300ml) and saturated sodium hydrogen carbonate solution (100ml), and then the mixture was neutralized with sodium hydrogen carbonate powder. After the aqueous layer was separated, the organic layer was washed with saturated sodium hydrogen carbonate solution (100ml) and saturated brine (100ml), dried over anhydrous magnesium sulfated, filtered, and the solvent was removed in vacuo. The residue was purified by silica gel column chromatography (silica gel 100g, hexane : ethyl acetate = 1:1) to give the object compound (23.2g, yield 91%) as a brown oil. The aqueous layer was acidic with hydrochloric acid, and extracted with chloroform to recover (4-acetyl-2-nitrophenyl)acetic acid (1.8g, yield 8%).
¹H NMR (400 MHz, CDCl₃) δ 1.26 (t, 3 H, J = 7.1 Hz), 2.67 (s, 3 H), 4.09 (s, 2 H), 4.18 (q, 2 H, J = 7.1 Hz), 7.49 (d, 1 H, J = 7.9 Hz), 8.16 (dd, 1 H, J = 1.8, 7.9Hz), 8.65 (d, 1 H, J = 1.8 Hz). LCMS (EI) 252 (M+1)+.

### Step d)

### A mixture of ethyl (4-acetyl-2-aminophenyl)acetate and ethyl [2-amino-4-(1-hydroxyethyl)phenyl]acetate

To a solution of ethyl 4-acetyl-2-nitrophenyl)acetate (23.2g) obtained in step c) in ethanol (660ml) was added 5% palladium carbon (5.9g) and the mixture was stirred for 9.5 hours under hydrogen gas under ordinary pressure. After removal of the catalyst over Celite, the solvent was removed in vacuo to the object compound (20.4g, quantitative yield) as a brown oil. The ratio of ketone form and alcohol form was 1:2 by NMR analysis. This product was used in the following reaction without purification.
LCMS (EI) 222 (M+1)+: ketone form, 224 (M+1)+: Alcohol form.

### Step e)

### Ethyl (4-acetyl-2-{[(allyloxy)carbonyl]amino}phenyl) acetate and ethyl [2-{[(allyloxy)carbonyl]amino}-4-(1-hydroxyethyl)phenyl]acetate

To a solution of a mixture of ethyl (4-acetyl-2-aminophenyl)acetate and ethyl [2-amino-4-(1-hydroxyethyl)phenyl] acetate (20.4g) obtained in step d) in pyridine (184ml) was dropped at room temperature allyloxycarbonyl chloride (22.2g), followed by stirring for 30 minutes. To the reaction mixture were added aqueous saturated ammonium chloride solution (100ml) and saturated brine (100ml). After pyridine was removed in vacuo, to the aqueous layer was added 2M hydrochloric acid (200ml) and the mixture was extracted with ethyl acetate (100ml x 3). The combined organic layer was washed with 2M hydrochloric acid (50ml x 3) and saturated brine (50ml), filtered and the solvent was removed in vacuo. The residue was purified by silica gel column chromatography (silica gel 500g, hexane : ethyl acetate = 3:1→1:1) to give ethyl (4-acetyl-2-{[(allyloxy)carbonyl]amino}phenyl)acetate (7.34g, yield 26%) as a gray oil, and ethyl [2-{[(allyloxy)carbonyl]amino}-4-(1-hydroxyethyl)phenyl]acetate (14.1g, yield 50%) as a purple oil.

### Ethyl (4-acetyl-2-{[(allyloxy)carbonyl]amino}phenyl) acetate

¹H NMR (400 MHz, CDCl₃) δ 1.28 (t, 3 H, J = 7.1 Hz), 2.60 (s, 3 H), 3.68 (s, 2 H), 4.18 (q, 2 H, J = 7.1 Hz), 4.69-4.71 (m, 2 H), 5.26-5.30 (m, 1 H), 5.37-5.41 (m, 1 H), 5.95-6.05 (m, 1 H), 7.30 (d, 1 H, J = 8.0 Hz), 7.69 (dd, 1 H, J = 1.8, 8.0 Hz), 8.12 (br.s, 1 H), 8.42 (br.s, 1 H). LCMS (EI) 306 (M+1)+. Ethyl [2-{[(allyloxy)carbonyl]amino}-4-(1-hydroxyethyl)phenyl] acetate ¹H NMR (400 MHz, CDCl₃) δ 1.28 (t, 3 H, J = 7.1 Hz), 1.49 (d, 3 H, J = 6.4 Hz), 3.61 (s, 2 H), 4.16 (q, 2 H, J = 7.1 Hz), 4.67-4.69 (m, 2 H), 4.89 (q, 1 H, J = 6.4 Hz), 5.24-5.28 (m, 1 H), 5.34-5.40 (m, 1 H), 5.94-6.03 (m, 1 H), 7.12 (dd, 1 H, J = 1.7, 7.8 Hz), 7.18 (d, 1 H, J = 7.8 Hz), 7.81 (br.s, 1 H), 8.07 (br.s, 1 H). LCMS (EI) 308 (M+1)+.

### Step f)

### Ethyl (4-acetyl-2-{[(allyloxy)carbonyl]amino}phenyl)acetate

To a solution of ethyl [2-{[(allyloxy)carbonyl]amino}-4-(1-hydroxyethyl)phenyl]acetate (10.3 g) obtained in step e) in acetone (30ml) was added at room temperature Jones reagent (10ml), followed by stirring for 30 minutes. To the reaction mixture were added a aqueous saturated sodium hydrogen carbonate solution (50ml) and saturated brine (100ml), and the mixture was extracted with ethyl acetate (50ml x 3). The combined organic layer was dried over anhydrous magnesium sulfate, filtered and concentrated in vacuo to give a crude product. The product was combined with a product separately obtained in 1g scale, and the combined product was purified by silica gel column chromatography (silica gel 300g, hexane : ethyl acetate = 1:1) to give the object compound (10.3g, yield 85%) as a yellow oil.

### Step g)

### (4-Acetyl-2-{[(allyloxy)carbonyl]amino}phenyl)acetic acid

To a solution of ethyl (4-acetyl-2-{[(allyloxy)carbonyl]amino}phenyl)acetate (17.6g) obtained in steps e) and f) in ethanol (176ml) was added at 0°C 1M aqueous sodium hydroxide solution (132ml), followed by stirring for 1 hour. To the reaction mixture was added 2M hydrochloric acid (400ml) and ethanol was removed in vacuo. The resulted solid was collected by filtration and washed with 2M hydrochloric acid and dried in vacuo to give the object compound (11.2g, yield 70%) as a pale brown solid. The aqueous layer was extracted with ethyl acetate, dried over magnesium sulfate, filtered and concentrated in vacuo to give the object compound (3.58 g, yield 22%) as a brown solid.
¹H NMR (400 MHz, CDCl₃) δ 2.59 (s, 3 H), 3.74 (s, 2 H), 4.68-4.70 (m, 2 H), 5.27 (br.d, 1 H, J = 10.8 Hz), 5.35 (br.d, 1 H, J = 15.3 Hz), 5.60 (br.s, 1 H), 7.34 (d, 1 H, J = 7.9 Hz), 7.70 (br.s, 1 H), 7.74 (dd, 1 H, J = 1.5, 7.9 Hz), 8.25 (br.s, 1 H).

### Step h)

### 4-Nitrobenzyl (4-acetyl-2-{[(allyloxy)carbonyl]amino}phenyl)acetate

To a solution of (4-acetyl 2-{[(allyloxy)carbonyl]amino}phenyl)acetic acid (10.9g) obtained in step g) and triethylamine (11ml) in DMF (100ml) was added at room temperature p-nitrobenzyl bromide (17.0g), followed by stirring 1 hour. To the reaction mixture were added saturated brine (500ml) and water (300ml), and the mixture was extracted with ethyl acetate (200ml and then 100ml x 2). The combined organic layer was washed with saturated brine (100ml), dried over anhydrous magnesium sulfate, filtered and concentrated in vacuo. The residue was purified by silica gel column chromatography (silica gel 300g, hexane : ethyl acetate : chloroform = 3:1:4→0:1:1) to give the object compound (16.4g, yield 98%) as a pale brown solid. This product was recrystallized from chloroform/hexane to the object compound (10.4g, yield 64%) as a white solid.
¹H NMR (400 MHz, CDCl₃) δ 2.61 (s, 3 H), 3.78 (s, 2 H), 4.68-4.70 (m, 2 H), 5.24 (s, 2 H), 5.27-5.30 (m, 1 H), 5.35-5.40 (m, 1 H), 5.93-6.01 (m, 1 H), 7.32 (d, 1 H, J = 8.0 Hz), 7.46-7.50 (m, 2 H), 7.71 (dd, 1 H, J = 1.8, 8.0 Hz), 7.73 (br.s, 1 H), 8.22 (td, 2 H, J = 2.3, 6.8 Hz), 8.38 (br.s, 1 H). LCMS (EI) 413 (M+1)+.

### Step i)

### 4-Nitorobenzyl (2-{[(allyloxy)carbonyl]amino}-4-{[(2R,3S)-3-((1R)-1-{[tert-butyl(dimethyl)silyl]oxy}ethyl)-4-oxoazetidin-2-yl]acetyl}phenyl)acetate

To a solution of 4-nitrobenzyl (4-acetyl-2-{[(allyloxy)carbonyl]amino}phenyl)acetate (10.0 g) obtained in step h) and triethylamine (8.2ml) in methylene chloride (100ml) were dropped at 0°C trifluoromethanesulfonic acid trimethylsilyl (11.9g) . After confirming production of silylenol ether using silica gel thin-layer chromatography, (2R,3R)-3-((1R)-1-{[tert-butyl(dimethyl)silyl]oxy}ethyl)-4-oxo-2-azetidinylacetate (6.98g) and zinc iodide (4.65g) were added at 0°C thereto, and the mixture was stirred for 1 hour. Thereto was further added (2R,3R)-3-((1R)-1-{[tert-butyl(dimethyl)silyl]oxy}ethyl)-4-oxo-2-azetidinylacetate (3.0g), followed by stirring at room temperature for 14 hours. To the reaction mixture were added 5% aqueous potassium hydrogen sulfate solution (380ml) and saturated brine (100 ml) and the mixture was extracted with ethyl acetate (200ml and then 100ml x 2). The combined organic layer was dried over anhydrous magnesium sulfate, filtered and concentrated in vacuo. The residue was purified by silica gel column chromatography (silica gel 500g, hexane : ethyl acetate = 1:1→1:3) to give the object compound (11.4g, yield 70%) as a pale yellow amorphous. ¹H NMR (400 MHz, CDCl₃) δ 0.08 (s, 3 H), 0.08 (s, 3 H), 0.88 (s, 9 H), 1.25 (d, 3 H, J = 6.2 Hz), 2.90 (dd, 1 H, J = 2.2, 5.1 Hz), 3.18 (dd, 1 H, J = 10.1, 17.8 Hz), 3.45 (dd, 1 H, J = 3.0, 17.8 Hz), 3.79 (s, 2 H), 4.10-4.15 (m, 1 H), 4.20-4.26 (m, 1 H), 4.69 (td, 2 H, J = 1.4, 4.4 Hz), 5.25 (s, 2 H), 5.27-5.31 (m, 1 H), 5.35-5.40 (m, 1 H), 5.93-6.03 (m, 1 H), 6.12 (bs, 1 H), 7.34 (d, 1 H, J = 8.0 Hz), 7.47-7.50 (m, 2 H), 7.70 (dd, 1 H, J = 1.8, 8.0 Hz), 7.83 (bs, 1 H), 8.21-8.24 (m, 2 H), 8.39 (br.s, 1 H). LCMS (EI) 640 (M+1)+.

### Step j)

### Allyl [(2R,3S)-2-[2-(3-{[(allyloxy)carbonyl]amino}-4-{2-[(4-nitrobenzyl)oxy]-2-oxoethyl}phenyl)-2-oxoethyl]-3-((1R)-1-{[tert-butyl(dimethyl)silyl]oxy}ethyl)-4-oxoazetidin-1-yl](triphenylphosphoanilidene)acetate

In accordance with the same method as referential example 2, using 4-nitrobenzyl (2-{[(allyloxy)carbonyl]amino}-4-{[(2R,3S)-3-((1R)-1-{[tert-butyl(dimethyl)silyl]oxy}ethyl)-4-oxoazetidin-2-yl]acetyl}phenyl)acetate (11.4g) obtained in step i), there was obtained the object compound (10.4g, yield 59%) as a pale brown amorphous.
LCMS (EI) 998 (M+1)+.

### Step k)

### (2-{[(Allyloxy)carbonyl]amino}-4-{[(2R,3S)-1-[2-(allyloxy)-2-oxo-1-(triphenylphosphoanilidene)ethyl]-3-((1R)-1-{[tert-butyl(dimethyl)silyl]oxy}ethyl)-4-oxoazetidin-2-yl]acetyl}phenyl)acetic acid

To a solution of allyl [(2R,3S)-2-[2-(3-{[(allyloxy)carbonyl]amino}-4-{2-[(4-nitrobenzyl)oxy]-2-oxoethyl}phenyl)-2-oxoethyl]-3-((1R)-1-{[tert-butyl(dimethyl)silyl]oxy}ethyl)-4-oxoazetidin-1-yl](triphenylphosphoanilidene)acetate (10.4g) obtained in step j) in THF (200ml) was suspended zinc (3.4g) and then added at room temperature 2M ammonium chloride solution (26ml), and the mixture was stirred for 2 hours. Thereto was added acetic acid (6.3g), followed by stirring for 1 hour. To the reaction mixture was added an aqueous 5% potassium hydrogen sulfate solution (200ml) and the mixture was extracted with ethyl acetate (100ml x 3). The combined extract was washed with aqueous 5% potassium hydrogen sulfate solution (100ml), dried over magnesium sulfate, filtered and concentrated in vacuo. The residue was purified by silica gel column chromatography (silica gel 300g, chloroform : methanol = 98:2→90:10) to give the object compound (9.11g, quantitative yield) as a brown amorphous. LCMS (EI) 863 (M+1)+.

### Referential example 32

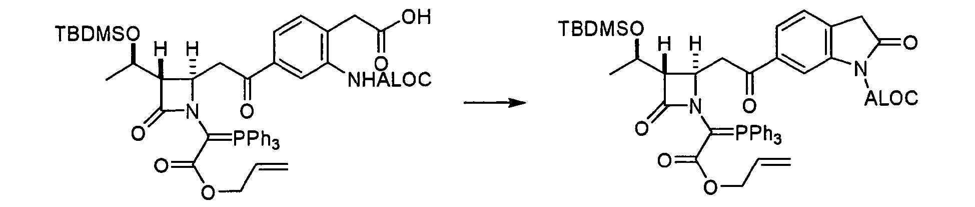

To a solution of (2-{[(allyloxy)carbonyl]amino}-4-{[(2R,3S)-1-[2-(allyloxy)-2-oxo-1-(triphenylphosphoanilidene)ethyl]-3-((1R)-1-{[tert-butyl(dimethyl)silyl]oxy}ethyl)-4-oxoazetidin-2-yl]acetyl}phenyl)acetic acid (0.86g) obtained by referential example 31 in THF (3ml) was added 0°C 1-ethyl-3-(3-dimethylaminopropyl)carbodiimido hydrochloride (0.23g), followed by stirring for 14 hours. To the reaction mixture were added an aqueous saturated ammonium chloride solution (15ml) and the mixture was extracted with chloroform (10ml x 3). The combined organic layer was dried over anhydrous magnesium sulfate, filtered and concentrated to give allyl 6-{[(2R,3S)-1-[2-(allyloxy)-2-oxo-1-(triphenylphosphoanilidene)ethyl]-3-((1R)-1-{[tert-butyl(dimethyl)silyl]oxy}ethyl)-4-oxoazetidin-2-yl]acetyl}-2-oxo-indoline-1-carboxylate (0.89g, quantitative yield) as a crude product. This product was used in the following reaction without purification. LCMS 845 (M+1)+.

### Referential example 33

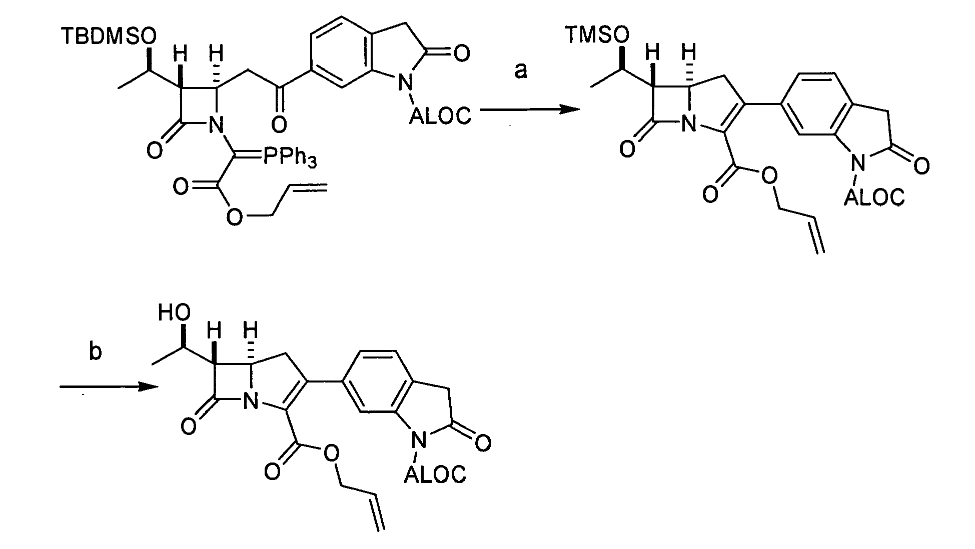

### Step a)

### Allyl 6-((5R,6S)-2-[(allyloxy)carbonyl]-7-oxo-6-{(1R)-1-[(trimethylsilyl)oxy]ethyl}-1-azabicyclo[3.2.0]hept-2-ene-3-yl)-2-oxo-indoline-1-carboxylate

In accordance with the same method as step a) of referential example 15, using allyl 6-{[(2R,3S)-1-[2-(allyloxy)-2-oxo-1-(triphenylphosphoranilidene)ethyl]-3-((1R)-1-{[tert-butyl(dimethyl)silyl]oxy}ethyl)-4-oxoazetidin-2-yl]acetyl}-2-oxoindoline-1-carboxylate (0.89g) obtained by referential example 32, there was obtained allyl 6-((5R,6S)-2-[(allyloxy)carbonyl]-7-oxo-6-{(1R)-1-[(trimethylsilyl)oxy]ethyl}-l-azabicyclo[3.2.0]hept-2-ene-3-yl)-2-oxoindoline-1-carboxylate (0.26g, yield 70%) as a pale yellow amorphous.
¹H NMR (400 MHz, CDCl₃) δ 0.15 (s, 9 H), 1.30 (d, 3 H, J = 6.2 Hz), 3.15-3.39 (m, 3 H), 3.69 (s, 2 H), 4.19-4.26 (m, 2 H), 4.61-4.75 (m, 2 H), 4.89-4.91 (m, 2 H), 5.17-5.36 (m, 2 H), 5.49-5.55 (m, 1 H), 5.82-5.92 (m, 1 H), 5.99-6.09 (m, 1 H), 7.18 (dd, 1 H, J = 1.5, 7.8 Hz), 7.24 (d, 1 H, J = 7.8 Hz), 7.93 (d, 1 H, J = 1.5 Hz).

### Step b)

### Allyl 6-{(5R,6S)-2-[(allyloxy)carbonyl]-6-[(1R)-1-hydroxyethyl]-7-oxo-1-azabicyclo[3.2.0]hept-2-ene-3-yl}-2-oxoindoline-1-carboxylate

In accordance with the same method as step b) of referential example 15, using allyl 6-((5R,6S)-2-[(allyloxy)carbonyl]-7-oxo-6-{(1R)-1-[(trimethylsilyl)oxy]ethyl}-1-azabicyclo[3.2.0]hept-2-ene-3-yl)-2-oxoindoline-1-carboxylate (0.26g) obtained in step a), there was obtained allyl 6-{(5R,6S)-2-[(allyloxy)carbonyl]-6-[(1R)-1-hydroxyethyl]-7-oxo-1-azabicyclo[3.2.0]hept-2-ene-3-yl}-2-oxoindoline-1-carboxylate (0.26g, quantitative yield) as a pale yellow solid.
¹H NMR (400 MHz, CDCl₃) δ 1.39 (d, 3 H, J = 6.3 Hz), 3.18-3.36 (m, 3 H), 3.70 (s, 2 H), 4.24-4.34 (m, 2 H), 4.61-4.75 (m, 2 H), 4.89-4.91 (m, 2 H), 5.17-5.21 (m, 1 H), 5.27-5.32 (m, 1 H), 5.33-5.37 (m, 1 H), 5.49-5.55 (m, 1 H), 5.82-5.92 (m, 1 H), 5.99-6.09 (m, 1 H), 7.18 (dd, 1 H, J = 1.6, 7.8 Hz), 7.25 (d, 1 H, J = 7.8 Hz), 7.93 (d, 1 H, J = 1.6 Hz).

### INDUSTRIAL APPLICABILITY

A carbapenem compound of the present invention can be used as antibacterial agent for oral administration having a potent antibacterial activity against Gram positive bacteria and Gram negative bacteria, especially Haemophilus influenzae (which obtain resistance to the inhibitory effect of existing β-lactam agents together with mutation of a penicillin binding proteins (PBP) such as β-lactamase non-producing ampicillin resistant Haemophilus influenzae (BLNAR), and penicillin resistant Streptococcus pneumoniae (PRSP), which are recently increasingly isolated and provide a clinical problem) and has excellent oral absorbability.

## Claims

1. The carbapenem compound represented by a following formula [1], 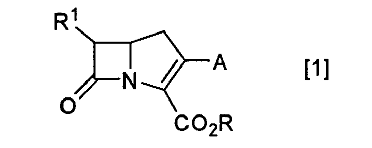 wherein R¹ is C₁ to C₃ alkyl or C₁ to C₃ alkyl substituted by hydroxy. R is hydrogen atom or a group which regenerates a carboxyl group by hydrolysis in a living body,
A is a following formula [1a], 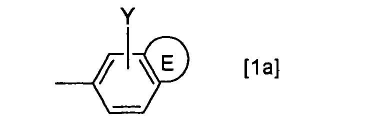 wherein E is a 5 to 7 membered cyclic ring optionally containing 1 to 3 hetero atoms (excluding benzene ring) which forms a bicyclic ring in corporation with the benzene ring, and
Y is hydrogen atom, C₁ to C₄ alkyl, C₁ to C₄ alkoxy, trifluoromethoxy, halogen atom or cyano group,
or its pharmaceutically acceptable salt.

2. The carbapenem compound or its pharmaceutically acceptable salt according to claim 1, wherein the group which regenerates a carboxyl group by hydrolysis in a living body is C₁ to C₄ alkyl, C₂ to C₁₂ alkyloxyalkyl, a following formula [2], 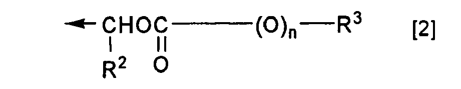 wherein R² is hydrogen atom or C₁ to C₆ alkyl, R³ is optionally substituted C₁ to C₁₀ alkyl or optionally substituted C₃ to C₁₀ cycloalkyl
and n is 0 or 1,
or a following formula [3], 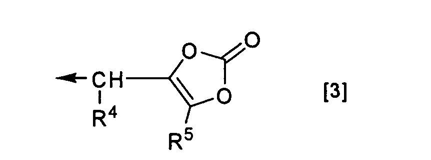 wherein R⁴ is hydrogen atom or C₁ to C₆ alkyl, and R⁵ is hydrogen atom, C₁ to C₆ alkyl, C₃ to C₁₀ cycloalkyl, or phenyl.

3. The carbapenem compound or its pharmaceutically acceptable salt according to claim 1, wherein R is hydrogen atom, pivaloyloxymethyl group or (5-methyl-2-oxo-1,3-dioxol-4-yl)methyl.

4. The carbapenem compound or its pharmaceutically acceptable salt according to any one of claims 1 to 3, wherein R¹ is 1-hydroxyethyl group.

5. The carbapenem compound or its pharmaceutically acceptable salt according to any of claims 1 to 4, wherein A is a following formula [1b], 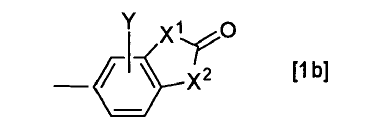 wherein Y is the same as defined in claim 1, X¹ is -O-, -NR⁶-(wherein R⁶ is hydrogen atom, a group which is metabolized in a living body to produce imino group, or optionally substituted C₁ to C₄ alkyl group) or methylene, X² is -O- or -NR⁷ wherein R⁷ is hydrogen atom, a group which is metabolized in a living body to produce imino group, or optionally substituted C₁ to C₄ alkyl group) or methylene.

6. The carbapenem compound or its pharmaceutically acceptable salt according to claim 5, wherein X¹ is -O- or methylene, X² is -NR⁷- (wherein R⁷ is the same as in the claim 5).

7. The carbapenem compound or its pharmaceutically acceptable salt according to claim 5, wherein X¹ is -NR⁶- (wherein R⁶ is the same as in the claim 5) and X² is -O- or methylene.

8. The carbapenem compound or its pharmaceutically acceptable salt according to any one of claims 1 to 4, wherein A is represented by a following formula [1c], 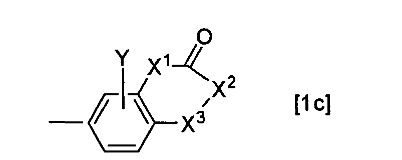 wherein X¹, X² and Y are the same as defined in claim 5, X³ is -O-, - NR⁸- (wherein R⁸ is hydrogen atom, a group which is metabolized in a living body to produce imino group or optionally substituted C₁ to C₄ alkyl group) or methylene, provided that when X³ is -O-, X² is -NR⁵- (wherein R⁵ is the same as defined in claim 5) or methylene.

9. The carbapenem compound or its pharmaceutically acceptable salt according to claim 8, wherein X¹ is -NR⁶- (wherein R⁶ is the same as defined in claim 5), X² is methylene and X³ is -O-.

10. The carbapenem compound or its pharmaceutically acceptable salt according to any one of claims 1 to 4, wherein A is represented by a following formula [1d], 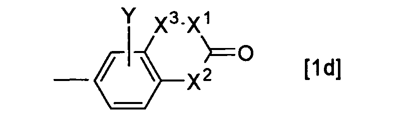 wherein X¹, X², X³ and Y are the same as defined in claim 8, provided that when X³ is -O-, X¹ is -NR⁶- (wherein R⁶ is the same as defined in claim 5) or methylene.

11. A medicament for oral administration, which comprises the carbapenem compound or its pharmaceutically acceptable salt according to any one of claims 1 to 10 as an active ingredient.

12. An antibacterial agent containing the carbapenem compound or its pharmaceutically acceptable salt according to any one of claims 1 to 10 as an active ingredient.

13. An antibacterial agent for oral administration containing the carbapenem compound or its pharmaceutically acceptable salt according to any one of claims 1 to 10 as an active ingredient.
